# EUROPEAN PATENT SPECIFICATION

(11) **EP 4 138 783 B1**
(45) Date of publication and mention of the grant of the patent: **12.08.2026**
(21) Application number: 21793476.9
(22) Date of filing: 22.04.2021
(51) Int. Cl.: A61K 9/00, A61K 9/50, A61K 9/10, A61K 9/58, A61K 47/34, A61K 47/38, A61K 31/445, A61P 23/00, A61P 25/04

(54) **LONG-ACTING BUPIVACAINE MICROSPHERE FORMULATIONS**
LANGWIRKENDE BUPIVACAINMIKROKUGELFORMULIERUNGEN
FORMULATIONS DE MICROSPHÈRES DE BUPIVACAÏNE À ACTION PROLONGÉE

(30) Priority: 22.04.2020 US 202063013736 P
(43) Date of publication of application: 01.03.2023
(73) Proprietor: Scienture, LLC, Commack NY 11725 (US)
(72) Inventor: HARIHARAN, Shankar, New York 11787 (US); SANGHVI, Suketu, Kendall Park, New Jersey 08824 (US); SURANA, Rahul, New York 11725 (US); DIJK, Maarten van, 9746 RT Groningen (NL); TERZIC, Ivan, 9718 ML Groningen (NL); BANUS, Kimberly Aniek, 7827 PH Emmen (NL); NGUYEN, Thanh, 9766 PW Eelderwolde (NL); STEENDAM, Rob, 9728 XR Groningen (NL)
(74) Representative: Appleyard Lees IP LLP
(86) International application number: PCT/US2021/028718
(87) International publication number: WO 2021/216928

(56) References cited:
- EP-A1- 3 586 828
- EP-A1- 3 586 828
- WO-A2-2009/129210
- US-A1- 2003 152 637
- US-A1- 2003 152 637
- US-A1- 2016 030 671
- US-A1- 2016 089 335
- US-A1- 2016 089 335
- US-A1- 2020 069 595
- US-A1- 2020 069 595
- US-B1- 6 238 702
- US-B1- 6 238 702
- US-B1- 6 383 168
- ANONYMOUS: "Drug Admistration-approved long-acting formulations on the market.* Home > Sustained-Release Injectable Drug Delivery Sustained-Release Injectable Drug Delivery", PHARMACEUTICAL TECHNOLOGY, no. 6, 1 November 2010 (2010-11-01), pages 1 - 7, XP055471445

## Description

### FIELD OF THE INVENTION

According to various aspects of this disclosure, the present disclosure relates to shelf-stable long-acting microsphere formulations of bupivacaine, kits, and stable pharmaceutically acceptable formulation for use in treating or preventing pain by parenteral injection of a microsphere formulation of bupivacaine.

### BACKGROUND OF THE INVENTION

Bupivacaine is an amide local anesthetic that blocks both the initiation and conduction of nerve impulses by decreasing the neuronal membrane's permeability to sodium ions, which results in inhibition of depolarization with resultant blockade of conduction. The hydrochloride salt of bupivacaine ("bupivacaine HCl") is indicated for local infiltration, peripheral nerve block, sympathetic nerve block, and epidural and caudal blocks.

Bupivacaine HCl is only useful as a local anesthetic for short-term pain relief. In dental injections, for instance, bupivacaine HCl has an onset of action of about 2-10 minutes and a duration of action of about 2-8 hours. As with other local anesthetics, bupivacaine HCl can be co-administered with epinephrine to extend the duration of analgesia, wherein epinephrine-induced vasoconstriction correlates with duration of analgesia (Liu et al. 1995). However, epinephrine can be toxic, and increases hypertensive risk, such that bupivacaine HCl plus epinephrine is cautioned against in patients receiving monoamine oxidase inhibitors or tricyclic antidepressants, wherein concurrent use can cause severe, persistent hypertension or cerebrovascular accidents.

Moreover, adverse reactions with bupivacaine HCl are consonant with other amide-type local anesthetics, wherein excessive plasma levels can cause systemic reactions involving the central nervous system and cardiovascular system, including: nervousness, dizziness, blurred vision, tremors, drowsiness, convulsions, unconsciousness, respiratory arrest, nausea, vomiting, chills, constriction of the pupils, tinnitus, depression of the myocardium, blood pressure changes (usually hypotension), cardiac arrest, and allergic reactions characterized by cutaneous lesions, edema, and other manifestations of allergy.

A liposomal formulation of bupivacaine (EXPAREL^{®}) has been shown to address some of these issues. However, liposomal formulations suffer from instability, and can not be stored for long. EXPAREL^{®} can be stored at room temperature no longer than 30 days in sealed, unopened vials, which cannot be re-refrigerated, and can never be frozen or exposed to temperatures greater than 40°C. A long acting bupivacaine solution (POSIMIR^{®}) has been shown to produce post-surgical analgesia, but only for 72 hours following arthroscopic subacromial decompression.

Therefore, there remains a need for a shelf-stable long-acting formulation of bupivacaine.

### DESCRIPTION OF FIGURES

FIG. 1. A one-compartment simulation of 800 mg active load microsphere formulations of bupivacaine concentration over 7 days, with an initial 10% burst release followed by a second phase with 0.7% or 0.8% release per hour, and with or without a 6 hour delay.
FIG. 2. A one-compartment simulation of 1000 mg active load microsphere formulations of bupivacaine concentration over 7 days, with an initial 10% burst release followed by a second phase with 0.7% or 0.8% release per hour, and with a 12 hour or 24 hour delay.
FIGs. 3A-3B. Particle size distribution of bupivacaine-loaded microspheres composed of 60LP2L20-D27 (120A-200240, 25.3% bupivacaine) (FIG. 3A) and 10LP10L20-GLL40 (120A-200241, 21.8% bupivacaine) (FIG. 3B).
FIGs. 4A-4F. Scanning electron microscopy images of bupivacaine-loaded microspheres composed of 60LP2L20-D27 (120A-200240, 25.3% bupivacaine) and 10LP10L20-GLL40 (120A-200241, 21.8% bupivacaine) using different magnification (FIG. 4A and FIG. 4D: 50X; FIG. 4B and FIG. 4E: 200X; FIG. 4C and FIG. 4F: 1500X).
FIG. 5. Cumulative release of bupivacaine from bupivacaine-loaded microspheres composed of 60LP2L20-D27 (120A-200240, 25.3% bupivacaine) and 10LP10L20-GLL40 (120A-200241, 21.8% bupivacaine).
FIG. 6. Cumulative release of bupivacaine from bupivacaine-loaded microspheres composed of 60LP2L20-D27 prepared using different formulation and process parameter settings.

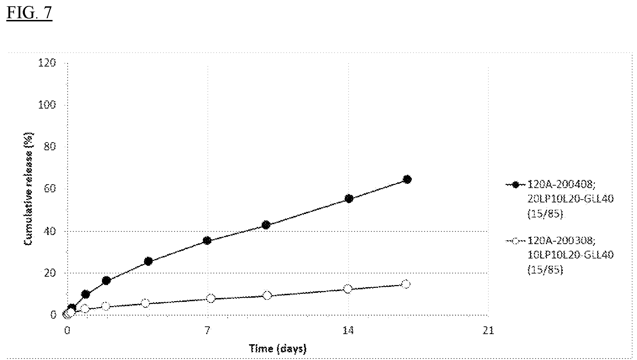

FIG. 7. Cumulative release of bupivacaine from bupivacaine-loaded microspheres composed of 10LP10L20-GLL40 (15/85) with 44.4% bupivacaine loading (120A-200308) and 20LP10L20-GLL40 (15/85) with 44.3% bupivacaine loading (120A-200408).
FIGs. 8A-8C. Scanning electron microscopy images of bupivacaine-loaded microspheres prepared at 5 g scale: 120A-210039 (60LP2L20-D27, 41.2% bupivacaine) (FIG. 8A), 120A-210040 (60LP2L20-D27, 34.8% bupivacaine) (FIG. 8B) and 120A-210092 (20LP10L20-GLL40, 46.3% bupivacaine) (FIG. 8C) (magnification of 200X).
FIG. 9. Cumulative release of bupivacaine from 120A-210039 (60LP2L20-D27, 41.2% bupivacaine), 120A-210040 (60LP2L20-D27, 34.8% bupivacaine) and 120A-210092 (20LP10L20-GLL40, 46.3% bupivacaine).
FIG. 10. Injectability of 60LP2L20-D27-based bupivacaine microspheres (120A-210039) at 100 mg/ml suspension concentration (41 mg bupivacaine/mL), 25 G 5/8" needle.
FIG. 11. Plasma concentration over time of bupivacaine in dogs after injection of 45 mg of formulation 120A-210039, 120A-210040, or 120A-210092.
FIG. 12. Cumulative area under the curve (AUC) from in vivo pharmacokinetics of bupivacaine formulations 120A-210039, 120A-210040, and 120A-210092.

### BRIEF SUMMARY OF THE INVENTION

The present disclosure provides a shelf-stable long-acting microsphere formulation of bupivacaine, which extends the duration of action of bupivacaine beyond that of bupivacaine HCl, contains no epinephrine, and avoids the adverse reactions associated with excessive plasma levels following injection.

### Stable Microsphere Formulation of Bupivacaine

In a first aspect, the present disclosure provides a stable pharmaceutically acceptable formulation comprising a microsphere, the microsphere comprising: a biodegradable multiblock copolymer; and an active drug load of bupivacaine free base or a pharmaceutically acceptable salt of bupivacaine, wherein the multiblock copolymer comprises at least one hydrolysable prepolymer (A) segment and at least one hydrolysable prepolymer (B) segment, wherein the segments are linked by a multifunctional chain extender, and wherein the segments are randomly and non alternatingly distributed over the polymer chain, the prepolymer (A) segment comprises polyethylene glycol, the prepolymer (A) segment comprises reaction products of glycolide and/or lactide (D and/or L), the prepolymer (B) segment comprises poly(glycolide co L lactide) with a Mn of about 4000 g/mol, the prepolymer (B) segment comprises a molar amount of 5 % to 25 % of glycolide relative to combined molar amount of glycolide and L lactide, the multiblock copolymer comprises from 10 % to 99 % of the prepolymer (B) segment relative to the total weight of the multiblock copolymer, the multifunctional chain extender is 1,4 butane diisocyanate, wherein the stable pharmaceutically acceptable formulation has a shelf life of 14 days at 25 °C following refrigeration.

In a second aspect, the present invention provides a method of producing a stable pharmaceutically acceptable formulation comprising a microsphere, the method comprising: providing a first phase comprising: a biodegradable polymer as defined in any one of claims 1 6; and an active drug load of bupivacaine free base or a pharmaceutically acceptable salt of bupivacaine; adding a second phase comprising an aqueous surfactant continuously into the first phase to form an emulsion; adding a quench solution to the emulsion to produce a volume comprising a microsphere; and washing, filtering, and drying the microsphere to reduce solvent content.

In a third aspect, the present invention provides a method of producing a stable pharmaceutically acceptable formulation comprising a microsphere, the method comprising: providing a first phase comprising: a biodegradable polymer as defined in an aspect of the invnetion; an active drug load of bupivacaine free base or a pharmaceutically acceptable salt of bupivacaine; and a solvent system suitable to dissolve the polymer and bupivacaine; emulsifying the first phase with a second phase, thereby forming an emulsion; wherein the second phase comprises an aqueous solution which comprises a surfactant; and removing a substantial portion of the solvent system from the emulsion, thereby obtaining a microsphere.

In a fourth aspect, the present invention provides a stable pharmaceutically acceptable formulation for use in the treatment of pain comprising a microsphere, the microsphere comprising a biodegradable polymer as defined in an aspect of the invention and 200 mg to 8000 mg of bupivacaine free base or a pharmaceutically acceptable salt thereof, wherein the pharmaceutical formulation effects a therapeutically effective concentration of bupivacaine free base or a pharmaceutically acceptable salt thereof for 2 days to 14 days following administration to a subject.

In a fifth aspect, the present invention provides a pre filled injector comprising: a stable pharmaceutically acceptable formulation comprising: a microsphere comprising: a biodegradable polymer as defined in an aspect of the invnetion; and an active drug load of bupivacaine free base or a pharmaceutically acceptable salt of bupivacaine.

In a sixth aspect, the present invention provides a method of manufacturing a pre-filled injector comprising a stable pharmaceutically acceptable formulation, the method comprising: preparing a stable pharmaceutically acceptable formulation comprising: a microsphere comprising: a biodegradable polymer as defined in an aspect of the invention; and an active drug load of bupivacaine free base or a pharmaceutically acceptable salt of bupivacaine, loading a sterile cartridge with the stable pharmaceutically acceptable formulation; and attaching the sterile cartridge operably to an injector.

In a seventh aspect, the present invention provides a long acting dosage form comprising: a microsphere comprising a biodegradable polymer as defined in an aspect of the invention; and 500 mg to 4000 mg of bupivacaine free base or a pharmaceutically acceptable salt thereof, wherein administration of a single dose of the long-acting dosage form to a subject results in at least one of the pharmacokinetic parameters selected from the group consisting of: a steady state plasma profile of bupivacaine from day 1 to day 7 following administration exhibiting a mean Cmax value no greater than the steady state plasma level of bupivacaine provided by 100 mg of immediate release subcutaneous injection of bupivacaine hydrochloride; a bupivacaine elimination half-life of 2 hours to 4 hours; and a zero order or first order release profile corresponding to 1 % to 50 % release of the total administered dose of bupivacaine or a pharmaceutically acceptable salt thereof per day.

In an eighth aspect, the present invention provides a kit comprising: a first vial comprising a concentrated form of the stable pharmaceutically acceptable formulation of an aspect of the invention or of the long-acting dosage form of an aspect of the invention; a second vial comprising a pharmaceutically acceptable diluent; a first syringe suitable for withdrawing the pharmaceutically acceptable diluent from the second vial; an adapter which can operably attach to the first syringe and is suitable for dispensing the pharmaceutically acceptable diluent into the first vial; a second syringe suitable for withdrawing a liquid from the second vial and for injecting the liquid into a subject in need thereof; and instructions for diluting the concentrated form and for administering the stable pharmaceutically acceptable formulation or the long-acting dosage form to the subject.

### DETAILED DESCRIPTION OF THE INVENTION

### Definitions

Unless defined otherwise, all technical and scientific terms used herein have the same meaning as commonly understood by one of ordinary skill in the art to which this disclosure belongs. In case of conflict, the present application including the definitions will control. Unless otherwise required by context, singular terms shall include pluralities and plural terms shall include the singular.

Although methods and materials similar or equivalent to those described herein can be used in practice or testing of the present disclosure, suitable methods and materials are described below. The materials, methods and examples are illustrative only and are not intended to be limiting. Other features and advantages of the disclosure will be apparent from the detailed description and from the claims.

In order to further define this disclosure, the following terms and definitions are provided.

The singular forms "a," "an" and "the" include plural referents unless the context clearly dictates otherwise. The terms "a" (or "an"), as well as the terms "one or more," and "at least one" can be used interchangeably herein. In certain aspects, the term "a" or "an" means "single." In other aspects, the term "a" or "an" includes "two or more" or "multiple."

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

Throughout this disclosure, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6, etc., as well as individual numbers within that range, for example, 1, 2, 3, 4, 5, and 6. This applies regardless of the breadth of the range. Numeric ranges recited are inclusive of the numbers defining the range and include each integer within the defined range.

Units, prefixes, and symbols are denoted in their Système International de Unites (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the disclosure. Thus, ranges recited herein are understood to be shorthand for all of the values within the range, inclusive of the recited endpoints. For example, a range of 1 to 10 is understood to include any number, combination of numbers, or sub-range from the group consisting of 1, 2, 3, 4, 5, 6, 7, 8, 9, and 10.

Where a value is explicitly recited, it is to be understood that values which are about the same quantity or amount as the recited value are also within the scope of the disclosure. Where a combination is disclosed, each subcombination of the elements of that combination is also specifically disclosed and is within the scope of the disclosure. Conversely, where different elements or groups of elements are individually disclosed, combinations thereof are also disclosed. Where any element of a disclosure is disclosed as having a plurality of alternatives, examples of that disclosure in which each alternative is excluded singly or in any combination with the other alternatives are also hereby disclosed; more than one element of a disclosure can have such exclusions, and all combinations of elements having such exclusions are hereby disclosed.

As used herein, the term "substantial" means more than a minimal or insignificant amount; and "substantially" means more than a minimally or insignificantly. Thus, for example, the phrase "substantially similar", as used herein, denotes a sufficiently high degree of similarity between two numeric values or features such that one of skill in the art would consider the difference between the two values to not be of statistical significance or qualitative significance within the context of the characteristic measured by said values or features. Thus, the difference between two values that are substantially similar to each other is typically less than about 10%, and can be less than about 20%, less than about 30%, less than about 40%, or less than about 50% as a function of the reference value or comparator value.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "pharmaceutically acceptable" as used herein refers to those compounds, materials, compositions, formulations, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "excipient" refers to any substance, not itself a therapeutic agent, which may be used in a composition for delivery of an active therapeutic agent to a subject or combined with an active therapeutic agent (e.g., to create a pharmaceutical composition) to improve its handling or storage properties or to permit or facilitate formation of a dose unit of the composition (e.g., formation of a hydrogel which may then be optionally incorporated into a patch). Excipients include, but are not limited to, solvents, penetration enhancers, wetting agents, antioxidants, lubricants, emollients, substances added to improve appearance or texture of the composition and substances used to form hydrogels. Any such excipients can be used in any dosage forms according to the present disclosure. The foregoing classes of excipients are not meant to be exhaustive but merely illustrative as a person of ordinary skill in the art would recognize that additional types and combinations of excipients could be used to achieve the desired goals for delivery of a drug. The excipient can be an inert substance, an inactive substance, and/or a not medicinally active substance. The excipient can serve various purposes. A person skilled in the art can select one or more excipients with respect to the particular desired properties by routine experimentation and without any undue burden. The amount of each excipient used can vary within ranges conventional in the art. Techniques and excipients which can be used to formulate dosage forms are described in Handbook of Pharmaceutical Excipients, 6th edition, Rowe et al., Eds., American Pharmaceuticals Association and the Pharmaceutical Press, publications department of the Royal Pharmaceutical Society of Great Britain (2009); and Remington: the Science and Practice of Pharmacy, 21th edition, Gennaro, Ed., Lippincott Williams & Wilkins (2005).

The term "effective amount" or "pharmaceutically effective amount" or "therapeutically effective amount" as used herein refers to the amount or quantity of a drug or pharmaceutically active substance which is sufficient to elicit the required or desired therapeutic response, or in other words, the amount which is sufficient to elicit an appreciable biological response when administered to a patient.

"Liposome" as the term is used herein refers to a closed structure comprising of an outer lipid bi- or multi-layer membrane surrounding an internal aqueous space. Liposomes can be used to package any biologically active agent for delivery to cells.

The term "Bupivacaine liposome" as used herein refers to liposome formulations of bupivacaine, wherein bupivacaine, a pharmaceutically acceptable salt of bupivacaine, or a bupivacaine prodrug is enclosed in a liposome structure. One such example of a bupivacaine liposome formulation is Exparel^{®}.

As used herein, the terms "polymer" and "polymers" include "copolymer" and "copolymers".

The term "biodegradable polymer" refers to polymers which are degraded within a patient. Generally, a polymer that loses its weight over time in the living body can be referred to as an absorbable, resorbable, bioabsorbable, or even biodegradable polymer. This terminology applies regardless of its degradation mode, in other words for both enzymatic and non-enzymatic hydrolysis. Biodegradable polymers, including resorbable polymers, can be classified on the basis of their origin as either naturally occurring or synthetic. Non-limiting examples of these each of which may individually or in combination be used to form all or part of the biodegradable polymer microspheres include poly(L-lactide), poly(glycolide) and polymers or copolymers based on L-lactide, L/DL-lactide, DL-lactide, glycolide, trimethyl carbonate, ε-caprolactone, p-dioxanone, and physical and chemical combinations thereof.

The term "microsphere" as used herein refers to a subcutaneously injectable particle comprising a biodegradable polymer, wherein the particle contains bupivacaine free base or a pharmaceutically acceptable salt of bupivacaine. Microspheres have certain physical characteristics useful for biodegradable delivery systems, including desirable solubilities and porosities for sustained delivery of an active ingredient in a relatively constant manner over an extended period of time. A microsphere of the present disclosure can be of a diameter of about 1 µm to about 500 µm, about 5 µm to about 200 µm, about 10 µm to about 100 µm, about 20 µm to about 70 µm, about 10 nm to about 10 µm, about 20 nm to about 10 µm, about 100 nm to about 1 µm, or about 250 nm to about 750 nm. In some aspects, a microsphere of the present disclosure can be of a diameter of about 50 µm. Microspheres of the present disclosure are suitable for embedding an API within the polymeric matrix. The terms "a microsphere" or "the microsphere" include multiple microspheres (e.g., a plurality of microspheres), unless the context clearly dictates otherwise. For example, a pharmaceutical composition comprising "a microsphere" should be interpreted to also encompass embodiments wherein the pharmaceutical composition comprises microspheres, or a plurality of microspheres. In a further example, a method comprising a step of "drying the microsphere" should be interpreted to also encompass embodiments wherein microspheres, or a plurality of microspheres, are dried.

The term "active drug load" as used herein refers to the amount of bupivacaine free base or a pharmaceutically acceptable salt of bupivacaine which is contained within the microspheres in a pharmaceutically acceptable formulation described herein. A non-limiting example is 35% active drug load, which refers to a microparticle composition comprising 35% bupivacaine by weight.

As used herein, "Dv10" is a characterization of a plurality of microspheres, such that 10% of the microspheres have a diameter less than the recited Dv10 value. As used herein, "Dv50" is a characterization of a plurality of microspheres, such that 50% of the microspheres have a diameter less than the recited Dv50 value. As used herein, "Dv90" is a characterization of a plurality of microspheres, such that 90% of the microspheres have a diameter less than the recited Dv90 value.

Unless context dictates otherwise, the term "span value" as used herein refers to a characterization of the width of the particle size distribution of a plurality of microspheres. The span value of a plurality of microspheres is calculated as (Dv90-Dv10)/Dv50.

As used herein, the term "long-acting" refers to the duration of action of a composition of API as disclosed and claimed herein. More specifically, the term "long-acting" refers to the period of time after which a desired blood plasma bupivacaine level is maintained after a certain dose of a formulation disclosed herein has been administered. In one non-limiting example, the formulations of the present invention provide effective plasma bupivacaine levels over a period of more than about 1 day to about 10 days after a single dose to a subject in need thereof. In alternative non-limiting examples, the formulations disclosed herein can provide effective plasma bupivacaine levels after a single dose to a subject in need thereof over a period of about 1 day, about 2 days, about 3 days, about 4 days, about 5 days, about 6 days, about 7 days, about 8 days, about 9 days, or about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days. In some aspects, the term "long-acting" refers to an enhancement of duration of physiological activity compared to that of bupivacaine hydrochloride injection.

The term "unit dosage form" or "unit dose composition" as used herein refers to a device containing a quantity of the therapeutic compound, said quantity being such that one or more predetermined units can be provided as a single therapeutic administration.

As used herein, the term "dose" refers to an amount of a composition described herein which is administered to a subject. A single dose can be administered as a single volume or divided among multiple administered volumes. For example, "the amount of the stable pharmaceutically acceptable formulation dispensed in a single dose contains about 200 mg to about 8000 mg of bupivacaine free base or a pharmaceutically acceptable salt of bupivacaine" refers to administration of about 200 mg to about 8000 mg of bupivacaine free base or a pharmaceutically acceptable salt of bupivacaine in either a

### single injection or divided among multiple injections.

The term "Cₘₐₓ" as used herein refers to the maximum plasma concentration of a drug after administration of the drug.

The term "Tₘₐₓ" as used herein refers to the time required to reach the maximal plasma concentration Cₘₐₓ after administration of a drug.

The term "AUC" as used herein refers to the area under the curve of a plot of plasma concentration versus time following administration of a drug.

The term "AUC₀₋ₜ" as used herein refers to the area under the drug concentration-time curve from time zero to the time of the last measurable concentration (Ct).

The term "AUC_{0-∞}" as used herein refers to the area under the drug concentration-time curve from time zero to infinity.

The term "steady state" as used herein means that the amount of the drug reaching the system is approximately the same as the amount of the drug leaving the system. Thus, at "steady-state," the patient's body eliminates the drug at approximately the same rate that the drug becomes available to the patient's system through absorption into the blood stream.

The term "zero-order release profile" as used herein refers to a relatively constant rate of release (i.e., exhibiting a substantially linear release profile over a period of time, preferably at least a few hours). Although a small portion of the release profile may not be zero-order, a substantial portion (e.g., several hours), and preferably a major portion, of the release profile is representative of zero-order release kinetics.

The term "first-order release profile" as used herein refers to a release profile that is characterized by a release rate that decreases over time. By plotting the cumulative release versus the square root of time, a linear curve is obtained. Although a small portion of the release profile may not be first-order, a substantial portion (e.g., several hours), and preferably a major portion, such as for example up to 60% cumulative release, of the release profile is representative of first-order release kinetics.

The term "mean" refers to an average value in a patient population. For example, a "mean maximum plasma level Cₘₐₓ" refers to an average of the maximum plasma concentrations of a drug in a patient population.

The term "treating" or "treatment" as used herein refers to the administration of a composition to a subject for therapeutic purposes.

The term "serum concentration" generally refers to the amount of a drug or other compound in the circulation, both bound to proteins and unbound, the latter of which generally corresponds to the therapeutically active fraction.

The term "bioavailability" generally refers to the rate and extent to which the active ingredient is absorbed from a drug product and becomes available at the site of action.

"Bioequivalence" is a term in pharmacokinetics generally used to assess the expected in vivo biological equivalence of two proprietary preparations of a drug. Two pharmaceutical products are bioequivalent if they are pharmaceutically equivalent and their bioavailabilities (rate and extent of availability) after administration in the same molar dose are similar to such a degree that their effects, with respect to both efficacy and safety, can be expected to be essentially the same.

The term "injector" as used herein refers to an apparatus wherein an individual can administer a formulation, such as a pharmaceutical formulation, to oneself. In some aspects, the injector delivers a single dose. In some aspects, the injector is adjustable to deliver various volumes of the bupivacaine formulation. In some aspects, multiple injections can be dispensed from the same injector. In other aspects, part or all of the injector is disposable and/or reusable. In some aspects, part or all of the injector is opaque, and in further specific embodiments at least one part of the injector that is opaque is the part that houses the pharmaceutical formulation. An injector can be supplied separately from the pharmaceutical formulations, in alternative aspects. The injector can comprise an exchangeable vessel for replacing the pharmaceutical formulation, such as an insert, cartridge, vial, and so forth. Such an exchangeable vessel may be glass or plastic, for example. It will be understood by the skilled artisan that the injector can be an autoinjector, a pen injector, a needle-less injector, or any other injection device suitable for the delivery of a pharmaceutical formulation.

In a further aspect, bupivacaine formulations can be administered via a parenteral route. As used herein, the term "parenteral" includes routes that bypass the alimentary tract. Specifically, the pharmaceutical compositions disclosed herein can be administered for example, but not limited to intravenously, intradermally, intramuscularly, intraarterially, intrathecally, subcutaneous, or intraperitoneally.

The term "bupivacaine" refers to 1-butyl-N-(2,6-dimethylphenyl)-2-piperidinecarboxamide. Bupivacaine has the following chemical structure:

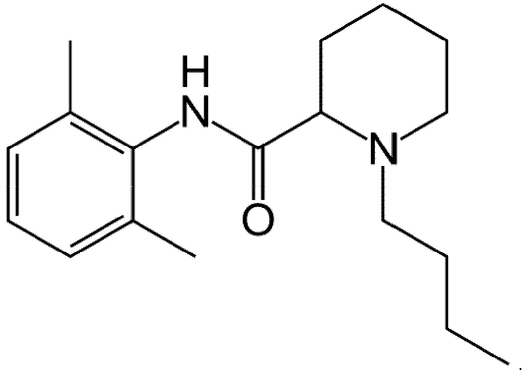

The term "Water for Injection" ("WFI") refers to water that meets the U.S.P. requirements (or foreign equivalent) for "Water for Injection." These requirements include bacterial endotoxins of not more than 0.25 U.S.P. EU per mL, total organic carbon (TOC) content of <500 parts per billion (ppb), and conductivity of 1.3 µS/cm. Water for Injection also includes compendial and non-compendial water classifications that meet the requirements of U.S.P. Water for Injection. Examples include water labeled or marketed as "Low Endotoxin U.S.P. Purified Water" and "WFI Quality Water."

The term "q.s." as used herein refers to "quantum satis", and would be understood by a person of ordinary skill in the art to refer to an amount which must be added to achieve a result. In one non-limiting example, "HCl/NaOH q.s. to pH 5-7" would be understood to refer to an amount of hydrochloric acid or sodium hydroxide which is added to a solution to achieve a final pH in the solution of between 5 and 7, inclusive of endpoints. In another non-limiting example, "WFI q.s. to 20 mL" would be understood to refer to a volume of Water for Injection which is added to a sample to achieve a final volume of the sample of 20 mL.

The term "multi-block" as used herein is meant to refer to the presence of at least two distinct pre-polymer segments in a polymer chain.

Certain terms relating to polymer chemistry, polymer compositions, and methods of polymer microparticle or microsphere formation, as used herein, are defined in WO 2013/015685 A1, WO 2005/068533 A1, and WO 2021/066650 A1.

It is understood that wherever aspects are described herein with the language "comprising," otherwise analogous aspects described in terms of "consisting of" and/or "consisting essentially of" are also provided.

It is further understood that headers (e.g., " Method of Producing Stable Microsphere Formulation of Bupivacaine") are provided herein merely for ease of reading, and that wherever aspects are described under a header, the header is not intended to limit the scope of the disclosure. As a non-limiting example, an aspect describing a suitable polymer under a header introducing a pre-filled injector should not be interpreted as limiting the use of that suitable polymer to only pre-filled injectors, unless the aspect itself specifies use of the suitable polymer in a pre-filled injector (e.g., "in some aspects, the pre-filled injector comprises a suitable polymer"). As another non-limiting example, "in some aspects, the antioxidant is sodium metabisulfite" under a header for pre-filled injectors is nevertheless intended to be applicable to methods or compositions unrelated to pre-filled injectors describing the use of one or more antioxidants.

### Microspheres Comprising Bupivacaine free base or a Pharmaceutically acceptable salt of bupivacaine

In some aspects, the present disclosure provides microspheres comprising a first biodegradable polymer and an active drug load of bupivacaine free base or a pharmaceutically acceptable salt of bupivacaine.

In some aspects, the microspheres are prepared by a method described herein. In some aspects, the microspheres comprise one or more polymers described herein.

### Stable Microsphere Formulations of Bupivacaine

In some aspects, the present disclosure provides a stable pharmaceutically acceptable formulation comprising a microsphere, the microsphere comprising a first biodegradable polymer and an active drug load of bupivacaine free base or a pharmaceutically acceptable salt of bupivacaine.

In some aspects, the microsphere comprises an active drug load of bupivacaine free base.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the stable pharmaceutically acceptable formulation further comprises one or more antioxidants. In some aspects, the one or more antioxidants comprises sodium metabisulfite. In some aspects, the antioxidant is sodium metabisulfite. In some aspects, the one or more antioxidants comprises sodium ascorbate. In some aspects, the antioxidant is sodium ascorbate.

**In** some aspects, the microsphere further comprises one or more additional biodegradable polymers.

**In** some aspects, the first biodegradable polymer is a copolymer. In some aspects, the first biodegradable polymer is a multi-block copolymer. In some aspects, the multiblock copolymer comprises at least one hydrolysable prepolymer (A) segment and at least one hydrolysable prepolymer (B) segment, wherein the segments are linked by a multifunctional chain extender, and wherein the segments are randomly and non-alternatingly distributed over the polymer chain.

**In** some aspects, the multiblock copolymer has a T_{g} of about 37 °C or less and a Tₘ of about 50 °C to about 250 °C under physiological conditions, and wherein the prepolymer (A) segment comprises polyethylene glycol.

**In** some aspects, the polyethylene glycol has a Mₙ of about 150 to about 5000 g/mol.

**In** some aspects, the polyethylene glycol has a Mₙ of about 150 to about 5000 g/mol. In some aspects, the polyethylene glycol has a Mₙ of about 200 g/mol to about 1500 g/mol. In some aspects, the polyethylene glycol has a Mₙ of about 600 to about 1000 g/mol. In some aspects, the polyethylene glycol has a Mₙ of about 400 to about 3000 g/mol. In some aspects, the polyethylene glycol has a Mₙ of about 600 to about 1500 g/mol. In some aspects, the polyethylene glycol has a Mₙ of about 600 to about 5000 g/mol. In some aspects, the polyethylene glycol has a Mₙ of about 1000 to about 3000 g/mol.

In some aspects, the multiblock copolymer is amorphous and has a glass transition temperature of 37 °C or less at physiological conditions.

In some aspects, the prepolymer (A) segment and/or the pre-polymer (B) segment comprises one or more linkages selected from the group consisting of: ester linkages, carbonate linkages, anhydride linkages, ether linkages, and combinations thereof. In some aspects, the prepolymer (A) segment comprises one or more polyether groups. In some aspects, the one or more polyether groups are selected from the group consisting of: polyethylene glycol, polyethylene glycol - polypropylene glycol, polytetramethylene ether glycol, and combinations thereof. In some aspects, the polyether group is polyethylene glycol. In some aspects, a polyether is present as an additional prepolymer in the multiblock copolymer.

In some aspects, the prepolymer (A) segment comprises products of a reaction of at least one cyclic monomer with at least one noncyclic initiator selected from the group consisting of diols, dicarboxylic acids and hydroxycarboxylic acids. In some aspects, the at least one cyclic monomer is selected from the group consisting of glycolide, lactide (D and/or L) , ε-caprolactone, δ-valerolactone, trimethylene carbonate, 1,4-dioxan-2-one (para-dioxanone), 1,5-dioxan-2-one, and a cyclic anhydride. In some aspects, the at least one noncyclic initiator is selected from the group consisting of succinic acid, glutaric acid, adipic acid, sebacic acid, lactic acid, glycolic acid, ethylene glycol, diethylene glycol, 1,4butanediol, and 1,6hexanediol.

In some aspects, the pre-polymer (A) segment comprises reactions products of ester forming monomers selected from diols, dicarboxylic acids, and hydroxycarboxylic acids, preferably the pre-polymer (A) segment comprises reaction products of glycolide, lactide (D and/or L) , ε-caprolactone, and/or δ-valerolactone.

In some aspects, the content of prepolymer (A) in the multiblock copolymer is from about 1 % to about 90 % based on total weight of the multiblock copolymer. In some aspects, the content of prepolymer (A) in the multiblock copolymer is from about 2 % to about 80 %. In some aspects, the content of prepolymer (A) in the multiblock copolymer is from about 3 % to about 70 %. In some aspects, the content of prepolymer (A) in the multiblock copolymer is from about 4 % to about 60 %. In some aspects, the content of prepolymer (A) in the multiblock copolymer is from about 5 % to about 50 %. In some aspects, the content of prepolymer (A) in the multiblock copolymer is from about 6 % to about 40 %. In some aspects, the content of prepolymer (A) in the multiblock copolymer is from about 8 % to about 30 %. In some aspects, the content of prepolymer (A) in the multiblock copolymer is from about 10 % to about 20 %.

In some aspects, the prepolymer (A) segment has a Mₙ of about 500 g/mol or more. In some aspects, the prepolymer (A) segment has a Mₙ of about 700 g/mol or more. In some aspects, the prepolymer (A) segment has a Mₙ of about 1000 g/mol or more. In some aspects, the prepolymer (A) segment has a Mₙ of about 2000 g/mol or more. In some aspects, the prepolymer (A) segment has a Mₙ of about 3000 g/mol or more. In some aspects, the prepolymer (A) segment has a Mₙ of about about 4000 g/mol or more.

In some aspects, the prepolymer (B) segment comprises a polymer derived from hydroxyalkanoate, glycolide, lactide (D and/or L), ε-caprolactone, δ-valerolactone, trimethylene carbonate, 1,4-dioxane-2-one (p-dioxanone) or combinations thereof.

In some aspects, the prepolymer (B) segment comprises poly(-glycolide-co- L-lactide). In some aspects-, the prepolymer (B) segment comprises poly(-glycolide-co-L-lactide)with a Mₙ of about 1000 g/mol or more. In some aspects, the prepolymer (B) segment comprises poly(-glycolide-co- L-lactide) with a Mₙ of about 2000 g/mol or more. In some aspects, the prepolymer (B) segment comprises poly(-glycolide-co-L-lactide) with a Mₙ of about 3000 g/mol or more. In some aspects, the prepolymer (B) segment comprises poly(-glycolide-co- L-lactide) with a Mₙ of about 4000 g/mol.

In some aspects, the prepolymer (B) segment comprises a molar amount of about 1% to about 90% of glycolide relative to combined molar amount of glycolide and L-lactide. In some aspects, the prepolymer (B) segment comprises a molar amount of about 2% to about 50%. In some aspects, the prepolymer (B) segment comprises a molar amount of about 5% to about 30%. In some aspects, the prepolymer (B) segment comprises a molar amount of about 10% to about 20%. In some aspects, the prepolymer (B) segment comprises a molar amount of about 5% to about 25% of glycolide relative to combined molar amount of glycolide and L-lactide. In some aspects, the prepolymer (B) segment comprises a molar amount of about 15% of glycolide relative to the combined molar amount of glycolide and L-lactide.

In some aspects, the prepolymer (B) segment comprises poly(p-dioxanone). In some aspects, the prepolymer (B) segment comprises poly(p-dioxanone) with a Mₙ of about 1000 g/mol or more. In some aspects, the prepolymer (B) segment comprises poly(p-dioxanone) with a Mₙ of about 1500 g/mol or more. In some aspects, the prepolymer (B) segment comprises poly(p-dioxanone) with a Mₙ of about 2000 g/mol or more. In some aspects, the prepolymer (B) segment comprises poly(p-dioxanone) with a Mₙ of about 2500 g/mol or more. In some aspects, the prepolymer (B) segment comprises poly(p-dioxanone) with a Mₙ of about 3000 g/mol or more. In some aspects, the prepolymer (B) segment comprises poly(p-dioxanone) with a Mₙ of about 3500 g/mol or more. In some aspects, the prepolymer (B) segment comprises poly(p-dioxanone) with a Mₙ of about 4000 g/mol or more.

In some aspects, the multiblock copolymer comprises from about 10% to about 99% of the prepolymer (B) segment relative to the total weight of the multiblock copolymer.

In some aspects, the multifunctional chain extender is a difunctional aliphatic chain extender. In some aspects, the difunctional aliphatic chain extender is a diisocyanate. In some aspects, the diisocyanate is 1,4-butane diisocyanate.

In some aspects, the first biodegradable polymer is selected from the group consisting of: a polylactide, a polyglycolide, and a poly(lactide-co-glycolide) copolymer.

In some aspects, the first biodegradable polymer is a poly(lactide-co-glycolide) copolymer, where the content of polymerized lactic acid units is at least 50 mol% based on the total amount of polymerized units or blends of poly(lactide-co-glycolide) copolymers with different relative amounts of glycolic acid and lactic acid, where the content of polymerized lactic acid units is at least 50 mol% based on the total amount of polymerized units.

In some aspects, the first biodegradable polymer is substantially enclosed by a second biodegradable polymer.

In some aspects, the first biodegradable polymer is not substantially identical in composition to the second biodegradable polymer.

In some aspects, the stable pharmaceutically acceptable formulation further comprises a diluent.

In some aspects, the active drug load of bupivacaine free base or a pharmaceutically acceptable salt of bupivacaine in the microspheres is about 10% to about 80% w/w. In some aspects, the active drug load of bupivacaine free base or a pharmaceutically acceptable salt of bupivacaine in the microspheres is about 30% to about 70% w/w. In some aspects, the active drug load of bupivacaine free base or a pharmaceutically acceptable salt of bupivacaine in the microspheres is about 50% to about 60% w/w. In some aspects, the active drug load of bupivacaine free base or a pharmaceutically acceptable salt of bupivacaine in the microspheres is about 40% to about 50% w/w. In some aspects, the active drug load of bupivacaine free base or a pharmaceutically acceptable salt of bupivacaine in the microspheres is about 30% w/w to about 40% w/w. In some aspects, the active drug load of bupivacaine free base or a pharmaceutically acceptable salt of bupivacaine in the microspheres is about 20% w/w to about 50% w/w. In some aspects, the active drug load of bupivacaine free base or a pharmaceutically acceptable salt of bupivacaine in the microspheres is about 30% w/w, about 31% w/w, about 32% w/w, about 33% w/w, about 34% w/w, about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 59% w/w, about 60% w/w, about 61% w/w, about 62% w/w, about 63% w/w, about 64% w/w, about 65% w/w, about 67% w/w, about 68% w/w, about 69% w/w, or about 70% w/w. In some aspects, the active drug load of bupivacaine free base or a pharmaceutically acceptable salt of bupivacaine in the microspheres is about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, about 71% w/w, about 72% w/w, about 73% w/w, about 74% w/w, about 75% w/w, about 76% w/w, about 77% w/w, about 78% w/w, about 79% w/w, or about 80% w/w. In some aspects, the active drug load of bupivacaine free base or a pharmaceutically acceptable salt of bupivacaine in the microspheres is about 40% w/w. In some aspects, the active drug load of bupivacaine free base or a pharmaceutically acceptable salt of bupivacaine in the microspheres is about 50% w/w.

In some aspects, the active drug load of bupivacaine free base or a pharmaceutically acceptable salt of bupivacaine in the microspheres is about 200 mg to about 8000 mg. In some aspects, the active drug load of bupivacaine free base or a pharmaceutically acceptable salt of bupivacaine in the microspheres is about 500 mg to about 1000 mg. In some aspects, the active drug load of bupivacaine free base or a pharmaceutically acceptable salt of bupivacaine in the microspheres is about 250 mg to about 1200 mg. In some aspects, the active drug load of bupivacaine free base or a pharmaceutically acceptable salt of bupivacaine in the microspheres is about 600 mg to about 800 mg. In some aspects, the active drug load of bupivacaine free base or a pharmaceutically acceptable salt of bupivacaine in the microspheres is about 1000 mg to about 3000 mg. In some aspects, the active drug load of bupivacaine free base or a pharmaceutically acceptable salt of bupivacaine in the microspheres is about 3000 mg to about 6000 mg. In some aspects, the active drug load of bupivacaine free base or a pharmaceutically acceptable salt of bupivacaine in the microspheres is about 5000 mg to about 8000 mg. In some aspects, the active drug load of bupivacaine free base or a pharmaceutically acceptable salt of bupivacaine in the microspheres is about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. In some aspects, the active drug load of bupivacaine free base or a pharmaceutically acceptable salt of bupivacaine in the microspheres is about 200 mg, about 2000 mg, about 3000 mg, about 4000 mg, about 5000 mg, about 6000 mg, about 7000 mg, or about 8000 mg. In some aspects, the active drug load of bupivacaine free base or a pharmaceutically acceptable salt of bupivacaine in the microspheres is about 800 mg. In some aspects, the active drug load of bupivacaine free base or a pharmaceutically acceptable salt of bupivacaine in the microspheres is about 1000 mg.

In some aspects, the stable pharmaceutically acceptable formulation is substantially sterile.

In some aspects, the stable pharmaceutically acceptable formulation comprises less than 5% w/w of a bupivacaine-derived impurity after sealed storage for 24 months at a temperature of 25°C. In some aspects, the stable pharmaceutically acceptable formulation comprises less than 2% w/w of a bupivacaine-derived impurity after sealed storage for 24 months at a temperature of 25°C. In some aspects, the stable pharmaceutically acceptable formulation comprises less than 1% w/w of a bupivacaine-derived impurity after sealed storage for 24 months at a temperature of 25°C.

In some aspects, the stable pharmaceutically acceptable formulation is substantially free of a bupivacaine-derived impurity after sealed storage for 24 months at a temperature of 25°C.

In some aspects, the bupivacaine-derived impurity is a bupivacaine oxidation product.

In some aspects, the stable pharmaceutically acceptable formulation has a shelf life of about 14 days at 25°C following refrigeration. In some aspects, the stable pharmaceutically acceptable formulation has a shelf life of about 7-21 days at 25°C following refrigeration. In some aspects, the stable pharmaceutically acceptable formulation has a shelf life of about 7 days, about 8 days, about 9 days, about 10 days, about 11 days, about 12 days, about 13 days, about 14 days, about 15 days, about 16 days, about 17 days, about 18 days, about 19 days, about 20 days, or about 21 days at 25°C following refrigeration.

In some aspects, the stable pharmaceutically acceptable formulation has a shelf life of about 24 months at 25°C. In some aspects, the stable pharmaceutically acceptable formulation has a shelf life of about 12 months to about 36 months at 25°C. In some aspects, the stable pharmaceutically acceptable formulation has a shelf life of about 12 months, about 13 months, about 14 months, about 15 months, about 16 months, about 17 months, about 18 months, about 19 months, about 20 months, about 21 months, about 22 months, about 23 months, about 24 months, about 25 months, about 26 months, about 27 months, about 28 months, about 29 months, about 30 months, about 31 months, about 32 months, about 33 months, about 34 months, about 35 months, or about 36 months at 25°C.

In some aspects, the stable pharmaceutically acceptable formulation is stored in a sterile container of about 3 mL to about 20 mL capacity. In some aspects, the stable pharmaceutically acceptable formulation is stored in a sterile container of about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 16 mL, about 17 mL, about 18 mL, about 19 mL, or about 20 mL capacity.

In some aspects, the stable pharmaceutically acceptable formulation is provided in an injector.

In some aspects, the injector is a disposable pen injector.

In some aspects, the stable pharmaceutically acceptable formulation is suitable for parenteral administration.

In some aspects, the stable pharmaceutically acceptable formulation is suitable for subcutaneous administration. In some aspects, the stable pharmaceutically acceptable formulation is suitable for intradermal administration. In some aspects, the stable pharmaceutically acceptable formulation is suitable for intraperitoneal administration. In some aspects, the stable pharmaceutically acceptable formulation is suitable for administration by local instillation. In some aspects, the stable pharmaceutically acceptable formulation is suitable for local administration.

In some aspects, the stable pharmaceutically acceptable formulation is suitable for intramuscular administration.

In some aspects, a single dose is divided among multiple administrable volumes.

In some aspects, the amount of bupivacaine free base or a pharmaceutically acceptable salt of bupivacaine per dose is about 200 mg to about 8000 mg. In some aspects, the amount of bupivacaine free base or a pharmaceutically acceptable salt of bupivacaine per dose is about 500 mg to about 1000 mg. In some aspects, the amount of bupivacaine free base or a pharmaceutically acceptable salt of bupivacaine per dose is about 250 mg to about 1200 mg. In some aspects, the amount of bupivacaine free base or a pharmaceutically acceptable salt of bupivacaine per dose is about 600 mg to about 800 mg. In some aspects, the amount of bupivacaine free base or a pharmaceutically acceptable salt of bupivacaine per dose is about 1000 mg to about 3000 mg. In some aspects, the amount of bupivacaine free base or a pharmaceutically acceptable salt of bupivacaine per dose is about 3000 mg to about 6000 mg. In some aspects, the amount of bupivacaine free base or a pharmaceutically acceptable salt of bupivacaine per dose is about 5000 mg to about 8000 mg. In some aspects, the amount of bupivacaine free base or a pharmaceutically acceptable salt of bupivacaine per dose is about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. In some aspects, the amount of bupivacaine free base or a pharmaceutically acceptable salt of bupivacaine per dose is about 200 mg, about 2000 mg, about 3000 mg, about 4000 mg, about 5000 mg, about 6000 mg, about 7000 mg, or about 8000 mg. In some aspects, the amount of bupivacaine free base or a pharmaceutically acceptable salt of bupivacaine per dose is about 800 mg. In some aspects, the amount of bupivacaine free base or a pharmaceutically acceptable salt of bupivacaine per dose is about 1000 mg.

In some aspects, the stable pharmaceutically acceptable formulation is provided as a stock from which individual doses of the stable pharmaceutically acceptable formulation can be withdrawn. In some aspects, the stock is provided in a volume of about 5 mL to about 2 L. In some aspects, the stock is provided in a volume of about 5 mL to about 100 mL. In some aspects, the stock is provided in a volume of about 5 mL to about 50 mL. In some aspects, the stock is provided in a volume of about 5 mL to about 10 mL. In some aspects, the stock is provided in a volume of about 500 mL to about 1.5 L. In some aspects, the stock is provided in a volume of about 5 mL, about 6 mL, about 7 mL, about 8 mL, about 9 mL, about 10 mL, about 15 mL, about 20 mL, about 25 mL, about 30 mL, about 35 mL, about 40 mL, about 45 mL, about 50 mL, about 100 mL, about 150 mL, about 200 mL, about 250 mL, about 300 mL, about 350 mL, about 400 mL, about 450 mL, about 500 mL, about 550 mL, about 600 mL, about 700 mL, about 750 mL, about 800 mL, about 900 mL, about 1 L, about 1.1 L, about 1.2 L, about 1.3 L, about 1.4 L, about 1.5 L, about 1.6 L, about 1.7 L, about 1.8 L, about 1.9 L, or about 2 L.

### Method of Producing Stable Microsphere Formulations of Bupivacaine

In some aspects, the present disclosure provides a method of producing a stable pharmaceutically acceptable formulation comprising a microsphere. In a further aspect, the method comprises providing a first phase, the first phase comprising: a first biodegradable polymer; and an active drug load of bupivacaine free base or a pharmaceutically acceptable salt of bupivacaine. In a further aspect, the method comprises adding a second phase comprising an aqueous surfactant continuously into the first phase to form an emulsion. In a further aspect, the method comprises adding a quench solution to the emulsion to produce a volume comprising a microsphere. In a further aspect, the method comprises washing, filtering, and drying the microsphere to reduce solvent content.

In some aspects, the present disclosure provides a method of producing a stable pharmaceutically acceptable formulation comprising microspheres. In a further aspect, the method comprises providing a first phase comprising: a first biodegradable polymer and a solvent system suitable to dissolve the polymer, wherein bupivacaine free base or a pharmaceutically acceptable salt of bupivacaine is dispersed but not substantially dissolved in the solvent system. In a further aspect, the method comprises combining the first phase with a second phase comprising an aqueous solution, which comprises a surfactant, thereby forming an emulsion.

In some aspects, the present disclosure provides a method of producing a stable pharmaceutically acceptable formulation comprising microspheres. In a further aspect, the method comprises providing a first phase comprising: a first biodegradable polymer; an active drug load of bupivacaine free base or a pharmaceutically acceptable salt of bupivacaine; and a solvent system suitable to dissolve the polymer and bupivacaine. In a further aspect, the method comprises emulsifying the first phase with a second phase, thereby forming an emulsion. In a further aspect, the second phase comprises an aqueous solution, which comprises a surfactant. In a further aspect, the method comprises removing a substantial portion of the solvent system from the emulsion, thereby obtaining microspheres.

In some aspects, the method comprises collecting and drying the microspheres.

In some aspects, emulsification comprises membrane emulsification. In some aspects, the step of emulsifying the first phase with the second phase comprises membrane emulsification of the first phase into the second phase. In some aspects, the step of emulsifying the first phase with the second phase comprises membrane emulsification using a membrane through which the first phase is introduced into the second phase.

In some aspects, the step of removing the substantial portion of the solvent system from the emulsion comprises extraction of the solvent system by the aqueous solution that comprises a surfactant. In some aspects, the step of removing the substantial portion of the solvent system from the emulsion comprises extraction of a first part of the solvent system by the aqueous solution that comprises a surfactant, followed by evaporation of a second part of the solvent system.

In some aspects, the substantial portion of the solvent system is about 80%, about 81%, about 82%, about 83%, about 84%, about 85%, about 86%, about 87%, about 88%, about 89%, about 90%, about 91%, about 92%, about 93%, about 95%, about 95%, about 96%, about 97%, about 98%, about 99%, or about 100% of the solvent system in the emulsion.

In some aspects, the method further comprises washing and/or filtering the microspheres.

In some aspects, the method further comprises drying the microspheres. In some aspects, the drying of the microspheres comprises one or more of lyophilization, vacuum-drying, and freeze-vacuum drying.

In some aspects, the method comprises a step of hardening the microspheres.

In some aspects, the first phase comprises bupivacaine free base.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the first phase comprises a solvent system in which bupivacaine free base or a pharmaceutically acceptable salt of bupivacaine has a solubility of about 25 mg/mL or more. In some aspects, the first phase comprises a solvent system in which bupivacaine free base or a pharmaceutically acceptable salt of bupivacaine has a solubility of about 50 mg/mL or more. In some aspects, the first phase comprises a solvent system in which bupivacaine free base or a pharmaceutically acceptable salt of bupivacaine has a solubility of about 100 mg/mL or more. In some aspects, the first phase comprises a solvent system in which bupivacaine free base or a pharmaceutically acceptable salt of bupivacaine has a solubility of about 200 mg/mL or more.

In some aspects, the first phase comprises one or more solvents selected from the group consisting of: dichloromethane, ethyl acetate, chloroform, methanol, benzyl alcohol, dimethyl formamide, dimethyl sulfoxide, N-methyl pyrrolidone, and dimethyl acetamide. In some aspects, the first phase comprises dichloromethane. In some aspects, the first phase comprises ethyl acetate. In some aspects, the first phase comprises chloroform. In some aspects, the first phase comprises methanol. In some aspects, the first phase comprises benzyl alcohol. In some aspects, the first phase comprises dimethyl formamide. In some aspects, the first phase comprises dimethyl sulfoxide. In some aspects, the first phase comprises N-methyl pyrrolidone. In some aspects, the first phase comprises dimethyl acetamide.

In some aspects, the first phase comprises a first solvent and a second solvent. In some aspects, the first solvent is present in an amount of about 1% to about 99% relative to the combined volume of the first solvent and the second solvent. In some aspects, the first solvent is present in an amount of about 5% to about 50% relative to the combined volume of the first solvent and the second solvent. In some aspects, the first solvent is present in an amount of about 10% to about 40% relative to the combined volume of the first solvent and the second solvent. In some aspects, the first solvent is present in an amount of about 15% to about 30% relative to the combined volume of the first solvent and the second solvent. In some aspects, the first solvent is present in an amount of about 20% to about 25% relative to the combined volume of the first solvent and the second solvent. In some aspects, the first solvent is present in an amount of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% relative to the combined volume of the first solvent and the second solvent.

In some aspects, the first phase comprises dichloromethane in combination with one or more additional solvents. In some aspects, the one or more additional solvents are selected from the group consisting of: dimethyl formamide, dimethyl sulfoxide, and N-methyl pyrrolidone. In some aspects, the first phase comprises dichloromethane and dimethyl sulfoxide. In some aspects, dimethyl sulfoxide is present in an amount of about 5% to about 50% relative to the combined volume of dimethyl sulfoxide and dichloromethane in the first phase. In some aspects, dimethyl sulfoxide is present in an amount of about 10% to about 40% relative to the combined volume of dimethyl sulfoxide and dichloromethane in the first phase. In some aspects, dimethyl sulfoxide is present in an amount of about 15% to about 30% relative to the combined volume of dimethyl sulfoxide and dichloromethane in the first phase. In some aspects, dimethyl sulfoxide is present in an amount of about 20% to about 25% relative to the combined volume of dimethyl sulfoxide and dichloromethane in the first phase.

In some aspects, the first phase comprises about 2% to about 25% by weight of the combined mass of the first biodegradable polymer and the one or more additional biodegradable polymers.

In some aspects, the first phase comprises about 2% to about 25% by weight of bupivacaine free base or a pharmaceutically acceptable salt of bupivacaine.

In some aspects, the first phase and/or the second phase comprises one or more stabilizers. In some aspects, the first phase and/or the second phase comprises one or more buffering agents. In some aspects, the first phase and/or the second phase comprises one or more preservatives.

In some aspects, the first phase comprises one or more antioxidants. In some aspects, the one or more antioxidants comprises sodium metabisulfite or sodium ascorbate. In some aspects, the one or more antioxidants comprises sodium metabisulfite or sodium ascorbate in an amount from about 0.01% to about 5% w/v relative to the first phase. In some aspects, the one or more antioxidants comprises sodium metabisulfite or sodium ascorbate in an amount from about 0.05% to about 2% w/v relative to the first phase. In some aspects, the one or more antioxidants comprises sodium metabisulfite or sodium ascorbate in an amount from about 0.1% to about 1% w/v relative to the first phase.

In some aspects, the second phase comprises polyvinyl alcohol.

In some aspects, the second phase comprises sodium chloride.

In some aspects, the second phase comprises an aqueous buffer.

In some aspects, the second phase is buffered to a pH of about 4 to about 10. In some aspects, the second phase is buffered to a pH of about 7 to about 10. In some aspects, the second phase is buffered to a pH of about 8.5.

In some aspects, the second phase comprises a TRIS buffer. In some aspects, the second phase comprises TRIZMA pellets.

In some aspects, the second phase comprises one or more antioxidants.

In some aspects, the one or more antioxidants comprises sodium metabisulfite. In some aspects, the one or more antioxidants comprises sodium metabisulfite in an amount of about 0.1% to about 1% w/v in the second phase. In some aspects, the one or more antioxidants comprises sodium metabisulfite in an amount of about 0.15% w/v in the second phase. In some aspects, the one or more antioxidants comprises sodium metabisulfite in an amount of about 0.1%, about 0.2%, about 0.3%, about 0.4%, about 0.5%, about 0.6%, about 0.7%, about 0.8%, about 0.9%, or about 1.0% w/v in the second phase.

In some aspects, the one or more antioxidants comprises sodium ascorbate. In some aspects, the one or more antioxidants comprises sodium ascorbate in an amount of about 0.1% to about 1% w/v in the second phase. In some aspects, the one or more antioxidants comprises sodium ascorbate in an amount of about 0.15% w/v in the second phase. In some aspects, the one or more antioxidants comprises sodium ascorbate in an amount of about 0.1%, about 0.2%, about 0.3%, about 0.4%, about 0.5%, about 0.6%, about 0.7%, about 0.8%, about 0.9%, or about 1.0% w/v in the second phase.

In some aspects, the first biodegradable polymer is selected from the group consisting of: a polylactide, a polyglycolide, and a poly(lactide-co-glycolide) copolymer.

In some aspects, the first biodegradable polymer is a poly(lactide-co-glycolide) copolymer, where the content of polymerized lactic acid units is at least 50 mol% based on the total amount of polymerized units or blends of poly(lactide-co-glycolide) copolymers with different relative amounts of glycolic acid and lactic acid, where the content of polymerized lactic acid units is at least 50 mol% based on the total amount of polymerized units.

In some aspects, the aqueous surfactant comprises one or more of a cationic surfactant, an anionic surfactant, or a non-ionic surfactant.

In some aspects, the second phase further comprises one or more of: a buffer solution, one or more agents for adjusting the viscosity of the aqueous surfactant, and an agent for adjusting the ionic strength of the solution.

In some aspects, the first phase is stirred prior to and/or during the addition of the second phase.

In some aspects, the emulsion is stirred prior to and/or during the addition of the quench solution.

In some aspects, the volume comprising a microsphere is stirred prior to and/or during any of the steps of washing, filtering and drying the microsphere.

In some aspects, the first biodegradable polymer is substantially enclosed by a second biodegradable polymer.

In some aspects, the first biodegradable polymer is not identical in composition to the second biodegradable polymer.

In some aspects, the stable pharmaceutically acceptable formulation further comprises a diluent.

In some aspects, the first phase is prepared by mixing a solution comprising the first biodegradable polymer with a solution comprising the bupivacaine free base or a pharmaceutically acceptable salt of bupivacaine.

In some aspects, the first biodegradable polymer and/or the second biodegradable polymer is dissolved in a solvent highly or fully miscible with water selected from the group consisting of: dimethyl sulfoxide, N-methyl-2-pyrrolidone, tetrahydrofuran, tetraglycol, acetone, an acetone/methyl ethyl ketone mixture, an acetone/methyl acetate mixture, a tetrahydrofuran/ethyl acetate mixture, and a tetrahydrofuran/ethyl formate mixture.

In some aspects, the solvent highly or fully miscible with water is an acetone/methyl ethyl ketone mixture.

In some aspects, the acetone/methyl ethyl ketone mixture comprises about 70% acetone and about 30% methyl ethyl ketone, by volume. In some aspects, the acetone/methyl ethyl ketone mixture comprises about 60% to about 80% acetone and about 20% to about 40% methyl ethyl ketone, by volume.

In some aspects, the first biodegradable polymer and/or the second biodegradable polymer is dissolved in a solvent having limited water solubility selected from the group consisting of: ethyl acetate, methyl acetate, ethyl formate, propyl formate, isopropyl formate, methyl ethyl ketone, and a mixture of two or more thereof.

### Pre-Filled Injector

In some aspects, the present disclosure provides a pre-filled injector, the pre-filled injector comprising a stable pharmaceutically acceptable formulation. In a further aspect, the formulation comprises microspheres. In a further aspect, the microspheres comprises a first biodegradable polymer and an active drug load of bupivacaine free base or a pharmaceutically acceptable salt of bupivacaine.

In some aspects, the pre-filled injector is configurable to administer more than one injection.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the first biodegradable polymer is selected from the group consisting of: a polylactide, a polyglycolide, and a poly(lactide-co-glycolide) copolymer.

In some aspects, the first biodegradable polymer is a poly(lactide-co-glycolide) copolymer, wherein the content of polymerized lactic acid units is at least 50 mol% based on the total amount of polymerized units or blends of poly(lactide-co-glycolide) copolymers with different relative amounts of glycolic acid and lactic acid, wherein the content of polymerized lactic acid units is at least 50 mol% based on the total amount of polymerized units.

In some aspects, the first biodegradable polymer is substantially enclosed by a second biodegradable polymer.

In some aspects, the first biodegradable polymer is not identical in composition to the second biodegradable polymer.

In some aspects, the active drug load of bupivacaine free base or a pharmaceutically acceptable salt of bupivacaine in the microspheres is about 10% to about 80% w/w. In some aspects, the active drug load of bupivacaine free base or a pharmaceutically acceptable salt of bupivacaine in the microspheres is about 30% to about 70% w/w. In some aspects, the active drug load of bupivacaine free base or a pharmaceutically acceptable salt of bupivacaine in the microspheres is about 50% to about 60% w/w. In some aspects, the active drug load of bupivacaine free base or a pharmaceutically acceptable salt of bupivacaine in the microspheres is about 40% to about 50% w/w. In some aspects, the active drug load of bupivacaine free base or a pharmaceutically acceptable salt of bupivacaine in the microspheres is about 30% to about 40% w/w. In some aspects, the active drug load of bupivacaine free base or a pharmaceutically acceptable salt of bupivacaine in the microspheres is about 30%, about 35%, about 40%, about 45%, about 50%, about 55%, about 60%, about 65%, or about 70% w/w. In some aspects, the active drug load of bupivacaine free base or a pharmaceutically acceptable salt of bupivacaine in the microspheres is about 10%, about 15%, about 20%, about 25%, about 75%, or about 80% w/w.

In some aspects, the active drug load of bupivacaine free base or a pharmaceutically acceptable salt of bupivacaine in the microspheres is about 50% w/w or less. In some aspects, the active drug load of bupivacaine free base or a pharmaceutically acceptable salt of bupivacaine in the microspheres is about 40% w/w or less. In some aspects, the active drug load of bupivacaine free base or a pharmaceutically acceptable salt of bupivacaine in the microspheres is about 30% w/w or less. In some aspects, the active drug load of bupivacaine free base or a pharmaceutically acceptable salt of bupivacaine in the microspheres is about 20% w/w or less. In some aspects, the active drug load of bupivacaine free base or a pharmaceutically acceptable salt of bupivacaine in the microspheres is about 10% w/w or less. In some aspects, the active drug load of bupivacaine free base or a pharmaceutically acceptable salt of bupivacaine in the microspheres is about 50% w/w or more. In some aspects, the active drug load of bupivacaine free base or a pharmaceutically acceptable salt of bupivacaine in the microspheres is about 40% w/w or more. In some aspects, the active drug load of bupivacaine free base or a pharmaceutically acceptable salt of bupivacaine in the microspheres is about 30% w/w or more. In some aspects, the active drug load of bupivacaine free base or a pharmaceutically acceptable salt of bupivacaine in the microspheres is about 20% w/w or more. In some aspects, the active drug load of bupivacaine free base or a pharmaceutically acceptable salt of bupivacaine in the microspheres is about 10% w/w or more. In some aspects, the active drug load of bupivacaine free base or a pharmaceutically acceptable salt of bupivacaine in the microspheres is about 1% w/w, about 2% w/w, about 3% w/w, about 4% w/w, about 5% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, about 30% w/w, about 35% w/w, about 40% w/w, about 45% w/w, about 50% w/w, or about 55% w/w.

In some aspects, the stable pharmaceutical formulation is substantially sterile. In some aspects, the stable pharmaceutically acceptable formulation is sterile.

In some aspects, the stable pharmaceutical formulation has a shelf life of about 14 days at 25 °C following refrigeration. In some aspects, the stable pharmaceutical formulation has a shelf life of about 7-21 days at 25 °C following refrigeration. In some aspects, the stable pharmaceutical formulation has a shelf life of about 7 days, about 8 days, about 9 days, about 10 days, about 11 days, about 12 days, about 13 days, about 14 days about 15 days, about 16 days, about 17 days, about 18 days about 19 days, about 20 days, or about 21 days at 25 °C following refrigeration.

In some aspects, the stable pharmaceutical formulation has a shelf life of about 24 months at 25 °C. In some aspects, the stable pharmaceutical formulation has a shelf life of about 12-36 months at 25 °C. In some aspects, the stable pharmaceutical formulation has a shelf life of about 12 months, about 13 months, about 14 months, about 15 months, about 16 months, about 17 months, about 18 months, about 19 months, about 20 months, about 21 months, about 22 months, about 23 months, or about 24 months at 25 °C.

In some aspects, the pre-filled injector is a pen injector or an autoinjector.

In some aspects, the pre-filled injector is disposable.

In some aspects, the pre-filled injector is a disposable pen injector.

In some aspects, the pre-filled injector comprises a cartridge comprising the stable pharmaceutically acceptable formulation.

In some aspects, the cartridge is a dual chamber cartridge.

In some aspects, the dual chamber cartridge comprises, in a first chamber, a first volume comprising a microsphere, the microsphere comprising an active drug load of bupivacaine free base or a pharmaceutically acceptable salt of bupivacaine. In some aspects, the dual chamber cartridge comprises, in a second chamber, a second volume comprising a dilution medium. In some aspects, the first volume further comprises an amount of bupivacaine free base or a pharmaceutically acceptable salt of bupivacaine which is not contained within a microsphere. In some aspects, the second volume further comprises an amount of bupivacaine free base or a pharmaceutically acceptable salt of bupivacaine.

In some aspects, the first volume and the second volume can be combined to produce a pharmaceutically acceptable formulation disclosed herein.

In some aspects, the pre-filled injector is configurable to combine the first volume and the second volume prior to injection. In some aspects, the pre-filled injector is configurable to combine the first volume and the second volume during injection.

In some aspects, the pre-filled injector is configurable to dispense the first volume and the second volume in about equal amounts by volume. In some aspects, the pre-filled injector is configurable to dispense the first volume and the second volume in amounts that are not equal by volume.

In some aspects, the first volume has a shelf life of about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 days at 25 °C following refrigeration. In some aspects, the first volume has a shelf life of about 7 days at 25 °C following refrigeration. In some aspects, the first volume has a shelf life of about 14 days at 25 °C following refrigeration. In some aspects, the first volume has a shelf life of about 21 days at 25 °C following refrigeration.

In some aspects, the first volume has a shelf life of about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, or about 24 months at 25 °C. In some aspects, the first volume has a shelf life of about 3 months at 25 °C. In some aspects, the first volume has a shelf life of about 6 months at 25 °C. In some aspects, the first volume has a shelf life of about 12 months at 25 °C.

In some aspects, the second volume has a shelf life of about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 days at 25 °C following refrigeration. In some aspects, the second volume has a shelf life of about 7 days at 25 °C following refrigeration. In some aspects, the second volume has a shelf life of about 14 days at 25 °C following refrigeration. In some aspects, the second volume has a shelf life of about 21 days at 25 °C following refrigeration.

In some aspects, the second volume has a shelf life of about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, or about 24 months at 25 °C. In some aspects, the second volume has a shelf life of about 3 months at 25 °C. In some aspects, the second volume has a shelf life of about 6 months at 25 °C. In some aspects, the second volume has a shelf life of about 12 months at 25 °C.

In some aspects, the pre-filled injector comprises a 18G to 30G needle. In some aspects, the pre-filled injector comprises a 21G needle. In some aspects, the pre-filled injector comprises a 23G needle. In some aspects, the pre-filled injector comprises a 25G needle.

In some aspects, the pre-filled injector does not comprise a needle.

In some aspects, the amount of the stable pharmaceutically acceptable formulation dispensed in a single injection or more than one injection contains about 3000 mg to about 8000 mg of bupivacaine free base or a pharmaceutically acceptable salt of bupivacaine.

In some aspects, the amount of the stable pharmaceutically acceptable formulation dispensed in a single injection or more than one injection contains about 500 mg to about 1000 mg of bupivacaine free base or a pharmaceutically acceptable salt of bupivacaine. In some aspects, the amount of the stable pharmaceutically acceptable formulation dispensed in a single injection or more than one injection contains about 250 mg to about 1200 mg of bupivacaine free base or a pharmaceutically acceptable salt of bupivacaine. In some aspects, the amount of the stable pharmaceutically acceptable formulation dispensed in a single injection or more than one injection contains about 600 mg to about 800 mg of bupivacaine free base or a pharmaceutically acceptable salt of bupivacaine. In some aspects, the amount of the stable pharmaceutically acceptable formulation dispensed in a single injection or more than one injection contains about 200 mg to about 8000 mg of bupivacaine free base or a pharmaceutically acceptable salt of bupivacaine. In some aspects, the amount of the stable pharmaceutically acceptable formulation dispensed in a single injection or more than one injection contains about 500 mg to about 4000 mg of bupivacaine free base or a pharmaceutically acceptable salt of bupivacaine. In some aspects, the amount of the stable pharmaceutically acceptable formulation dispensed in a single injection or more than one injection contains about 1000 mg to about 2000 mg of bupivacaine free base or a pharmaceutically acceptable salt of bupivacaine. In some aspects, the amount of the stable pharmaceutically acceptable formulation dispensed in a single injection or more than one injection contains about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 300 mg, about 400 mg, about 500 mg, about 600 mg, about 700 mg, about 800 mg, about 900 mg, about 1000 mg, about 1200 mg, about 1400 mg, about 1600 mg, about 1800 mg, about 2000 mg, about 2500 mg, about 3000 mg, about 3500 mg, or about 4000 mg of bupivacaine free base or a pharmaceutically acceptable salt of bupivacaine. In some aspects, the amount of the stable pharmaceutically acceptable formulation dispensed in a single injection or more than one injection contains about 5000 mg, about 6000 mg, about 7000 mg, or about 8000 mg of bupivacaine free base or a pharmaceutically acceptable salt of bupivacaine. In some aspects, the amount of the stable pharmaceutical formulation dispensed in a single injection or more than one injection contains about 4000 mg of bupivacaine free base or a pharmaceutically acceptable salt of bupivacaine.

### Method of Manufacturing Pre-Filled Injector

In some aspects, the present disclosure provides a method of manufacturing a pre-filled injector comprising a stable pharmaceutically acceptable formulation. In a further aspect, the method comprises preparing a stable pharmaceutically acceptable formulation. In a further aspect, the formulation comprises microspheres. In a further aspect, the microspheres comprise a first biodegradable polymer and an active drug load of bupivacaine free base or a pharmaceutically acceptable salt of bupivacaine. In a further aspect, the method comprises loading a sterile cartridge with the stable pharmaceutically acceptable formulation. In a further aspect, the method comprises attaching the sterile cartridge operably to an injector.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the first biodegradable polymer is selected from the group consisting of: a polylactide, a polyglycolide, and a poly(lactide-co-glycolide) copolymer. In some aspects, the first biodegradable polymer comprises one or more of: a polylactide, a polyglycolide, a poly(lactide-co-glycolide) copolymer.

In some aspects, the first biodegradable polymer is a poly(lactide-co-glycolide) copolymer, wherein the content of polymerized lactic acid units is at least 50 mol% based on the total amount of polymerized units or blends of poly(lactide-co-glycolide) copolymers with different relative amounts of glycolic acid and lactic acid, wherein the content of polymerized lactic acid units is at least 50 mol% based on the total amount of polymerized units.

In some aspects, the first biodegradable polymer is substantially enclosed by a second biodegradable polymer.

In some aspects, the first biodegradable polymer is not identical in composition to the second biodegradable polymer.

In some aspects, the active drug load of bupivacaine free base or a pharmaceutically acceptable salt of bupivacaine in the microspheres is about 10% to about 80% w/w. In some aspects, the active drug load of bupivacaine free base or a pharmaceutically acceptable salt of bupivacaine in the microspheres is about 30% to about 70% w/w. In some aspects, the active drug load of bupivacaine free base or a pharmaceutically acceptable salt of bupivacaine in the microspheres is about 50% to about 60% w/w. In some aspects, the active drug load of bupivacaine free base or a pharmaceutically acceptable salt of bupivacaine in the microspheres is about 40% to about 50% w/w. In some aspects, the active drug load of bupivacaine free base or a pharmaceutically acceptable salt of bupivacaine in the microspheres is about 30% to about 40% w/w. In some aspects, the active drug load of bupivacaine free base or a pharmaceutically acceptable salt of bupivacaine in the microspheres is about 30%, about 35%, about 40%, about 45%, about 50%, about 55%, about 60%, about 65%, or about 70% w/w. In some aspects, the active drug load of bupivacaine free base or a pharmaceutically acceptable salt of bupivacaine in the microspheres is about 10%, about 15%, about 20%, about 25%, about 75%, or about 80% w/w.

In some aspects, the active drug load of bupivacaine free base or a pharmaceutically acceptable salt of bupivacaine in the microspheres is about 80% w/w or less. In some aspects, the active drug load of bupivacaine free base or a pharmaceutically acceptable salt of bupivacaine in the microspheres is about 70% w/w or less. In some aspects, the active drug load of bupivacaine free base or a pharmaceutically acceptable salt of bupivacaine in the microspheres is about 60% w/w or less. In some aspects, the active drug load of bupivacaine free base or a pharmaceutically acceptable salt of bupivacaine in the microspheres is about 50% w/w or less. In some aspects, the active drug load of bupivacaine free base or a pharmaceutically acceptable salt of bupivacaine in the microspheres is about 40% w/w or less. In some aspects, the active drug load of bupivacaine free base or a pharmaceutically acceptable salt of bupivacaine in the microspheres is about 30% w/w or less. In some aspects, the active drug load of bupivacaine free base or a pharmaceutically acceptable salt of bupivacaine in the microspheres is about 20% w/w or less. **In** some aspects, the active drug load of bupivacaine free base or a pharmaceutically acceptable salt of bupivacaine in the microspheres is about 10% w/w or less. **In** some aspects, the active drug load of bupivacaine free base or a pharmaceutically acceptable salt of bupivacaine in the microspheres is about 80% w/w or more. **In** some aspects, the active drug load of bupivacaine free base or a pharmaceutically acceptable salt of bupivacaine in the microspheres is about 70% w/w or more. **In** some aspects, the active drug load of bupivacaine free base or a pharmaceutically acceptable salt of bupivacaine in the microspheres is about 60% w/w or more. **In** some aspects, the active drug load of bupivacaine free base or a pharmaceutically acceptable salt of bupivacaine in the microspheres is about 50% w/w or more. **In** some aspects, the active drug load of bupivacaine free base or a pharmaceutically acceptable salt of bupivacaine in the microspheres is about 40% w/w or more. **In** some aspects, the active drug load of bupivacaine free base or a pharmaceutically acceptable salt of bupivacaine in the microspheres is about 30% w/w or more. **In** some aspects, the active drug load of bupivacaine free base or a pharmaceutically acceptable salt of bupivacaine in the microspheres is about 20% w/w or more. **In** some aspects, the active drug load of bupivacaine free base or a pharmaceutically acceptable salt of bupivacaine in the microspheres is about 10% w/w or more. **In** some aspects, the active drug load of bupivacaine free base or a pharmaceutically acceptable salt of bupivacaine in the microspheres is about 1% w/w, about 2% w/w, about 3% w/w, about 4% w/w, about 5% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, or about 30% w/w, about 35% w/w, about 40% w/w, about 45% w/w, about 50% w/w, or about 55% w/w. **In** some aspects, the active drug load of bupivacaine free base or a pharmaceutically acceptable salt of bupivacaine in the microspheres is about 60% w/w, about 65% w/w, about 70% w/w, about 75% w/w, or about 80% w/w.

In some aspects, the stable pharmaceutical formulation is substantially sterile. In some aspects, the stable pharmaceutical formulation is sterile.

In some aspects, the stable pharmaceutical formulation has a shelf life of about 14 days at 25 °C following refrigeration. In some aspects, the stable pharmaceutical formulation has a shelf life of about 7-21 days at 25 °C following refrigeration. In some aspects, the stable pharmaceutical formulation has a shelf life of about 7 days, about 8 days, about 9 days, about 10 days, about 11 days, about 12 days, about 13 days, about 14 days about 15 days, about 16 days, about 17 days, about 18 days about 19 days, about 20 days, or about 21 days at 25 °C following refrigeration.

In some aspects, the stable pharmaceutical formulation has a shelf life of about 24 months at 25 °C. In some aspects, the stable pharmaceutical formulation has a shelf life of about 12-36 months at 25 °C. In some aspects, the stable pharmaceutical formulation has a shelf life of about 12 months, about 13 months, about 14 months, about 15 months, about 16 months, about 17 months, about 18 months, about 19 months, about 20 months, about 21 months, about 22 months, about 23 months, or about 24 months at 25 °C.

In some aspects, the pre-filled injector is a pen injector or an autoinjector.

In some aspects, the pre-filled injector is disposable.

In some aspects, the pre-filled injector is a disposable pen injector.

In some aspects, the pre-filled injector comprises a cartridge comprising the stable pharmaceutically acceptable formulation.

In some aspects, the cartridge is a dual chamber cartridge.

In some aspects, the dual chamber cartridge comprises, in a first chamber, a first volume comprising a microsphere, the microsphere comprising an active drug load of bupivacaine free base or a pharmaceutically acceptable salt of bupivacaine. In some aspects, the dual chamber cartridge comprises, in a second chamber, a second volume comprising a dilution medium. In some aspects, the first volume further comprises an amount of bupivacaine free base or a pharmaceutically acceptable salt of bupivacaine which is not contained within a microsphere. In some aspects, the second volume further comprises an amount of bupivacaine free base or a pharmaceutically acceptable salt of bupivacaine.

In some aspects, the first volume and the second volume can be combined to produce a pharmaceutically acceptable formulation disclosed herein.

In some aspects, the pre-filled injector is configurable to combine the first volume and the second volume prior to injection. In some aspects, the pre-filled injector is configurable to combine the first volume and the second volume during injection.

In some aspects, the pre-filled injector is configurable to dispense the first volume and the second volume in about equal amounts by volume. In some aspects, the pre-filled injector is configurable to dispense the first volume and the second volume in amounts that are not equal by volume.

In some aspects, the pre-filled injector comprises a 18G to 30G needle. In some aspects, the pre-filled injector comprises a 21G needle. In some aspects, the pre-filled injector comprises a 23G needle. In some aspects, the pre-filled injector comprises a 25G needle.

In some aspects, the pre-filled injector does not comprise a needle.

In some aspects, the amount of the stable pharmaceutically acceptable formulation dispensed in a single injection or more than one injection contains about 200 mg to about 8000 mg of bupivacaine free base or a pharmaceutically acceptable salt of bupivacaine.

In some aspects, the amount of the stable pharmaceutically acceptable formulation dispensed in a single injection or more than one injection contains about 500 mg to about 8000 mg of bupivacaine free base or a pharmaceutically acceptable salt of bupivacaine.

In some aspects, the amount of the stable pharmaceutically acceptable formulation dispensed in a single injection or more than one injection contains about 200 mg to about 4000 mg of bupivacaine free base or a pharmaceutically acceptable salt of bupivacaine.

In some aspects, the amount of the stable pharmaceutically acceptable formulation dispensed in a single injection or more than one injection contains about 500 mg to about 1000 mg of bupivacaine free base or a pharmaceutically acceptable salt of bupivacaine. In some aspects, the amount of the stable pharmaceutically acceptable formulation dispensed in a single injection or more than one injection contains about 250 mg to about 1200 mg of bupivacaine free base or a pharmaceutically acceptable salt of bupivacaine. In some aspects, the amount of the stable pharmaceutically acceptable formulation dispensed in a single injection or more than one injection contains about 600 mg to about 800 mg of bupivacaine free base or a pharmaceutically acceptable salt of bupivacaine. In some aspects, the amount of the stable pharmaceutically acceptable formulation dispensed in a single injection or more than one injection contains about 200 mg to about 8000 mg of bupivacaine free base or a pharmaceutically acceptable salt of bupivacaine. In some aspects, the amount of the stable pharmaceutically acceptable formulation dispensed in a single injection or more than one injection contains about 500 mg to about 4000 mg of bupivacaine free base or a pharmaceutically acceptable salt of bupivacaine. In some aspects, the amount of the stable pharmaceutically acceptable formulation dispensed in a single injection or more than one injection contains about 1000 mg to about 2000 mg of bupivacaine free base or a pharmaceutically acceptable salt of bupivacaine. In some aspects, the amount of the stable pharmaceutically acceptable formulation dispensed in a single injection or more than one injection contains about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 300 mg, about 400 mg, about 500 mg, about 600 mg, about 700 mg, about 800 mg, about 900 mg, about 1000 mg, about 1200 mg, about 1400 mg, about 1600 mg, about 1800 mg, about 2000 mg, about 2500 mg, about 3000 mg, about 3500 mg, or about 4000 mg of bupivacaine free base or a pharmaceutically acceptable salt of bupivacaine. In some aspects, the amount of the stable pharmaceutically acceptable formulation dispensed in a single injection or more than one injection contains about 4500 mg, about 5000 mg, about 5500 mg, about 6000 mg, about 6500 mg, about 7000 mg, about 7500 mg, or about 8000 mg of bupivacaine free base or a pharmaceutically acceptable salt of bupivacaine. In some aspects, the amount of the stable pharmaceutical formulation dispensed in a single injection or more than one injection contains about 4000 mg of bupivacaine free base or a pharmaceutically acceptable salt of bupivacaine.

### Method of Treatment

Any references to methods of treatment by therapy or surgery or *in vivo* diagnosis methods of this description is to be interpreted as a reference to compounds, pharmaceutical compositions and medicaments of the present invention for use in those methods. In some aspects, the present disclosure provides a method of treating pain. In a further aspect, the method comprises administering to a subject in need thereof a stable pharmaceutically acceptable formulation. In a further aspect, the stable pharmaceutically acceptable formulation comprises microspheres. In a further aspect, the microspheres comprise a first biodegradable polymer and an active drug load of bupivacaine free base or a pharmaceutically acceptable salt of bupivacaine.

In some aspects, the stable pharmaceutically acceptable formulation is administered as one or more injections. In some aspects, the stable pharmaceutically acceptable formulation is administered as a single injection. In some aspects, the stable pharmaceutically acceptable formulation is administered as more than one injection. In some aspects, the stable pharmaceutically acceptable formulation is administered as 2, 3, 4, 5, 6, 7, 8, 9, 10, 11, 12, 13, 14, or 15 injections. In some aspects, the stable pharmaceutically acceptable formulation is administered as more than 15 injections.

In some aspects, the stable pharmaceutically acceptable formulation is administered to the subject in need at a surgical site. In some aspects, the stable pharmaceutically acceptable formulation is administered to the subject in need near or around a surgical site.

In some aspects, the stable pharmaceutically acceptable formulation is administered prior to, during, and/or after a surgical procedure. In some aspects, the surgical procedure is a soft tissue surgical procedure. In some aspects, the surgical procedure is an orthopedic procedure. In some aspects, the surgical procedure is an intra-articular procedure. In some aspects, the surgical procedure is a boney procedure. In some aspects, the surgical procedure is an oophorectomy. In some aspects, the surgical procedure is a hemorrhoidectomy. In some aspects, the surgical procedure is a hernioplasty. In some aspects, the surgical procedure is a cholectomy. In some aspects, the surgical procedure is a cholecystectomy. In some aspects, the surgical procedure is an oophorectomy. In some aspects, the surgical procedure is a bunionectomy.

In some aspects, the stable pharmaceutically acceptable formulation is administered to the subacromial space of the subject.

In some aspects, the stable pharmaceutically acceptable formulation is administered for interscalene brachial plexus nerve block.

In some aspects, the stable pharmaceutically acceptable formulation is administered to an internal organ or tissue of the subject. In some aspects, the stable pharmaceutically acceptable formulation is administered to an external organ or tissue of the subject. In some aspects, the stable pharmaceutically acceptable formulation is administered to the leg of the subject. In some aspects, the stable pharmaceutically acceptable formulation is administered to the thigh of the subject. In some aspects, the stable pharmaceutically acceptable formulation is administered to the calf of the subject. In some aspects, the stable pharmaceutically acceptable formulation is administered to the buttocks of the subject. In some aspects, the stable pharmaceutically acceptable formulation is administered to the foot of the subject. In some aspects, the stable pharmaceutically acceptable formulation is administered to the knee of the subject. In some aspects, the stable pharmaceutically acceptable formulation is administered to the upper or lower back of the subject. In some aspects, the stable pharmaceutically acceptable formulation is administered to the chest of the subject. In some aspects, the stable pharmaceutically acceptable formulation is administered to the abdomen of the subject. In some aspects, the stable pharmaceutically acceptable formulation is administered to the groin of the subject. In some aspects, the stable pharmaceutically acceptable formulation is administered to the head or neck of the subject. In some aspects, the stable pharmaceutically acceptable formulation is administered to the jaw of the subject. In some aspects, the stable pharmaceutically acceptable formulation is administered to a location within the mouth of the subject. In some aspects, the stable pharmaceutically acceptable formulation is administered to the arm of the subject. In some aspects, the stable pharmaceutically acceptable formulation is administered to the shoulder of the subject. In some aspects, the stable pharmaceutically acceptable formulation is administered to the upper arm of the subject. In some aspects, the stable pharmaceutically acceptable formulation is administered to the lower arm of the subject. In some aspects, the stable pharmaceutically acceptable formulation is administered to the hand of the subject.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the active drug load of bupivacaine free base or a pharmaceutically acceptable salt of bupivacaine in the microspheres is about 10% to about 70% w/w. In some aspects, the active drug load of bupivacaine free base or a pharmaceutically acceptable salt of bupivacaine in the microspheres is about 50% to about 60% w/w. In some aspects, the active drug load of bupivacaine free base or a pharmaceutically acceptable salt of bupivacaine in the microspheres is about 40% to about 50% w/w. In some aspects, the active drug load of bupivacaine free base or a pharmaceutically acceptable salt of bupivacaine in the microspheres is about 30% to about 40% w/w. In some aspects, the active drug load of bupivacaine free base or a pharmaceutically acceptable salt of bupivacaine in the microspheres is about 30% w/w, about 31% w/w, about 32% w/w, about 33% w/w, about 34% w/w, about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 59% w/w, about 60% w/w, about 61% w/w, about 62% w/w, about 63% w/w, about 64% w/w, about 65% w/w, about 66% w/w, about 67% w/w, about 68% w/w, about 69% w/w, or about 70% w/w. In some aspects, the active drug load of bupivacaine free base or a pharmaceutically acceptable salt of bupivacaine in the microspheres is about 40% w/w. In some aspects, the active drug load of bupivacaine free base or a pharmaceutically acceptable salt of bupivacaine in the microspheres is about 50% w/w.

In some aspects, the amount of the stable pharmaceutically acceptable formulation administered to the subject contains an amount of bupivacaine free base or a pharmaceutically acceptable salt of bupivacaine of about 200 mg to about 8000 mg. In some aspects, the amount of the stable pharmaceutically acceptable formulation administered to the subject contains an amount of bupivacaine free base or a pharmaceutically acceptable salt of bupivacaine of about 500 mg to about 4000 mg. In some aspects, the amount of the stable pharmaceutically acceptable formulation administered to the subject contains an amount of bupivacaine free base or a pharmaceutically acceptable salt of bupivacaine of about 1000 mg to about 2000 mg. In some aspects, the amount of the stable pharmaceutically acceptable formulation administered to the subject contains an amount of bupivacaine free base or a pharmaceutically acceptable salt of bupivacaine of about 600 mg to about 800 mg. In some aspects, the amount of the stable pharmaceutically acceptable formulation administered to the subject contains an amount of bupivacaine free base or a pharmaceutically acceptable salt of bupivacaine of about 1000 mg to about 3000 mg. In some aspects, the amount of the stable pharmaceutically acceptable formulation administered to the subject contains an amount of bupivacaine free base or a pharmaceutically acceptable salt of bupivacaine of about 3000 mg to about 6000 mg. In some aspects, the amount of the stable pharmaceutically acceptable formulation administered to the subject contains an amount of bupivacaine free base or a pharmaceutically acceptable salt of bupivacaine of about 5000 mg to about 8000 mg. In some aspects, the amount of the stable pharmaceutically acceptable formulation administered to the subject contains an amount of bupivacaine free base or a pharmaceutically acceptable salt of bupivacaine of about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. In some aspects, the amount of the stable pharmaceutically acceptable formulation administered to the subject contains an amount of bupivacaine free base or a pharmaceutically acceptable salt of bupivacaine of about 2000 mg, about 3000 mg, about 4000 mg, about 5000 mg, about 6000 mg, about 7000 mg, or about 8000 mg. In some aspects, the amount of the stable pharmaceutically acceptable formulation administered to the subject contains an amount of bupivacaine free base or a pharmaceutically acceptable salt of bupivacaine of about 800 mg. In some aspects, the amount of the stable pharmaceutically acceptable formulation administered to the subject contains an amount of bupivacaine free base or a pharmaceutically acceptable salt of bupivacaine of about 1000 mg.

In some aspects, the stable pharmaceutically acceptable formulation is administered with an injector.

In some aspects, the stable pharmaceutically acceptable formulation is administered parenterally.

In some aspects, the stable pharmaceutically acceptable formulation is administered intramuscularly.

In some aspects, the stable pharmaceutically acceptable formulation is administered subcutaneously.

In some aspects, administration of the stable pharmaceutically acceptable formulation effects a therapeutically effective concentration of bupivacaine for about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days following an initial burst of bupivacaine in the plasma. In some aspects, administration of the stable pharmaceutically acceptable formulation effects a therapeutically effective concentration of bupivacaine for about 7 days following an initial burst of bupivacaine in the plasma.

In some aspects, the therapeutically effective concentration of bupivacaine is about 10 µg/L, about 20 µg/L, about 30 µg/L, about 40 µg/L, about 50 µg/L, about 60 µg/L, about 70 µg/L, about 80 µg/L, about 90 µg/L, about 100 µg/L, about 110 µg/L, about 120 µg/L, about 130 µg/L, about 140 µg/L, about 150 µg/L, about 160 µg/L, about 170 µg/L, about 180 µg/L, about 190 µg/L, or about 200 µg/L. In some aspects, the therapeutically effective concentration of bupivacaine is about 200 µg/L, about 210 µg/L, about 220 µg/L, about 230 µg/L, about 240 µg/L, about 250 µg/L, about 260 µg/L, about 270 µg/L, about 280 µg/L, about 290 µg/L, about 300 µg/L, about 310 µg/L, about 320 µg/L, about 330 µg/L, about 340 µg/L, about 350 µg/L, about 360 µg/L, about 370 µg/L, about 380 µg/L, about 390 µg/L, or about 400 µg/L.

In some aspects, the therapeutically effective concentration is maintained for about 7 days. In some aspects, the therapeutically effective concentration is maintained for about 3-10 days. In some aspects, the therapeutically effective concentration is maintained for about 14 days. In some aspects, the therapeutically effective concentration is maintained for about 21 days. In some aspects, the therapeutically effective concentration is maintained for about 28 days. In some aspects, the therapeutically effective concentration is maintained for about 3 days, about 4 days, about 5 days, about 6 days, about 7 days, about 8 days, about 9 days, or about 10 days.

In some aspects, the initial burst of bupivacaine is about 5% of the total dose of bupivacaine free base or a pharmaceutically acceptable salt of bupivacaine. In some aspects, the initial burst of bupivacaine is about 1% to about 80% of the total dose of bupivacaine free base or a pharmaceutically acceptable salt of bupivacaine. In some aspects, the burst of bupivacaine is about 20% to about 80% of the total dose of bupivacaine free base or a pharmaceutically acceptable salt of bupivacaine in 24 hours. In some aspects, the burst of bupivacaine is about 40% of the total dose of bupivacaine free base or a pharmaceutically acceptable salt of bupivacaine in 24 hours.

In some aspects, the initial burst of bupivacaine is about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 30%, about 35%, about 40%, about 45%, about 50%, about 55%, about 60%, about 65%, about 70%, or about 80% of the total dose of bupivacaine free base or a pharmaceutically acceptable salt of bupivacaine. In some aspects, the initial burst of bupivacaine is about 20-80% of the total dose of bupivacaine free base or a pharmaceutically acceptable salt of bupivacaine. In some aspects, the initial burst of bupivacaine is about 40-60% of the total dose of bupivacaine free base or a pharmaceutically acceptable salt of bupivacaine. In some aspects, the initial burst of bupivacaine is about 30-50% of the total dose of bupivacaine free base or a pharmaceutically acceptable salt of bupivacaine. In some aspects, the initial burst of bupivacaine is about 10-30% of the total dose of bupivacaine free base or a pharmaceutically acceptable salt of bupivacaine. In some aspects, the initial burst of bupivacaine free base or a pharmaceutically acceptable salt of bupivacaine is about 1% to about 15% of the total dose of bupivacaine free base or a pharmaceutically acceptable salt of bupivacaine. In some aspects, the initial burst of bupivacaine free base or a pharmaceutically acceptable salt of bupivacaine is about 1%, 2%, 3%, 4%, 5%, 6%, 7%, 8%, 9%, 10%, 11%, 12%, 13%, 14%, or about 15% of the total dose of bupivacaine free base or a pharmaceutically acceptable salt of bupivacaine. In some aspects, the initial burst of bupivacaine free base or a pharmaceutically acceptable salt of bupivacaine is about 1% to about 25% of the total dose of bupivacaine free base or a pharmaceutically acceptable salt of bupivacaine. In some aspects, the initial burst of bupivacaine free base or a pharmaceutically acceptable salt of bupivacaine is about 10% the total dose of bupivacaine free base or a pharmaceutically acceptable salt of bupivacaine.

In some aspects, the stable pharmaceutically acceptable formulation is administered twice per week. In some aspects, the stable pharmaceutically acceptable formulation is administered once per week. In some aspects, the stable pharmaceutically acceptable formulation is administered three, four, five, six, or seven times per week. In some aspects, the stable pharmaceutically acceptable formulation is administered once every two weeks. In some aspects, the stable pharmaceutically acceptable formulation is administered once per month.

In some aspects, the subject in need suffers from pain.

**In** some aspects, administration of the stable pharmaceutically acceptable formulation is effective in reducing the occurrence, duration, or severity of pain.

**In** some aspects, a patient suitable for the method of treatment is a nonhuman animal. In some aspects, a patient suitable for the method of treatment is a mammal. In some aspects, a patient suitable for the method of treatment is a non-primate, e.g., rabbit, cow, pig, horse, cat, dog, rat, or a primate, such as a Cynomolgous monkey. In some aspects, a patient suitable for the method of treatment is a human. In some aspects, a patient suitable for the method of treatment is a human male. In some aspects, a patient suitable for the method of treatment is a human male of age 50 or older. In some aspects, a patient suitable for the method of treatment is a human female. In some aspects, a patient suitable for the method of treatment is a human female of age 50 or older. In some aspects, a patient suitable for the method of treatment is a pre-menopause human female. In some aspects, a patient suitable for the method of treatment is a perimenopause human female. In some aspects, a patient suitable for the method of treatment is a menopausal human female. In some aspects, a patient suitable for the method of treatment is a post-menopause human female. In some aspects, a patient suitable for the method of treatment is a pregnant human female.

**In** some aspects, a patient suitable for the method of treatment is a child of about age 5 or younger.

**In** some aspects, a patient suitable for the method of treatment is a child of about age 6 to about age 12.

**In** some aspects, a patient suitable for the method of treatment is an adolescent of about age 13 to about age 17.

In some aspects, a patient suitable for the method of treatment is an adult of about age 18 or older.

### Long-Acting Dosage Form

**In** some aspects, the present disclosure provides a long-acting dosage form comprising microspheres. In a further aspect, the microspheres comprise a first biodegradable polymer and about 500 mg to about 1000 mg of an active drug load of bupivacaine free base or a pharmaceutically acceptable salt of bupivacaine. In some aspects, the microspheres comprise a first biodegradable polymer and about 200 mg to about 8000 mg of an active drug load of bupivacaine free base or a pharmaceutically acceptable salt of bupivacaine. In some aspects, the microspheres comprise a first biodegradable polymer and about 250 mg to about 1200 mg of an active drug load of bupivacaine free base or a pharmaceutically acceptable salt of bupivacaine. In some aspects, the microspheres comprise a first biodegradable polymer and about 600 mg to about 800 mg of an active drug load of bupivacaine free base or a pharmaceutically acceptable salt of bupivacaine. In some aspects, the microspheres comprise a first biodegradable polymer and about 1000 mg to about 3000 mg of an active drug load of bupivacaine free base or a pharmaceutically acceptable salt of bupivacaine. In some aspects, the microspheres comprise a first biodegradable polymer and about 3000 mg to about 6000 mg of an active drug load of bupivacaine free base or a pharmaceutically acceptable salt of bupivacaine. In some aspects, the microspheres comprise a first biodegradable polymer and about 5000 mg to about 8000 mg of an active drug load of bupivacaine free base or a pharmaceutically acceptable salt of bupivacaine. In some aspects, the microspheres comprise a first biodegradable polymer and about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 2000 mg, about 3000 mg, about 4000 mg, about 5000 mg, about 6000 mg, about 7000 mg, or about 8000 mg of an active drug load of bupivacaine free base or a pharmaceutically acceptable salt of bupivacaine. In some aspects, the microspheres comprise a first biodegradable polymer and about 800 mg of an active drug load of bupivacaine free base or a pharmaceutically acceptable salt of bupivacaine. In some aspects, the microspheres comprise a first biodegradable polymer and about 1000 mg of an active drug load of bupivacaine free base or a pharmaceutically acceptable salt of bupivacaine. In a further aspect, administration of a single dose of the long-acting dosage form to a subject results in at least one of the pharmacokinetic parameters selected from the group consisting of: (a) a steady state plasma profile of bupivacaine from day 1 to day 7 following administration exhibiting a mean concentration value no greater than the mean maximum plasma Cₘₐₓ level of bupivacaine provided by 100 mg of immediate release injection of bupivacaine hydrochloride; (b) an bupivacaine elimination half-life of about 2 hours to about 4 hours; and (c) a zero-order or a first-order release profile of bupivacaine free base or a pharmaceutically acceptable salt of bupivacaine.

In some aspects, administration of a single dose of the long-acting dosage form to a subject results in a zero-order release profile. In some aspects, administration of a single dose of the long-acting dosage form to a subject results in a first-order release profile.

In some aspects, the release profile corresponds to about 1% to about 50% release of the total administered dose of bupivacaine free base or a pharmaceutically acceptable salt of bupivacaine per day. In some aspects, the release profile corresponds to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, or about 50% release of the total administered dose of bupivacaine free base or a pharmaceutically acceptable salt of bupivacaine per day. In some aspects, the release profile corresponds to about 3% to about 15% release of the total administered dose of bupivacaine free base or a pharmaceutically acceptable salt of bupivacaine per day. In some aspects, the release profile corresponds to about 12% to about 14% release of the total administered dose of bupivacaine free base or a pharmaceutically acceptable salt of bupivacaine per day.

In some aspects, the pharmaceutically acceptable salt of bupivacaine is an acid addition salt of bupivacaine.

In some aspects, the pharmaceutically acceptable salt of bupivacaine is bupivacaine hydrochloride.

In some aspects, the long-acting dosage form comprises a plurality of microspheres comprising bupivacaine free base or a pharmaceutically acceptable salt of bupivacaine, wherein the plurality of microspheres is characterized by a median particle size ("Dv50") of about 20 µm to about 80 µm. In some aspects, the plurality of microspheres is characterized by a Dv50 of about 20 µm, about 25 µm, 30 µm, about 35 µm, about 40 µm, about 45 µm, about 50 µm, about 55 µm, about 60 µm, about 65 µm, about 70 µm, about 75 µm, or about 80 µm. In some aspects, the plurality of microspheres is characterized by a Dv50 of about 50 µm. In some aspects, the plurality of microspheres is characterized by a Dv50 of about 70 µm.

In some aspects, the plurality of microspheres is characterized by a particle size having a CV less than 70%. In some aspects, the plurality of microspheres is characterized by a particle size having a CV less than 60%. In some aspects, the plurality of microspheres is characterized by a particle size having a CV less than 50%. In some aspects, the plurality of microspheres is characterized by a particle size having a CV less than 40%. In some aspects, the plurality of microspheres is characterized by a particle size having a CV less than 30%. In some aspects, the plurality of microspheres is characterized by a particle size having a CV less than 20%. In some aspects, the plurality of microspheres is characterized by a particle size having a CV less than 10%.

In some aspects, the plurality of microspheres is characterized by a particle size having a span value of from about 0.1 to about 1.5. In some aspects, the plurality of microspheres is characterized by a particle size having a span value of from about 0.1 to about 2.5, from about 0.1 to about 3, from about 0.1 to about 3, from about 0.1 to about 4, from about 0.1 to about 4.5, from about 0.1 to about 5, from about 1.5 to about 2, from about 1.5 to about 2.5, from about 1.5 to about 3, from about 1.5 to about 3.5, from about 1.5 to about 4, from about 1.5 to about 4.5, from about 1.5 to about 5, from about 2 to about 2.5, from about 2 to about 3, from about 2 to about 3.5, from about 2 to about 4, from about 2 to about 4.5, from about 2 to about 5, from about 2.5 to about 3, from about 2.5 to about 3.5, from about 2.5 to about 4, from about 2.5 to about 4.5, from about 2.5 to about 5, from about 3 to about 3.5, from about 3 to about 4, from about 3 to about 4.5, from about 3 to about 5, from about 3.5 to about 4, from about 3.5 to about 4.5, from about 3.5 to about 5, from about 4 to about 4.5, from about 4 to about 5, or from about 4.5 to about 5.

In some aspects, the plurality of microspheres is characterized by a particle size having a span value of about 1. In some aspects, the plurality of microspheres is characterized by a particle size having a span value of about 0.1, about 0.2, about 0.3, about 0.4, about 0.5, about 0.6, about 0.7, about 0.8, about 0.9, about 1, about 1.1, about 1.2, about 1.3, about 1.4, about 1.5, about 2, about 3.5, about 4, about 4.5, or about 5.

In some aspects, the active drug load of bupivacaine free base or a pharmaceutically acceptable salt of bupivacaine in the microspheres is about 10% to about 80% w/w. In some aspects, the active drug load of bupivacaine free base or a pharmaceutically acceptable salt of bupivacaine in the microspheres is about 30% to about 70% w/w. In some aspects, the active drug load of bupivacaine free base or a pharmaceutically acceptable salt of bupivacaine in the microspheres is about 50% to about 60% w/w. In some aspects, the active drug load of bupivacaine free base or a pharmaceutically acceptable salt of bupivacaine in the microspheres is about 40% to about 50% w/w. In some aspects, the active drug load of bupivacaine free base or a pharmaceutically acceptable salt of bupivacaine in the microspheres is about 30% to about 40% w/w. In some aspects, the active drug load of bupivacaine free base or a pharmaceutically acceptable salt of bupivacaine in the microspheres is about 30% w/w, about 31% w/w, about 32% w/w, about 33% w/w, about 34% w/w, about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 59% w/w, about 60% w/w, about 61% w/w, about 62% w/w, about 63% w/w, about 64% w/w, about 65% w/w, about 66% w/w, about 67% w/w, about 68% w/w, about 69% w/w, or about 70% w/w. In some aspects, the active drug load of bupivacaine free base or a pharmaceutically acceptable salt of bupivacaine in the microspheres is about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, about 71% w/w, about 72% w/w, about 73% w/w, about 74% w/w, about 75% w/w, about 76% w/w, about 77% w/w, about 78% w/w, about 79% w/w, or about 80% w/w. In some aspects, the active drug load of bupivacaine free base or a pharmaceutically acceptable salt of bupivacaine in the microspheres is about 40% w/w. In some aspects, the active drug load of bupivacaine free base or a pharmaceutically acceptable salt of bupivacaine in the microspheres is about 50% w/w.

In some aspects, the amount of the stable pharmaceutically acceptable formulation administered to the subject contains an amount of bupivacaine free base or a pharmaceutically acceptable salt of bupivacaine of about 200 mg to about 8000 mg. In some aspects, the amount of the stable pharmaceutically acceptable formulation administered to the subject contains an amount of bupivacaine free base or a pharmaceutically acceptable salt of bupivacaine of about 500 mg to about 1000 mg. **In** some aspects, the amount of the stable pharmaceutically acceptable formulation administered to the subject contains an amount of bupivacaine free base or a pharmaceutically acceptable salt of bupivacaine of about 250 mg to about 1200 mg. **In** some aspects, the amount of the stable pharmaceutically acceptable formulation administered to the subject contains an amount of bupivacaine free base or a pharmaceutically acceptable salt of bupivacaine of about 600 mg to about 800 mg. **In** some aspects, the amount of the stable pharmaceutically acceptable formulation administered to the subject contains an amount of bupivacaine free base or a pharmaceutically acceptable salt of bupivacaine of about 1000 mg to about 3000 mg. **In** some aspects, the amount of the stable pharmaceutically acceptable formulation administered to the subject contains an amount of bupivacaine free base or a pharmaceutically acceptable salt of bupivacaine of about 3000 mg to about 6000 mg. **In** some aspects, the amount of the stable pharmaceutically acceptable formulation administered to the subject contains an amount of bupivacaine free base or a pharmaceutically acceptable salt of bupivacaine of about 5000 mg to about 8000 mg. **In** some aspects, the amount of the stable pharmaceutically acceptable formulation administered to the subject contains an amount of bupivacaine free base or a pharmaceutically acceptable salt of bupivacaine of about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. **In** some aspects, the amount of the stable pharmaceutically acceptable formulation administered to the subject contains an amount of bupivacaine free base or a pharmaceutically acceptable salt of bupivacaine of about 200 mg, about 2000 mg, about 3000 mg, about 4000 mg, about 5000 mg, about 6000 mg, about 7000 mg, or about 8000 mg. **In** some aspects, the amount of the stable pharmaceutically acceptable formulation administered to the subject contains an amount of bupivacaine free base or a pharmaceutically acceptable salt of bupivacaine of about 800 mg. **In** some aspects, the amount of the stable pharmaceutically acceptable formulation administered to the subject contains an amount of bupivacaine free base or a pharmaceutically acceptable salt of bupivacaine of about 1000 mg.

In some aspects, the first biodegradable polymer is selected from the group consisting of: a polylactide, a polyglycolide, and a poly(lactide-co-glycolide) copolymer.

In some aspects, the first biodegradable polymer is a poly(lactide-co-glycolide) copolymer, where the content of polymerized lactic acid units is at least 50 mol% based on the total amount of polymerized units or blends of poly(lactide-co-glycolide) copolymers with different relative amounts of glycolic acid and lactic acid, where the content of polymerized lactic acid units is at least 50 mol% based on the total amount of polymerized units.

In some aspects, the first biodegradable polymer is substantially enclosed by a second biodegradable polymer.

In some aspects, the first biodegradable polymer is not identical in composition to the second biodegradable polymer.

In some aspects, the stable pharmaceutically acceptable formulation further comprises a diluent.

In some aspects, the active drug load of bupivacaine free base or a pharmaceutically acceptable salt of bupivacaine in the microspheres is about 50% w/w or less. In some aspects, the active drug load of bupivacaine free base or a pharmaceutically acceptable salt of bupivacaine in the microspheres is about 40% w/w or less. In some aspects, the active drug load of bupivacaine free base or a pharmaceutically acceptable salt of bupivacaine in the microspheres is about 30% w/w or less. In some aspects, the active drug load of bupivacaine free base or a pharmaceutically acceptable salt of bupivacaine in the microspheres is about 20% w/w or less. In some aspects, the active drug load of bupivacaine free base or a pharmaceutically acceptable salt of bupivacaine in the microspheres is about 10% w/w or less. In some aspects, the active drug load of bupivacaine free base or a pharmaceutically acceptable salt of bupivacaine in the microspheres is about 50% w/w or more. In some aspects, the active drug load of bupivacaine free base or a pharmaceutically acceptable salt of bupivacaine in the microspheres is about 40% w/w or more. In some aspects, the active drug load of bupivacaine free base or a pharmaceutically acceptable salt of bupivacaine in the microspheres is about 30% w/w or more. In some aspects, the active drug load of bupivacaine free base or a pharmaceutically acceptable salt of bupivacaine in the microspheres is about 20% w/w or more. In some aspects, the active drug load of bupivacaine free base or a pharmaceutically acceptable salt of bupivacaine in the microspheres is about 10% w/w or more. In some aspects, the active drug load of bupivacaine free base or a pharmaceutically acceptable salt of bupivacaine in the microspheres is about 1% w/w, about 2% w/w, about 3% w/w, about 4% w/w, about 5% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, about 30% w/w, about 35% w/w, about 40% w/w, about 45% w/w, about 50% w/w, or about 55% w/w.

In some aspects, the active drug load of bupivacaine free base or a pharmaceutically acceptable salt of bupivacaine in the microspheres is about 10% w/w to about 80%.

In some aspects, the active drug load of bupivacaine free base or a pharmaceutically acceptable salt of bupivacaine in the microspheres is about 200 mg to about 8000 mg. In some aspects, the active drug load of bupivacaine free base or a pharmaceutically acceptable salt of bupivacaine in the microspheres is about 500 mg to about 1000 mg. In some aspects, the active drug load of bupivacaine free base or a pharmaceutically acceptable salt of bupivacaine in the microspheres is about 250 mg to about 1200 mg. In some aspects, the active drug load of bupivacaine free base or a pharmaceutically acceptable salt of bupivacaine in the microspheres is about 600 mg to about 800 mg. In some aspects, the active drug load of bupivacaine free base or a pharmaceutically acceptable salt of bupivacaine in the microspheres is about 1000 mg to about 3000 mg. In some aspects, the active drug load of bupivacaine free base or a pharmaceutically acceptable salt of bupivacaine in the microspheres is about 3000 mg to about 6000 mg. In some aspects, the active drug load of bupivacaine free base or a pharmaceutically acceptable salt of bupivacaine in the microspheres is about 5000 mg to about 8000 mg. In some aspects, the active drug load of bupivacaine free base or a pharmaceutically acceptable salt of bupivacaine in the microspheres is about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. In some aspects, the active drug load of bupivacaine free base or a pharmaceutically acceptable salt of bupivacaine in the microspheres is about 200 mg, about 2000 mg, about 3000 mg, about 4000 mg, about 5000 mg, about 6000 mg, about 7000 mg, or about 8000 mg. In some aspects, the active drug load of bupivacaine free base or a pharmaceutically acceptable salt of bupivacaine in the microspheres is about 800 mg. In some aspects, the active drug load of bupivacaine free base or a pharmaceutically acceptable salt of bupivacaine in the microspheres is about 1000 mg.

In some aspects, the stable pharmaceutical formulation is substantially sterile.

In some aspects, the stable pharmaceutical formulation has a shelf life of about 14 days at 25°C following refrigeration.

In some aspects, the stable pharmaceutical formulation has a shelf life of about 24 months at 25°C.

In some aspects, the stable pharmaceutically acceptable formulation is administered from an injector.

In some aspects, the injector is a pen injector or an autoinjector.

In some aspects, the injector is disposable.

In some aspects, the injector is a disposable pen injector.

In some aspects, the injector is a pre-filled injector.

In some aspects, the injector comprises a cartridge comprising the stable pharmaceutically acceptable formulation.

In some aspects, the cartridge is a dual chamber cartridge.

In some aspects, the dual chamber cartridge comprises, in a first chamber, a first volume comprising a microsphere, the microsphere comprising an active drug load of bupivacaine free base or a pharmaceutically acceptable salt of bupivacaine. In some aspects, the dual chamber cartridge comprises, in a second chamber, a second volume comprising a dilution medium. In some aspects, the first volume further comprises an amount of bupivacaine free base or a pharmaceutically acceptable salt of bupivacaine which is not contained within a microsphere. In some aspects, the second volume further comprises an amount of bupivacaine free base or a pharmaceutically acceptable salt of bupivacaine.

In some aspects, the first volume and the second volume can be combined to produce a pharmaceutically acceptable formulation disclosed herein.

In some aspects, the pre-filled injector is configurable to combine the first volume and the second volume prior to injection. In some aspects, the pre-filled injector is configurable to combine the first volume and the second volume during injection.

In some aspects, the pre-filled injector is configurable to dispense the first volume and the second volume in about equal amounts by volume. In some aspects, the pre-filled injector is configurable to dispense the first volume and the second volume in amounts that are not equal by volume.

In some aspects, the injector comprises a 18G to 30G needle. In some aspects, the injector comprises a 21G needle. In some aspects, the pre-filled injector comprises a 23G needle. In some aspects, the pre-filled injector comprises a 25G needle.

In some aspects, the stable pharmaceutically acceptable formulation is administered parenterally.

In some aspects, the stable pharmaceutically acceptable formulation is administered intramuscularly.

In some aspects, the stable pharmaceutically acceptable formulation is administered subcutaneously.

In some aspects, administration of the stable pharmaceutically acceptable formulation effects a therapeutically effective concentration of bupivacaine for about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days following an initial burst of bupivacaine in the plasma. In some aspects, administration of the stable pharmaceutically acceptable formulation effects a therapeutically effective concentration of bupivacaine for about 7 days following an initial burst of bupivacaine in the plasma.

In some aspects, the therapeutically effective concentration of bupivacaine is about 10 µg/L, about 20 µg/L, about 30 µg/L, about 40 µg/L, about 50 µg/L, about 60 µg/L, about 70 µg/L, about 80 µg/L, about 90 µg/L, about 100 µg/L, about 110 µg/L, about 120 µg/L, about 130 µg/L, about 140 µg/L, about 150 µg/L, about 160 µg/L, about 170 µg/L, about 180 µg/L, about 190 µg/L, or about 200 µg/L.

In some aspects, administration of the stable pharmaceutically acceptable formulation effects a steady-state plasma concentration of bupivacaine following an initial burst of bupivacaine in the plasma.

In some aspects, the steady-state plasma concentration is about 35 µg/L. In some aspects, the steady-state plasma concentration is about 20-50 µg/L. In some aspects, the steady-state plasma concentration is about 30-40 µg/L. In some aspects, the steady-state plasma concentration is about 20 µg/L, about 21 µg/L, about 22 µg/L, about 23 µg/L, about 24 µg/L, about 25 µg/L, about 26 µg/L, about 27 µg/L, about 28 µg/L, about 29 µg/L, about 30 µg/L, about 31 µg/L, about 32 µg/L, about 33 µg/L, about 34 µg/L, about 35 µg/L, about 36 µg/L, about 37 µg/L, about 38 µg/L, about 39 µg/L, about 40 µg/L, about 41 µg/L, about 42 µg/L, about 43 µg/L, about 44 µg/L, about 45 µg/L, about 46 µg/L, about 47 µg/L, about 48 µg/L, about 49 µg/L, or about 50 µg/L.

In some aspects, the steady state plasma concentration is from about 10 µg/L to about 500 µg/L. In some aspects, the steady state plasma concentration is from about 10 µg/L to about 500 µg/L, from about 50 µg/L to about 500 µg/L, from about 100 µg/L to about 500 µg/L, from about 150 µg/L to about 500 µg/L, from about 200 µg/L to about 500 µg/L, from about 250 µg/L to about 500 µg/L, from about 300 µg/L to about 500 µg/L, from about 350 µg/L to about 500 µg/L, from about 400 µg/L to about 500 µg/L, from about 450 µg/L to about 500 µg/L, from about 10 µg/L to about 450 µg/L, from about 50 µg/L to about 450 µg/L, from about 100 µg/L to about 450 µg/L, from about 150 µg/L to about 450 µg/L, from about 200 µg/L to about 450 µg/L, from about 250 µg/L to about 450 µg/L, from about 300 µg/L to about 450 µg/L, from about 350 µg/L to about 450 µg/L, from about 400 µg/L to about 450 µg/L, from about 10 µg/L to about 400 µg/L, from about 50 µg/L to about 400 µg/L, from about 100 µg/L to about 400 µg/L, from about 150 µg/L to about 400 µg/L, from about 200 µg/L to about 400 µg/L, from about 250 µg/L to about 400 µg/L, from about 300 µg/L to about 400 µg/L, from about 350 µg/L to about 400 µg/L, from about 10 µg/L to about 350 µg/L, from about 50 µg/L to about 350 µg/L, from about 100 µg/L to about 350 µg/L, from about 150 µg/L to about 350 µg/L, from about 200 µg/L to about 350 µg/L, from about 250 µg/L to about 350 µg/L, from about 300 µg/L to about 350 µg/L, from about 10 µg/L to about 300 µg/L, from about 50 µg/L to about 300 µg/L, from about 100 µg/L to about 300 µg/L, from about 150 µg/L to about 300 µg/L, from about 200 µg/L to about 300 µg/L, from about 250 µg/L to about 300 µg/L, from about 10 µg/L to about 250 µg/L, from about 50 µg/L to about 250 µg/L, from about 100 µg/L to about 250 µg/L, from about 150 µg/L to about 250 µg/L, from about 200 µg/L to about 250 µg/L, from about 10 µg/L to about 200 µg/L, from about 50 µg/L to about 200 µg/L, from about 100 µg/L to about 200 µg/L, from about 150 µg/L to about 200 µg/L, from about 10 µg/L to about 150 µg/L, from about 50 µg/L to about 150 µg/L, from about 100 µg/L to about 150 µg/L, from about 10 µg/L to about 100 µg/L, from about 50 µg/L to about 100 µg/L, or from about 10 µg/L to about 50 µg/L. In some aspects, the steady state plasma concentration is about 100 µg/L. In some aspects, the steady state plasma concentration is about 110 µg/L, about 120 µg/L, about 130 µg/L, about 140 µg/L, about 150 µg/L, about 160 µg/L, about 170 µg/L, about 180 µg/L, about 190 µg/L, about 200 µg/L, about 210 µg/L, about 220 µg/L, about 230 µg/L, about 240 µg/L, about 250 µg/L, about 260 µg/L, about 270 µg/L, about 280 µg/L, about 290 µg/L, about 300 µg/L, about 310 µg/L, about 320 µg/L, about 330 µg/L, about 340 µg/L, about 350 µg/L, about 360 µg/L, about 370 µg/L, about 380 µg/L, about 390 µg/L, or about 400 µg/L.

In some aspects, the steady-state plasma concentration is maintained for about 7 days. In some aspects, the steady-state plasma concentration is maintained for about 3-10 days. In some aspects, the steady-state plasma concentration is maintained for about 3 days, about 4 days, about 5 days, about 6 days, about 7 days, about 8 days, about 9 days, or about 10 days.

In some aspects, the initial burst of bupivacaine is completed about 0.5 to about 24 hours after injection. In some aspects, the initial burst of bupivacaine is completed about 1 to about 12 hours after injection. In some aspects, the initial burst of bupivacaine is completed about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, or about 24 hours after injection.

In some aspects, the initial burst of bupivacaine is about 5% of the total dose of bupivacaine free base or a pharmaceutically acceptable salt of bupivacaine. In some aspects, the initial burst of bupivacaine is about 1-50% of the total dose of bupivacaine free base or a pharmaceutically acceptable salt of bupivacaine. In some aspects, the initial burst of bupivacaine is about 30-50% of the total dose of bupivacaine free base or a pharmaceutically acceptable salt of bupivacaine. In some aspects, the initial burst of bupivacaine is about 10-30% of the total dose of bupivacaine free base or a pharmaceutically acceptable salt of bupivacaine. In some aspects, the initial burst of bupivacaine is about 1-15% of the total dose of bupivacaine free base or a pharmaceutically acceptable salt of bupivacaine. In some aspects, the initial burst of bupivacaine is about 1%, about 2%, about 3%, about 4%, about 5%, about 6%, about 7%, about 8%, about 9%, about 10%, about 11%, about 12%, about 13%, about 14%, or about 15% of the total dose of bupivacaine free base or a pharmaceutically acceptable salt of bupivacaine.

In some aspects, following the initial burst of bupivacaine free base or a pharmaceutically acceptable salt of bupivacaine, there is an extended release of bupivacaine for about 7 days. In some aspects, the extended release corresponds to a release of about 5% to about 25% release of the total administered dose of bupivacaine free base or a pharmaceutically acceptable salt of bupivacaine per day. In some aspects, the extended release corresponds to a release of about 2% to about 25% release of the total administered dose of bupivacaine free base or a pharmaceutically acceptable salt of bupivacaine per day. In some aspects, the extended release corresponds to a release of about 10% to about 15% release of the total administered dose of bupivacaine free base or a pharmaceutically acceptable salt of bupivacaine per day. In some aspects, the extended release corresponds to a release of about 2% to about 5%, about 2% to about 10%, about 2% to about 15%, about 2% to about 20%, about 5% to about 10%, about 5% to about 15%, about 5% to about 20%, about 5% to about 25%, about 10% to about 20%, about 10% to about 25%, about 15% to about 20%, about 15% to about 25%, or about 20% to about 25% of the total administered dose of bupivacaine free base or a pharmaceutically acceptable salt of bupivacaine per day.

In some aspects, following the initial burst of bupivacaine free base or a pharmaceutically acceptable salt of bupivacaine, there is a zero-order release profile for the subsequent about 4 to about 10 days. In some aspects, following the initial burst of bupivacaine free base or a pharmaceutically acceptable salt of bupivacaine, there is a zero-order release profile for the subsequent about 5 to about 7 days. In some aspects, following the initial burst of bupivacaine free base or a pharmaceutically acceptable salt of bupivacaine, there is a zero-order release profile for the subsequent about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days. In some aspects, following the initial burst of bupivacaine free base or a pharmaceutically acceptable salt of bupivacaine, there is a zero-order release profile for the subsequent about 5 days. In some aspects, following the initial burst of bupivacaine free base or a pharmaceutically acceptable salt of bupivacaine, there is a zero-order release profile for the subsequent about 6 days. In some aspects, following the initial burst of bupivacaine free base or a pharmaceutically acceptable salt of bupivacaine, there is a zero-order release profile for the subsequent about 7 days. In some aspects, following the initial burst of bupivacaine free base or a pharmaceutically acceptable salt of bupivacaine, there is a zero-order release profile for the subsequent about 8 days. In some aspects, following the initial burst of bupivacaine free base or a pharmaceutically acceptable salt of bupivacaine, there is a zero-order release profile for the subsequent about 9 days. In some aspects, following the initial burst of bupivacaine free base or a pharmaceutically acceptable salt of bupivacaine, there is a zero-order release profile for the subsequent about 10 days. In some aspects, following the initial burst of bupivacaine free base or a pharmaceutically acceptable salt of bupivacaine, there is a zero-order release profile for the subsequent about 11 days. In some aspects, following the initial burst of bupivacaine free base or a pharmaceutically acceptable salt of bupivacaine, there is a zero-order release profile for the subsequent about 12 days. In some aspects, following the initial burst of bupivacaine free base or a pharmaceutically acceptable salt of bupivacaine, there is a zero-order release profile for the subsequent about 13 days. In some aspects, following the initial burst of bupivacaine free base or a pharmaceutically acceptable salt of bupivacaine, there is a zero-order release profile for the subsequent about 14 days.

In some aspects, the zero-order or first-order release profile corresponds to a release of about 12% to about 14% release of the total administered dose of bupivacaine free base or a pharmaceutically acceptable salt of bupivacaine per day.

In some aspects, the zero-order or first-order release profile corresponds to a release of about 5% to about 14% release of the total administered dose of bupivacaine free base or a pharmaceutically acceptable salt of bupivacaine per day.

In some aspects, the zero-order or first-order release profile corresponds to a release of about 1% to about 5% release of the total administered dose of bupivacaine free base or a pharmaceutically acceptable salt of bupivacaine per day.

In some aspects, the elimination half-life of bupivacaine is about 40 minutes to about 60 minutes. In some aspects, the elimination half-life of bupivacaine is about 30 minutes to about 90 minutes. In some aspects, the elimination half-life of bupivacaine is from about 1 hour to about 2 hours, from about 1 hour to about 3 hours, from about 1 hour to about 4 hours, from about 2 hours to about 3 hours, from about 2 hours to about 4 hours, or from about 3 hours to about 4 hours. In some aspects, the elimination half-life of bupivacaine is about 2.1 h. In some aspects, the elimination half-life of bupivacaine is about 2.2 h. In some aspects, the elimination half-life of bupivacaine is about 2.3 h. In some aspects, the elimination half-life of bupivacaine is about 2.4 h. In some aspects, the elimination half-life of bupivacaine is about 2.5 h. In some aspects, the elimination half-life of bupivacaine is about 2.6 h. In some aspects, the elimination half-life of bupivacaine is about 2.7 h. In some aspects, the elimination half-life of bupivacaine is about 2.8 h. In some aspects, the elimination half-life of bupivacaine is about 2.9 h. In some aspects, the elimination of half-life of bupivacaine is about 1 hour, about 2 hours, about 3 hours, or about 4 hours.

In some aspects, the steady state plasma profile of bupivacaine from day 1 to day 7 following administration exhibits a mean concentration lower than the mean maximum plasma level Cₘₐₓ of bupivacaine provided by immediate release injection of bupivacaine hydrochloride.

In some aspects, the bioavailability of bupivacaine free base or a pharmaceutically acceptable salt of bupivacaine is substantially the same under fed and fasting conditions.

### Kit

In some aspects, the present disclosure provides a kit. In a further aspect, the kit comprises a first vial comprising a concentrated form of the one of the stable pharmaceutically acceptable formulations described herein or one of the long-acting dosage forms described herein. In a further aspect, the kit comprises a first vial comprising a dry powder form of bupivacaine free base. In a further aspect, the kit comprises a second vial comprising a pharmaceutically acceptable diluent. In a further aspect, the kit comprises a first syringe suitable for withdrawing the pharmaceutically acceptable diluent from the second vial. In a further aspect, the kit comprises an adapter, which can operably attach to the first syringe and is suitable for dispensing the pharmaceutically acceptable diluent into the first vial. In a further aspect, the kit comprises a second syringe suitable for withdrawing a liquid from the second vial and for injecting the liquid into a subject. In a further aspect, the kit comprises instructions for diluting the concentrated form and for administering the stable pharmaceutically acceptable formulation or the long-acting dosage form to a patient in need thereof.

Solutions of the active compounds as free base or pharmacologically acceptable salts may be prepared in water suitably mixed with a surfactant, such as hydroxypropylcellulose. Solutions of the active compounds as free base or pharmaceutically acceptable salts may be prepared in a solution comprising an additive, such as carboxymethyl cellulose or hyaluronic acid, in water or aqueous buffer. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile dry powders for the extemporaneous preparation of sterile injectable solutions or dispersions (U.S. Pat. No. 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, polyol (i.e., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for, intramuscular, subcutaneous, and intraperitoneal administration. In this connection, sterile aqueous media that can be employed will be known to those of skill in the art in light of the present disclosure. For example, one dosage may be dissolved in 1 ml of isotonic NaCl solution and either added to 1,000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, e.g., water or a saline solution, with or without a stabilizing agent. In one aspect, a dry powder form of bupivacaine free base is combined with a liquid carrier such as, e.g., water or a saline solution, with or without a stabilizing agent.

In some aspects, the pharmaceutically acceptable formulation is administered at a quantity sufficient to eliminate or reduce pain. In some aspects, the method of treatment reduces the severity, duration, or occurrence of pain experienced by the patient.

In some aspects, the dry powder form of bupivacaine free base comprises less than 5% water, by weight. In some aspects, the dry powder form of bupivacaine free base comprises less than 4% water, by weight. In some aspects, the dry powder form of bupivacaine free base comprises less than 3% water, by weight. In some aspects, the dry powder form of bupivacaine free base comprises less than 2% water, by weight. In some aspects, the dry powder form of bupivacaine free base comprises less than 1% water, by weight. In some aspects, the dry powder form of bupivacaine free base comprises less than 0.5% water, by weight. In some aspects, the dry powder form of bupivacaine free base comprises less than 0.4% water, by weight. In some aspects, the dry powder form of bupivacaine free base comprises less than 0.3% water, by weight. In some aspects, the dry powder form of bupivacaine free base comprises less than 0.2% water, by weight. In some aspects, the dry powder form of bupivacaine free base comprises less than 0.1% water, by weight.

### Conditions to be Treated

The present disclosure relates to a method of reducing the severity of, preventing, or eliminating pain.

### Combination Therapy

In some aspects, a pharmaceutically acceptable formulation comprising bupivacaine can be administered in combination with one or more additional therapeutic agents, in a single dosage form or as separate dosage forms.

In some aspects, when administered as a separate dosage form, bupivacaine may be administered prior to, concurrently as, or following administration of one or more additional therapeutic agents. In some embodiments, when administered as a separate dosage form, one or more doses of one or more additional therapeutic agents may be administered prior to the bupivacaine.

As used herein, the administration in "combination" of bupivacaine, and one or more additional therapeutic agents refers not only to simultaneous or sequential administration of the agents, but also to the administration of the agents during a single treatment cycle, as understood by one skilled in the art.

In some aspects, a pharmaceutically acceptable formulation comprising bupivacaine can further comprise an anticoagulant, which can be heparin or a pharmaceutically acceptable salt of bupivacaine.

### Pharmacokinetics

In some aspects, bupivacaine is about 84% to about 95% bound to plasma proteins at some time following administration of a pharmaceutically acceptable formulation of bupivacaine.

In some aspects, bupivacaine is metabolized in the liver, producing pipecoloxylidide as a metabolite. In some aspects, about 5% of bupivacaine is converted to pipecoloxylidide.

In some aspects, about 5-7% of administered bupivacaine is excreted in the urine.

In some aspects, pain relief is observed within about 15 to about 60 minutes following intramuscular treatment with a pharmaceutically acceptable formulation comprising bupivacaine. In some aspects, pain relief is observed within about 5 to about 60 minutes following intramuscular treatment with a pharmaceutically acceptable formulation comprising bupivacaine.

In some aspects, the peak plasma concentration of bupivacaine is reached within about 30 to about 60 minutes following subcutaneous administration of a pharmaceutically acceptable formulation of bupivacaine. In some aspects, the peak plasma concentration of bupivacaine is reached within about 15 to about 120 minutes following subcutaneous administration of a pharmaceutically acceptable formulation of bupivacaine.

In some aspects, the method of treatment provides about 5 days of relief from pain. In some aspects, the method of treatment provides pain relief for about 2 days, about 3 days, about 4 days, about 5 days, about 6 days, about 7 days, about 8 days, about 9 days, or about 10 days.

In some aspects, the formulations described herein are substantially bioequivalent to EXPAREL^{®} or POSIMIR^{®}.

In some aspects, the mean Tₘₐₓ of the formulations described herein is about 80% to about 125% of the Tₘₐₓ of EXPAREL^{®} or POSIMIR^{®}.

In some aspects, the mean Tₘₐₓ of the formulations described herein is about 75% to about 130% of the Tₘₐₓ of EXPAREL^{®} or POSIMIR^{®}.

In some aspects, the mean Tₘₐₓ of the formulations described herein is about 70% to about 135% of the Tₘₐₓ of EXPAREL^{®} or POSIMIR^{®}.

In some aspects, the mean Tₘₐₓ of the formulations described herein is about 65% to about 140% of the Tₘₐₓ of EXPAREL^{®} or POSIMIR^{®}.

In some aspects, the mean Tₘₐₓ of the formulations described herein is about 60% to about 145% of the Tₘₐₓ of EXPAREL^{®} or POSIMIR^{®}.

In some aspects, the mean Tₘₐₓ of the formulations described herein is about 30% to about 60% of the Tₘₐₓ of EXPAREL^{®} or POSIMIR^{®}.

In some aspects, the mean Tₘₐₓ of the formulations described herein is about 20% to about 60% of the Tₘₐₓ of EXPAREL^{®} or POSIMIR^{®}.

In some aspects, the mean Tₘₐₓ of the formulations described herein is about 10% to about 50% of the Tₘₐₓ of EXPAREL^{®} or POSIMIR^{®}.

In some aspects, the mean Tₘₐₓ of the formulations described herein is about 10%, about 15%, about 20%, about 25%, about 30%, about 35%, about 40%, about 40%, about 50%, about 55%, about 60%, about 65%, about 70%, about 75%, about 80%, about 85%, about 90%, about 95%, about 100%, about 105%, about 110%, about 115%, about 120% about 125%, about 130%, about 135%, about 140%, or about 145% of the Tₘₐₓ of EXPAREL^{®} or POSIMIR^{®}.

In some aspects, the mean Cₘₐₓ of the formulations described herein is about 80% to about 125% of the Cₘₐₓ of EXPAREL^{®} or POSIMIR^{®}.

In some aspects, the mean Cₘₐₓ of the formulations described herein is about 75% to about 130% of the Cₘₐₓ of EXPAREL^{®} or POSIMIR^{®}.

In some aspects, the mean Cₘₐₓ of the formulations described herein is about 70% to about 135% of the Cₘₐₓ of EXPAREL^{®} or POSIMIR^{®}.

In some aspects, the mean Cₘₐₓ of the formulations described herein is about 65% to about 140% of the Cₘₐₓ of EXPAREL^{®} or POSIMIR^{®}.

In some aspects, the mean Cₘₐₓ of the formulations described herein is about 60% to about 145% of the Cₘₐₓ of EXPAREL^{®} or POSIMIR^{®}.

In some aspects, the mean Cₘₐₓ of the formulations described herein is about 60%, about 65%, about 70%, about 75%, about 80%, about 85%, about 90%, about 95%, about 100%, about 105%, about 110%, about 115%, about 120% about 125%, about 130%, about 135%, about 140%, or about 145% of the Cₘₐₓ of EXPAREL^{®} or POSIMIR^{®}.

In some aspects, the mean AUC_{0-∞} of the formulations described herein is about 80% to about 125% of the AUC_{0-∞} of EXPAREL^{®} or POSIMIR^{®}.

In some aspects, the mean AUC_{0-∞} of the formulations described herein is about 75% to about 130% of the AUC_{0-∞} of EXPAREL^{®} or POSIMIR^{®}.

In some aspects, the mean AUC_{0-∞} of the formulations described herein is about 70% to about 135% of the AUC_{0-∞} of EXPAREL^{®} or POSIMIR^{®}.

In some aspects, the mean AUC_{0-∞} of the formulations described herein is about 65% to about 140% of the AUC_{0-∞} of EXPAREL^{®} or POSIMIR^{®}.

In some aspects, the mean AUC_{0-∞} of the formulations described herein is about 60% to about 145% of the AUC_{0-∞} of EXPAREL^{®} or POSIMIR^{®}.

In some aspects, the mean AUC_{0-∞} of the formulations described herein is about 100% to about 165% of the AUC_{0-∞} of EXPAREL^{®} or POSIMIR^{®}.

In some aspects, the mean AUC_{0-∞} of the formulations described herein is about 155% to about 220% of the AUC_{0-∞} of EXPAREL^{®} or POSIMIR^{®}.

In some aspects, the mean AUC_{0-∞} of the formulations described herein is about 210% to about 275% of the AUC_{0-∞} of EXPAREL^{®} or POSIMIR^{®}.

In some aspects, the mean AUC_{0-∞} of the formulations described herein is about 265% to about 325% of the AUC_{0-∞} of EXPAREL^{®} or POSIMIR^{®}.

In some aspects, the mean AUC_{0-∞} of the formulations described herein is about 315% to about 370% of the AUC_{0-∞} of EXPAREL^{®} or POSIMIR^{®}.

In some aspects, the mean AUC_{0-∞} of the formulations described herein is about 60%, about 65%, about 70%, about 75%, about 80%, about 85%, about 90%, about 95%, about 100%, about 105%, about 110%, about 115%, about 120% about 125%, about 130%, about 135%, about 140%, or about 145% of the AUC_{0-∞} of EXPAREL^{®} or POSIMIR^{®}. In some aspects, the mean AUC_{0-∞} of the formulations described herein is about 150%, about 155%, about 160%, about 165%, about 170%, about 175%, about 180%, about 185%, about 190%, about 195%, about 200%, about 205%, about 210%, about 215%, about 220%, about 225%, about 230%, about 235%, about 240%, about 245%, about 250%, about 255%, about 260%, about 265%, about 270%, about 275%, about 280%, about 285%, about 290%, about 295%, about 300%, about 305%, about 310%, about 315%, about 320%, about 325%, about 330%, about 335%, about 340%, about 345%, about 350%, about 355%, about 360%, about 365%, about 370%, or about 375% of the AUC_{0-∞} of EXPAREL^{®} or POSIMIR^{®}.

In some aspects, the mean AUC_{0-7 days} of the formulations described herein is about 800 day*ng/mL to about 1200 day*ng/mL.

In some aspects, the mean AUC_{0-7 days} of the formulations described herein is about 800 day*ng/mL, about 850 day*ng/mL, about 900 day*ng/mL, about 950 day*ng/mL, about 1000 day*ng/mL, about 1050 day*ng/mL, about 1100 day*ng/mL, about 1150 day*ng/mL, or about 1200 day*ng/mL.

### EXAMPLES

The following are non-limiting, illustrative examples, and are not intended to limit the scope of the claims.

### Example 1. A Method of Producing a Microsphere Formulation

Included here is an example of a method to produce a microsphere formulation of bupivacaine. This example is not intended to be limiting.
1. Provide a first phase comprising:
   (a) a first biodegradable polymer, where the first biodegradable polymer is a polyorthoester, a polylactide, a polyglycolide, or a poly(lactide-co-glycolide) copolymer; and
   (b) about 250 mg to about 1200 mg of bupivacaine free base or a pharmaceutically acceptable salt of bupivacaine.
2. Add a second phase comprising an aqueous surfactant continuously into the first phase to form an emulsion, where the aqueous surfactant can be one or more of a cationic surfactant, an anionic surfactant, or a non-ionic surfactant.
3. Add a quench solution to the emulsion to produce a volume comprising a microsphere.
4. Wash, filter, and dry the microsphere to reduce solvent content.

### Example 2. Exemplary Microsphere Formulation

A composition of microspheres containing about 35% bupivacaine free base is diluted in an appropriate diluent solution to produce an injectable long-acting formulation of bupivacaine-loaded microspheres.

**Table 1. Formulation for Injection**

| **Component** | **Amount** |
|---|---|
| Microsphere containing 35% bupivacaine free base (1050 mg) | 3000 mg |
| Diluent Solution (below) | 5 mL to 20 mL |

**Table 2. Diluent Solution**

| **Component** | **Amount** |
|---|---|
| Carboxymethyl cellulose (CMC) | 0-30 mg/mL |
| Polysorbate 20 | 0-30 mg/mL |
| Sodium chloride | q.s. to isotonicity |
| Sodium phosphate (monobasic), sodium phosphate (dibasic), or citric acid | q.s. to pH 3-7 |
| Hydrochloric acid or sodium hydroxide | q.s. to pH 3-7 |
| WFI | q.s. to final volume |

### Example 3. Long-Acting Release Profile Simulations

One-compartment model simulations of bupivacaine release were performed using an initial burst of 10% active drug load and 0.7%/hr or 0.8%/hr second phase release, with or without a delay of onset for a steady-state second phase of release. Seven simulations were carried out, according to the parameters of Table 3.

**Table 3. Simulations of Bupivacaine Release from Microsphere Formulations**

| **Formulation** | **Loading Dose (mg)** | **Burst Release** | **2^{nd} Phase Release (per hour)** | **Delay of 2^{nd} Phase Release (hour)** |
|---|---|---|---|---|
| 1 | 800 | 10% | 0.7% | 0 |
| 2 | 800 | 10% | 0.7% | 6 |
| 3 | 800 | 10% | 0.8% | 6 |
| 4 | 1000 | 10% | 0.7% | 12 |
| 5 | 1000 | 10% | 0.7% | 24 |
| 6 | 1000 | 10% | 0.8% | 12 |
| 7 | 1000 | 10% | 0.8% | 24 |

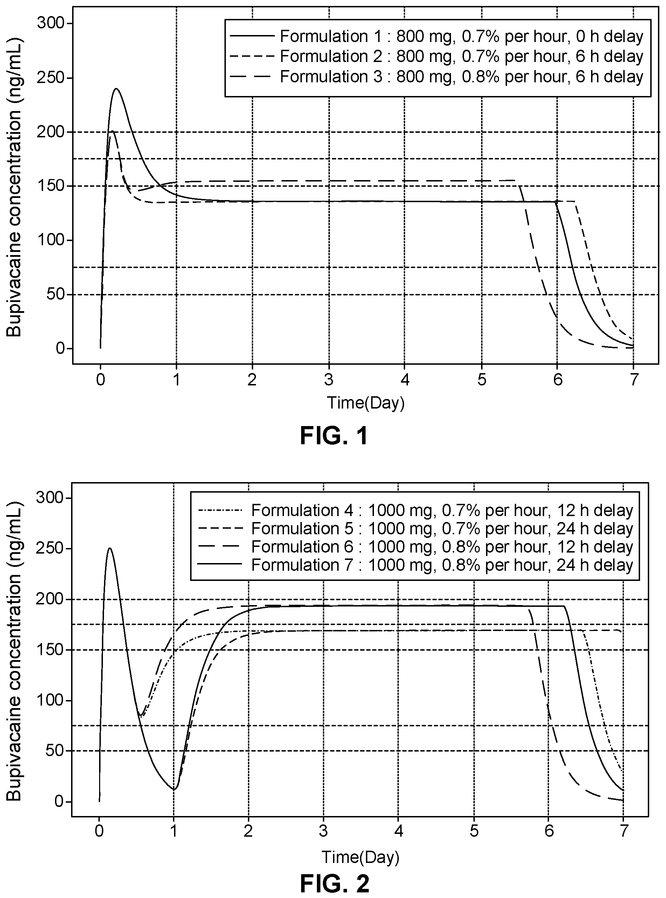

Results of the simulations are organized into FIG. 1, which corresponds to 800 mg active load microsphere formulations.

As shown in FIG. 1, delayed release of 2nd phase in Formulation 2 (6 hour delay) decreased the peak concentration of bupivacaine, but maintained the same steady state concentration and had a longer duration of steady state, when compared to Formulation 1 (no delay). Formulations 2 (0.7%/hr steady-state release) and 3 (0.8%/hr steady-state release), which had the same 6 hour delay, had the same peak concentration. This peak concentration was lower than that of Formulation 1 (no delay, 0.7%/hr release). These data suggest the peak concentration in Formulation 1 was contributed to by both burst release and immediate 2nd phase release. With its higher release rate, Formulation 3 (0.8%/hr steady-state release) exhibited a higher concentration through day 5, but a shorter duration, when compared to Formulation 2 (0.7%/hr steady-state release)

FIG. 2, which corresponds to 1000 mg active load microsphere formulations.

As shown in FIG. 2, 1000 mg active drug load demonstrated higher peak concentration and steady state concentration when compared to 800 mg (FIG. 1). Formulations 4-7 demonstrated equal burst release and peak concentrations. Comparing a 12 hour delay to a 24 hour delay, steady state concentrations were equal, but 24 hour delayed formulations (5 and 7) had a later onset to steady state and a delayed decrease in concentration following steady state, relative to 12 hour delayed formulations (4 and 6). As with 800 mg formulations (FIG. 1), a higher release rate of 0.8%/hr (Formulations 6 and 7) resulted in a higher steady state concentration, but a shorter duration of steady state relative to 0.7%/hr formulations (4 and 5).

Table 4 summarizes the simulation results.

**Table 4. Simulation Result Summary**

| **Formulation** | **Loading Dose (mg)** | **Burst Release** | **2^{nd} Phase Release (per hour)** | **Delay of 2^{nd} Phase Release (hour)** | **Peak 1 (ng/mL)** | **Peak 2 (ng/mL)** | **D5 (ng/mL)** | **D7 (ng/mL)** | **AUCO-7 days (day*ng/mL)** |
|---|---|---|---|---|---|---|---|---|---|
| 1 | 800 | 10% | 0.7% | 0 | 240 | 135 | 135 | 3 | 895 |
| 2 | 800 | 10% | 0.7% | 6 | 201 | 135 | 135 | 8 | 894 |
| 3 | 800 | 10% | 0.8% | 6 | 201 | 155 | 155 | 0 | 896 |
| 4 | 1000 | 10% | 0.7% | 12 | 251 | 169 | 169 | 29 | 1113 |
| 5 | 1000 | 10% | 0.7% | 24 | 251 | 169 | 169 | 163 | 1071 |
| 6 | 1000 | 10% | 0.8% | 12 | 251 | 194 | 194 | 1 | 1120 |
| 7 | 1000 | 10% | 0.8% | 24 | 251 | 194 | 194 | 12 | 1117 |

### Example 4. Synthesis of multi-block copolymers

This example describes the synthesis and characterization of multiblock copolymers used in the preparation of bupivacaine-loaded microspheres.

[Poly(D,L-Lactide)-co-poly(ethylene glycol)-co-poly(D,L-lactide)]-b-[poly(glycolide-co-L-lactide)] multi-block copolymers with 10/90 and 20/80 block ratio were synthesized using similar procedures as described in WO 2013/015685. In brief, poly(DL-Lactide)-co-PEG1000-co-poly(DL-Lactide) pre-polymer with Mₙ of around 2000 g/mol (abbreviated as "PDLA-PEG1000-PDLA" or "LP10L20") was prepared by ring-opening polymerisation of D,L-Lactide using poly(ethylene glycol) ("PEG") with a molecular weight 1000 g/mol (abbreviated as "PEG1000") as initiator and stannous octanoate as a catalyst. Poly(glycolide-co-L-lactide) pre-polymers with Mₙ of about 4000 g/mol (abbreviated as "PGLLA" or "GLL40") were synthesised by solution ring-opening polymerisation of glycolide and L-lactide with a 15/85 monomer ratio in*p-*dioxane using 1,4-butanediol as initiator and stannous octanoate as a catalyst. Molecular weight and chemical structure of the pre-polymers were analysed by ¹H-NMR.

[PDLAPEG1000-PDLA]-b-[PGLLA] multi-block copolymers with a block ratio of 10/90 or 20/80 w/w were prepared by chain-extension of the PDLA-PEG1000-PDLA prepolymer with the PGLLA prepolymer in *p*-dioxane using 1,4butanediisocyanate as a chain extender followed by freeze-drying to remove *p*-dioxane. The polymers are abbreviated as "10LP10L20-GLL40" and "20LP10L20-GLL40".

A [poly(D,L-Lactide)-co-poly(ethylene glycol)-co-poly(D,L-lactide)]-b-[poly(p-dioxanone)] multi-block copolymer with a block ratio of 60/40 (abbreviated as "60LP2L20-D27") was synthesized using similar procedures as described in WO 2020/071912. 60LP2L20-D27 is a multi-block copolymer composed of a [poly(D,L-lactide)-co-poly(ethylene glycol)-co-poly(D,L-lactide)] [PDLA-PEG200-PDLA] pre-polymer segment (A) with a molecular weight of 2000 g/mole (containing 10 mole% of polyethylene glycol with a molecular weight of 200 g/mole) and a semi-crystalline poly(p-dioxanone) pre-polymer segment (B) with a molecular weight of 2700 g/mole which are chain extended in a 60/40 wt.% block ratio by 1,4-butanediisocyanate

All polymers were analysed for their chemical composition, intrinsic viscosity, residual p-dioxane content, and thermal characteristics.

The chemical composition (monomer ratio) and molecular weight (Mₙ) of the prepolymers, as well as the block ratio of the multi-block copolymers was determined by ¹H-NMR. For this determination a Bruker Avance DRX 500 MHz NMR spectrometer B AV500 was used equipped with Bruker Automatic Sample Changer BACS 60 (VARIAN^{®}) operating at 500 MHz. The d₁ delay time was set to 20 s, and the number of scans was 16. Spectra were recorded from 0 to 14 ppm. ¹H-NMR samples were prepared by adding about 1.3 g of deuterated chloroform to about 25 mg of polymer.

Intrinsic viscosity was measured using an Ubbelohde Viscosimeter (DIN), type 0C, SI ANALYTICS^{®} The measurements were performed in chloroform at 25 °C. The temperature was controlled using a water bath. The polymer concentration in chloroform was such that the relative viscosity was in the range of 1.2-2.0.

Residual p-Dioxane content was determined using a gas chromatography flame ionization detection ("GC-FID") headspace method. Measurements were performed on a GC-FID Combi Sampler supplied with an AGILENT^{®} Column, DB624 / 30 m / 0.53 mm. Samples were prepared in dimethylsulphoxide ("DMSO"). Residual solvent content was determined using p-dioxane calibration standards.

Modulated differential scanning calorimetry ("MDSC") was used to determine the thermal behaviour of the multi-block copolymers using a Q2000 MDSC (TA INSTRUMENTS^{®}, Ghent, Belgium). About 5-10 mg of dry material was accurately weighed and heated under a nitrogen atmosphere from -85 °C to 120 °C at a heating rate of 2 °C/min and a modulation amplitude of +/- 0.42°C every 80 seconds. The glass transition temperature (T_{g}, midpoint), melting temperature (maximum of endothermic peak, Tₘ) were determined from the reversing heat flow of the first heating run, whereas the melting enthalpy (ΔHₘ), was calculated from the sum of the surface areas of the melting endotherms of the reversing and non-reversing heat flow of the first heating run. Temperature and enthalpy were calibrated using an indium standard.

Table 5 lists the characteristics of the various multi-block copolymers.

**Table 5. Characteristics of multi-block copolymers used for the preparation of bupivacaine-loaded microspheres.**

| **RCP** | **Polymer grade** | **Block ratio (w/w)** | **PEG MW (g/mol)** | **IV (dl/g)** | **Residual dioxane (ppm)** | **Tg (°C)** | **Tₘ (°C)** | **ΔHₘ (J/g)** |
|---|---|---|---|---|---|---|---|---|
| 1926 | 60LP2L20-D27 | 60/40 | 200 | 0.66 | 533 | 31 | 85 | 50 |
| 1920A | 10LP10L20-GLL40 | 10/90 | 1000 | 0.85 | > 2384 | 43 | - | - |
| 1919 | 20LP10L20-GLL40 | 20/80 | 1000 | 0.85 | > 2391 | 35 | - | - |

### Example 5. General procedures for preparation and characterization of bupivacaine loaded microspheres

### Preparation of bupivacaine loaded microspheres

A 4.0 wt.% polyvinyl alcohol ("PVA") solutions were prepared by dissolving 40 g of PVA with a viscosity of 4.3-5.7 mPa·s (40 g/L,; water) and a degree of hydrolysis (USP) of 85-89% (PVA 5-88 EMPROVE^{®} ESSENTIAL, Merck KGaA) or PVA with a viscosity of 3.4-4.6 mPa·s (40 g/L,; water) and a degree of hydrolysis (USP) of 85-89% (PVA 4-88 EMPROVE^{®} ESSENTIAL, Merck KGaA) in 960 g of ultrapure (UP)-water heated at 75 °C, followed by filtration of the solution over a 5 µm filter at room temperature. A 0.4 wt.% PVA solution with 5% NaCl was prepared by diluting 500 g of the 4 wt% PVA solution with 4250 g UP water and adding 250 g of NaCl under stirring. After complete dissolution of NaCl, the resulting solution was filtered over 0.2 µm polyethersulfone ("PES") filter capsule. Similarly, a 0.4 wt.% PVA solution with 100 mM tris(hydroxymethyl)aminomethane ("TRIS") pH 8.5 was prepared by diluting 500 g of the 4 wt% PVA solution with 4000 g UP water and adding 70 g of TRIZMA^{®} pellets pH 8 under stirring. After complete dissolution of TRIZMA^{®} pellets the pH was set to 8.5 by adding 10M NaOH and additional UP water was added to 5 kg total weight. The resulting solution was filtered over 0.2 µm PES filter capsule.

A 0.05 wt% Tween-80 solution was prepared by adding 2.5 g of Tween into 5 L of UP water and stirring the solution for 15 minutes at room temperature.

0.6% aqueous carboxymethyl cellulose ("CMC") solution (injection vehicle / diluent) was prepared by dissolving 3 g of CMC in 500 g of water for injection ("WFI") under stirring at a temperature of 75 °C. Upon dissolution of all CMC, 50 g of phosphate buffered saline ("PBS") (10X, pH 7.4) and additional WFI was added to obtain the required concentration.

Bupivacaine-loaded microspheres with a target loading of 30-50 wt.% were prepared via oil-in-water ("O/W") membrane emulsification followed by solvent extraction/evaporation. Polymer and bupivacaine base (molecular weight 288 g/mole) were both dissolved in dichloromethane ("DCM") to the desired concentrations to form the dispersed phase ("DP") and stirred overnight to assure complete dissolution of polymer and bupivacaine.

Following filtration over a 0.2 µm polytetrafluoroethylene ("PTFE") filter, DP was emulsified with aqueous 0.4 wt.% PVA solution (containing either 5 wt% NaCl or 100mM TRIS pH 8.5) based continuous phase ("CP")) by pumping DP at a controlled flow rate via a membrane with 20 µm pores into a stirred vessel into which CP was pumped at a CP/DP ratio of 100 v/v. The formed O/W emulsion was stirred at 200 rpm for 3 hours at room temperature under an airflow of 5 L/min to extract and evaporate DCM and harden the microspheres. After completion of solvent evaporation, the hardened microspheres were collected by filtration and washed three times with 250 mL 0.05 wt.% Tween-80 solution and three times with 250 mL WFI. Finally the microspheres were transferred to 10 mL lyophilization vials, frozen at -70°C and lyophilized according to the program detailed in Table 6.

**Table 6. Lyophilization cycle used for vacuum drying of bupivacaine loaded microspheres**

| **Step #** | **Phase** | **Time (hh:mm)** | **Shelf Temp (°C)** | **Vacuum (mbar)** |
|---|---|---|---|---|
| **01** | Load | - | -45 | - |
| **02** | Freeze | 01:00 | -45 | - |
| **03** | Main dry | 00:10 | -10 | 2.0 |
| **04** | Main dry | 02:00 | -10 | 2.0 |
| **05** | Final dry | 06:00 | 20 | 2.0 |
| **06** | Final dry | 06:00 | 20 | 0.001 |
| **07** | Final dry | 00:30 | 30 | 0.001 |
| **08** | Final dry | 02:00 | 30 | 0.001 |
| **09** | Final dry | 00:30 | 5 | 0.001 |
| **10** | Final dry | 72:00 | 5 | 0.001 |

### Characterization of bupivacaine loaded microspheres

Microspheres were analysed for their particle size distribution ("PSD") using a HORIBA^{®} LA-960 Laser Particle Size Analyser.

Characterization of microsphere surface morphology and particle shape was performed by scanning electron microscopy ("SEM") using a JEOL^{®} JCM-5000 NEOSCOPE^{™}.

Bupivacaine content of the microspheres was determined by elemental analysis. In brief, 2.5-5 mg of bupivacaine loaded microspheres, bupivacaine and polymer were accurately weighed in a tin foil and combusted at 1150°C in an ELEMENTAR^{®} Micro Cube with an excess of oxygen to ensure complete sample combustion. The formed N₂, CO₂, H₂O and SO₂ gasses were retained by an adsorption column and eluted separately and analysed using a thermal conductivity detector. By comparing the nitrogen content of the bupivacaine loaded microspheres with that of bupivacaine and polymer, the bupivacaine content of the bupivacaine loaded microspheres was calculated.

The *in vitro* release kinetics of the prepared bupivacaine microspheres was determined by incubating 10 mg of bupivacaine microsphere in 50 mL polypropylene centrifuge tube containing 45 ml *in vitro* release buffer (100 mM PO₄ buffer, 0.025% Tween-20, 0.02% NaN₃, 290 mOsm/Kg, pH 6.5) which were placed in a climate chamber thermostated at 37 °C. At predetermined time points (2 and 6 hours, 1, 2, 4 and 7 days and subsequently twice a week until completion of release), following centrifugation of the vials, aliquots of 100 µL release buffer were collected. Bupivacaine concentrations in the release buffer were determined via reversed phase ultra performance liquid chromatography ("UPLC") with UV-detection using a Waters Acquity H-Class UPLC system, equipped with a PDA or UV detector, an Acquity BEH C18 column (50 x 2.1 mm, 1.7 µm), maintained at 40°C. Mobile phase A consisted of a 20 mM phosphate buffer pH 6.5 and acetonitrile ("ACN") at a ratio of 90 : 10 v/v and 100% of acetonitrile was used as mobile phase B. The mobile phase composition started at 30% B and increased to 70%B in 2 minutes, at a constant flow rate of 0.600 mL/min. Detection was performed at 235 nm.

### Example 6. Preparation of bupivacaine loaded microspheres composed of 60LP2L20-D27 and 10LP10L20-GLL40

Bupivacaine loaded microspheres with a target loading of 34 wt.% bupivacaine were prepared of 60LP2L20-D27 and 10LP10L20-GLL40 according to the O/W membrane emulsification procedure described in Example 5. In brief, 1.0 g polymer was dissolved in DCM to a final polymer concentration of 10 wt.% and 500 mg bupivacaine base was added. In case of 60LP2L20-D27, the solution was stirred overnight and additionally heated for one hour at 35 °C to assure complete dissolution of polymer and bupivacaine. Following filtration over a 0.2 µm PTFE filter, DP was emulsified with an aqueous 0.4 wt.% PVA 5-88 5% NaCl based CP by pumping DP at a flow rate of 1.6 mL/min via a membrane with 20 µm pores into a stirred vessel into which CP solution was pumped at a CP/DP ratio of 100 v/v. The O/W emulsion was stirred (stirring speed 9 V) for 3 hours at room temperature under an airflow of 5 L/min to extract and evaporate DCM and harden the microspheres. After completion of solvent evaporation, the microspheres were collected by filtration, washed with 0.05 wt.% aqueous Tween-80 solution and WFI and finally dried by lyophilization as described in Example 5.

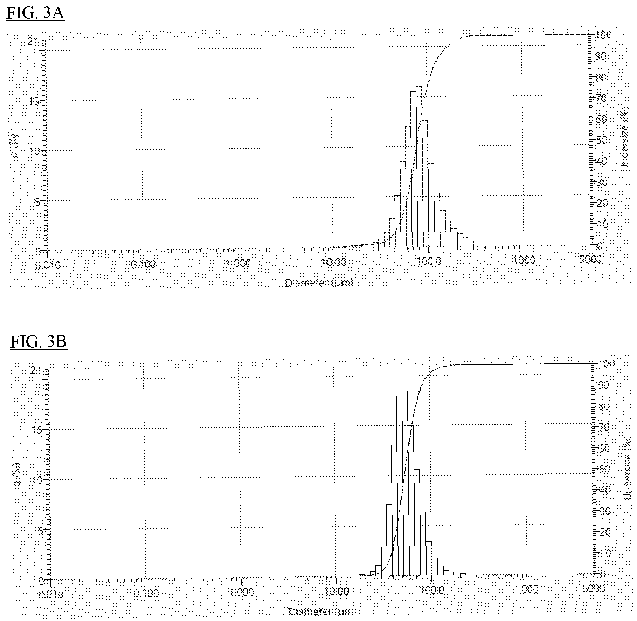

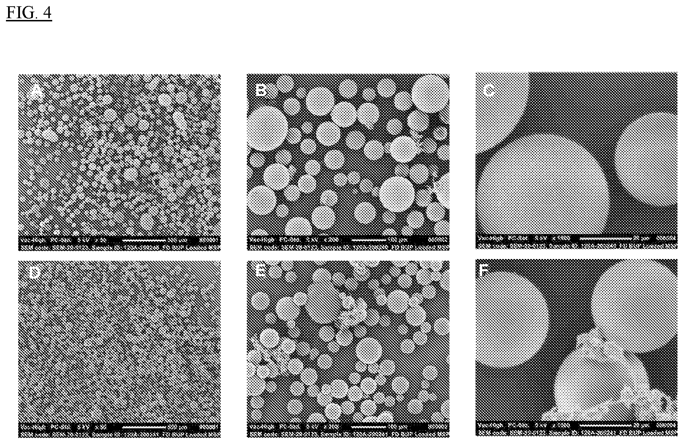

The resulting microspheres were analysed according to the methods described in Example 5. The 60LP2L20-D27 based bupivacaine microspheres (batch 120A-200240) had an average particle size of 79 µm whereas the 10LP10L20-GLL40 based bupivacaine microspheres (batch 120A-200241) had an average particle size of 54 µm. FIG. 3 shows the particle size distribution of the 60LP2L20-D27 based bupivacaine microspheres (120A-200240) and the 10LP10L20-GLL40 based bupivacaine microspheres (120A-200241). Scanning electron microscopy shows that the O/W microencapsulation process yielded spherical microspheres with a smooth surface without any pores (FIGs. 4B-4D).

The bupivacaine content of the 60LP2L20-D27 microspheres as measured via elemental analysis was 25.3 wt.% representing an encapsulation efficiency ("EE") of 74.8%, whereas bupivacaine content of the 10LP10L20-GLL40 (15/85) microspheres was 21.8 wt.% (EE 64.8%).

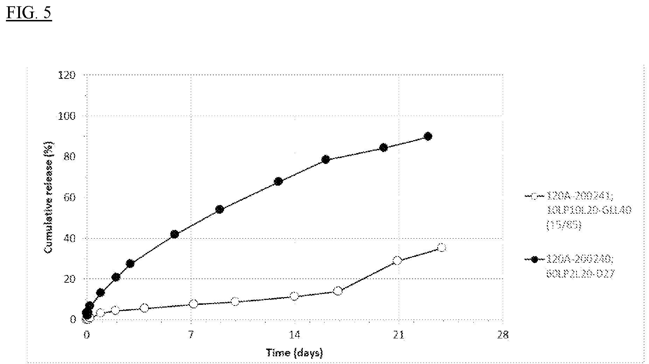

FIG. 5 shows the cumulative release of bupivacaine from 60LP2L20-D27 and 10LP10L20-GLL40 based bupivacaine microspheres. 60LP2L20-D27 based microspheres released around 85% of encapsulated bupivacaine gradually over three weeks whereas 10LP10L20-GLL40 based bupivacaine microspheres only released around 40% of encapsulated bupivacaine in the same period.

### Example 7. Preparation of 60LP2L20-D27-based microspheres with increased bupivacaine loading and faster release

To increase the bupivacaine loading and bupivacaine release rate a series of 60LP2L20-D27 based bupivacaine loaded microspheres were prepared according to the O/W membrane emulsification procedure described in Example 5 while systematically varying critical formulation and process parameters. (Table 7). The bupivacaine loading of the microspheres was increased by using a higher bupivacaine to polymer ratio. Furthermore, as smaller microspheres will result in a larger surface to volume ratio, which has an enhancing effect on the release rate, the size of the microspheres was decreased by increasing the stirring speed and/or reducing the DP injection flow rate. Other parameters that were varied include polymer concentration and CP composition (PVA grade and buffer type).

In brief, both polymer and bupivacaine were dissolved in DCM to the desired concentrations. Following filtration over a 0.2 µm PTFE filter, DP was emulsified with aqueous 0.4 wt.% PVA by pumping DP at a controlled flow rate via a membrane with 20 µm pores into a stirred vessel into which CP solution was pumped at a CP/DP ratio of 100 v/v. The O/W emulsion was stirred for 3 hours at room temperature under an air flow to allow extraction and evaporation of DCM and hardening of the microspheres. Subsequently, the microspheres were collected by filtration, washed with 0.05 wt.% aqueous Tween-80 solution and WFI and finally dried by lyophilization.

**Table 7 - Formulation and process settings used for preparation of bupivacaine-loaded 60LP2L20-D27 microspheres with increased bupivacaine loading.**

| **120A batch** | **Pol conc wt%** | **Target drug load (wt%)** | **CP/DP (v/v)** | **DP Inj mL/ min** | **CP Inj (RPM)** | **Stir. Rate (rpm)** | **CP** | | |
|---|---|---|---|---|---|---|---|---|---|
| | | | | | | | **PVA grade** | **Wt%** | **Buffer*** |
| 200300 | 10 | 35.7 | 100 | 1.6 | 80 | 900 | 4-88 | 0.4 | Tris 8.5 |
| 200301 | 15 | 35.3 | 100 | 1.6 | 80 | 900 | 4-88 | 0.4 | Tris 8.5 |
| 200307 | 15 | 50.3 | 100 | 1.6 | 80 | 900 | 4-88 | 0.4 | Tris 8.5 |
| 200314 | 15 | 49.9 | 100 | 1 | 50 | 1300 | 4-88 | 0.4 | Tris 8.5 |
| 200341 | 15 | 50.3 | 100 | 1 | 50 | 1300 | 5-88 | 0.4 | 5% NaCl |

| | | | | | | | | | |
|---|---|---|---|---|---|---|---|---|---|
| *Tris 8.5 is 100 mM Tris buffer, pH 8.5 | | | | | | | | | |

**Table 8. Characteristics of bupivacaine-loaded 60LP2L20-D27 microspheres with increased bupivacaine loading (120A-200240 was added as reference)**

| **Batch code (120A-)** | **Polymer grade** | **PSD BUP MSP*** | | | **BUP load (wt%)** | **EE (%)** |
|---|---|---|---|---|---|---|
| | | **D 10 (µm)** | **D 50 (µm)** | **D 90 (µm)** | | |
| 200240 | 60LP2L20-D27 | 49 | 79 | 134 | 25.3 | 74.8 |
| 200300 | 60LP2L20-D27 | 37 | 52 | 85 | 24.5 | 68.6 |
| 200301 | 60LP2L20-D27 | 36 | 51 | 307 | 21.0 | 59.4 |
| 200307 | 60LP2L20-D27 | 39 | 52 | 70 | 39.4 | 78.4 |
| 200314 | 60LP2L20-D27 | 28 | 37 | 49 | 37.5 | 75.1 |
| 200341 | 60LP2L20-D27 | 31 | 41 | 55 | 42.8 | 86.1 |

| | | | | | | |
|---|---|---|---|---|---|---|
| *PSD BUP MSP means particle size distribution of bupivacaine microspheres with Dv10 (distribution percentile) meaning that 10% of the particles have a diameter smaller than the table value), Dv50 (distribution percentile) is median particle size) and Dv90 (distribution percentile) meaning that 90% of the particles have a diameter smaller than the table value). | | | | | | |

The microspheres were analysed according to the methods described in Example 5. By increasing the stirring speed and decreasing the injection speed of the DP in the dispersion cell, microspheres size could be reduced. The average particle size (Dv50) of the new 60LP2L20-D27 based bupivacaine microspheres ranged from 37 to 52 µm, which was significantly smaller as compared to the average particle size of 79 µm of batch 120A-200240 prepared in Example 6.

The bupivacaine loading was successfully increased to 37-43% by using a higher bupivacaine to polymer ratio in combination with increasing the polymer concentration and use of Tris pH 8.5 buffer-based CP due to which the encapsulation efficiency could be increased to almost 90%.

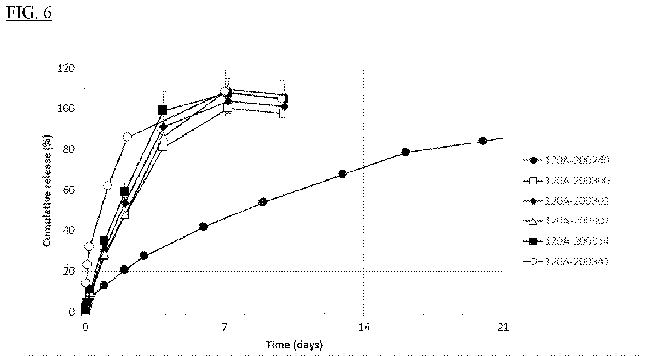

FIG. 6 shows the cumulative release of bupivacaine from the 60LP2L20-D27 based bupivacaine microspheres prepared using the adjusted formulation and process settings. All formulations showed 100% release within a week, which was significantly faster as compared to release of bupivacaine from the 60LP2L20-D27-based bupivacaine microspheres prepared in Example 6. Batch 120A-200341 was prepared with CP containing 5% NaCl and exhibited significantly faster release as compared to the batches that were prepared with TRIS pH 8.5.

### Example 8. Preparation of xxLP10L20-GLL40-based microspheres with increased bupivacaine loading and faster release

To obtain bupivacaine loaded microspheres with higher bupivacaine loading and faster bupivacaine release as compared to the 10LP10L20-GLL40 15/85) based bupivacaine microspheres (120A-200241) in Example 6, bupivacaine loaded microspheres with a target loading of 50 wt% bupivacaine were prepared of 10LP10L20-GLL40 (15/85) and 20LP10L20-GLL40 (15/85) according to the general procedures described in Example 5 using the specific formulation and process settings listed in Table 9. In brief, both polymer and bupivacaine were dissolved in dichloromethane (DCM) to obtain a solution of 15 wt% polymer and 15 wt% bupivacaine in DCM. Following filtration over a 0.2 µm PTFE filter, DP was emulsified with aqueous 0.4 wt.% PVA by pumping DP at a controlled flow rate via a membrane with 20 µm pores into a stirred vessel into which CP solution was pumped at a CP/DP ratio of 100 v/v. The O/W emulsion was stirred for 3 hours at room temperature under an air flow to allow extraction and evaporation of DCM and hardening of the microspheres. Subsequently, the microspheres were collected by filtration, washed with 0.05 wt.% aqueous Tween-80 solution and WFI and finally dried by lyophilization.

**Table 9. Formulation and process settings used to increase bupivacaine loading and release rate of 10LP10L20-GLL40 and 20LP10L20-GLL40 based bupivacaine microspheres**

| **Batch code (120A-)** | **Polymer grade** | **Pol conc wt%** | **Target drug load (wt%)** | **CP/DP (v/v)** | **DP Inj mL/ min** | **CP Inj (RPM)** | **Stir. Rate (rpm)** | **CP** | | |
|---|---|---|---|---|---|---|---|---|---|---|
| | | | | | | | | **PVA grade** | **Wt, %** | **Buffer** |
| 200308 | 10LP10L20-GLL40 | 15 | 49.6 | 100 | 1.6 | 80 | 900 | 4-88 | 0.4 | Tris 8.5 |
| 200408 | 20LP10L20-GLL40 | 14.4 | 49.8 | 100 | 1 | 50 | 1300 | 5-88 | 0.4 | 5% NaCl |

The microspheres were analysed according to Example 5. Bupivacaine microspheres prepared with polymer 10LP10L20-GLL40 (Batch 120A-200308) had a high bupivacaine loading of 44 wt%, corresponding to an encapsulation efficiency of 89% and an average particle size of 57 µm (Table 10). Bupivacaine loaded microspheres prepared with the 20LP10L20-GLL40 polymer (Batch 120A-200408) had comparable high bupivacaine loading and encapsulation efficiency (44 wt% and 89%). However, due to a higher stirring-and slower DP/CP injection speed, the average particle size was smaller (43 µm, Table 10).

**Table 10. Characteristics of 10LP10L20-GLL40- and 20LP10L20-GLL40-based bupivacaine microspheres with increased bupivacaine loading (120A-200241 was added as reference)**

| **Batch code (120A-)** | **Polymer grade** | **PSD BUP MSP** | | | **BUP load (wt%)** | **EE (%)** |
|---|---|---|---|---|---|---|
| | | **D 10 (µm)** | **D 50 (µm)** | **D 90 (µm)** | | |
| 200241 | 10LP10L20-GLL40 | 38 | 54 | 83 | 21.3 | 74.8 |
| 200308 | 10LP10L20-GLL40 | 42 | 57 | 82 | 44.4 | 89.4 |
| 200408 | 20LP10L20-GLL40 | 32 | 43 | 57 | 44.3 | 88.2 |

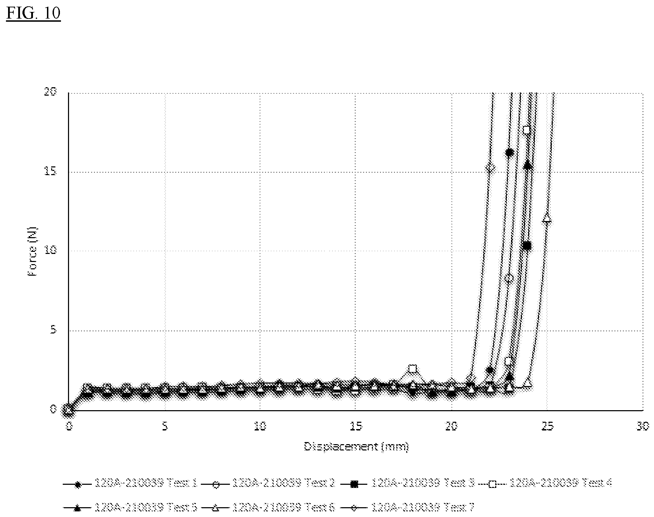

As shown in FIG. 10, microspheres based on polymer 10LP10L20-GLL40 (15/85) with an increased bupivacaine loading of 44.4% (batch 120A-200308) had comparable *in vitro* release kinetics as the 10LP10L20-GLL40 microsphere with lower bupivacaine loading of 21.8% (120A-200241) prepared in Example 6. Bupivacaine microspheres prepared with 20LP10L20-GLL40 (15/85) with similar bupivacaine loading (44.3 %) (120A-200408) showed significantly faster bupivacaine release, which is attributed to the increased amount of PEG as compared to polymer 10LP10L20-GLL40 (15/85).

### Example 9. Manufacturing of bupivacaine microspheres at 5 g scale

60LP2L20-D27- and 20LP10L20-GLL40-based bupivacaine microspheres with a target loading of 50 wt% bupivacaine were prepared at a scale of 5 g and analyzed according to the general procedures described in Example 5 using the specific formulation and process settings listed in Table 11. In brief, 2.5 g polymer dissolved in 11 mL DCM to obtain a solution of 15 wt% polymer, whereafter 2.5 g bupivacaine base was added. Following filtration over a 0.2 µm PTFE filter, DP was emulsified with aqueous 0.4 wt.% PVA by pumping DP at a controlled flow rate via a membrane with 20 µm pores into a stirred vessel into which CP solution was pumped at a CP/DP ratio of 100 v/v. The O/W emulsion was stirred for 3 hours at room temperature under an air flow to allow extraction and evaporation of DCM. Completion of DCM removal (and hardening of the microspheres) was confirmed by measuring DCM concentration in the outgoing air flow by means of a VOC detector. Subsequently, the microspheres were collected by filtration, washed with 0.05 wt.% aqueous Tween-80 solution and WFI and finally dried by lyophilization.

**Table 11. Formulation and process settings used for preparation of bupivacaine microspheres at a scale of 5 g**

| **Batch code (120A-)** | **Polymer grade** | **Pol conc wt%** | **DP Inj mL/ min** | **CP Inj (RPM)** | **Stir. Rate (rpm)** | **CP** | | |
|---|---|---|---|---|---|---|---|---|
| | | | | | | **PVA grade** | **Wt. %** | **Buffer** |
| 210039 | 60LP2L20-D27 | 15 | 1.6 | 80 | 900 | 4-88 | 0.4 | Tris 8.5 |
| 210040 | 60LP2L20-D27 | 15 | 1 | 50 | 1300 | 5-88 | 0.4 | 5% NaCl |
| 210092 | 20LP10L20-GLL40 | 15 | 1 | 50 | 1300 | 5-88 | 0.4 | 5% NaCl |

The resulting microspheres were analysed according to the methods in Example 5. All batches had a narrow particle size distribution with an average particle size between 41 and 53 µm.

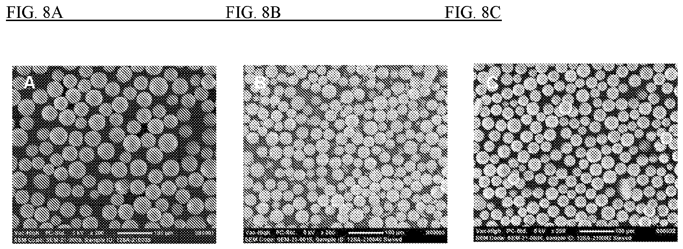

Scanning electron microscopy shows that the O/W microencapsulation process yielded spherical microspheres with a smooth surface without any pores (FIGs 8A-8C), similar to the bupivacaine microspheres prepared in the previous Examples.

**Table 12. Characteristics of 60LP2L20-D27 and 20LP10L20-GLL40-based bupivacaine microspheres prepared at a scale of 5 g.**

| **Batch code (120A-)** | **Polymer grade** | **PSD BUP MSP** | | | **DCM content (ppm)** | **BUP load (wt%)** | **EE (%)** |
|---|---|---|---|---|---|---|---|
| | | **D10 (µm)** | **D 50 (µm)** | **D 90 (µm)** | | | |
| 210039 | 60LP2L20-D27 | 40 | 53 | 73 | < 55 | 41.2 | 81.9 |
| 210040 | 60LP2L20-D27 | 31 | 41 | 55 | 69 | 34.8 | 69.7 |
| 210092 | 20LP10L20-GLL40 | 32 | 43 | 55 | < 55 | 46.3 | 91.4 |

Residual dichloromethane content of the microspheres was determined by gas chromatography with headspace injection and flame-ionization detection. In brief, 100 mg of sample was dissolved in 5.0 mL of DMSO containing octane as the internal standard. The samples were analyzed by GC-Headspace using an Agilent 6850 gas chromatograph, equipped with a Combi-Pal headspace sampler. The calibration range of the method was 18 - 4902 ppm DCM in 100 mg sample, using a first order linear regression (Weighing factor=1/X). The residual dichloromethane content of the bupivacaine microparticles was lower than 70 ppm.

Bupivacaine content of the 60LP2L20-D27-based microspheres as determined by elemental analysis varied from 34.8% (EE 69.7%) for 120A-210040 to 41.2% for 120A-210039 (EE 81.9%). The 20LP10L20-GLL40 (15/85)-based microspheres had a loading of 46.3% representing a high encapsulation of 91.4%.

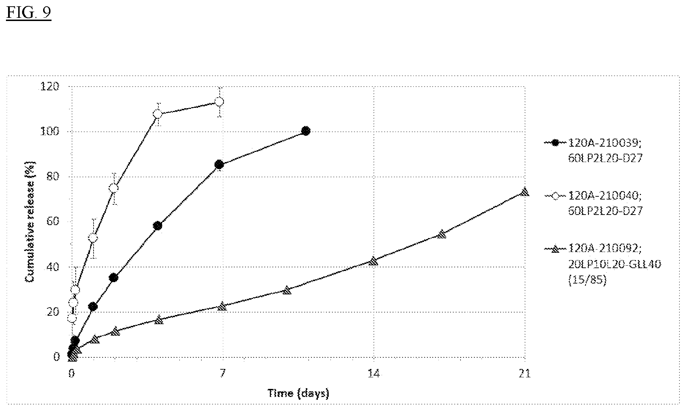

FIG. 9 shows the *in vitro* release of bupivacaine from the 60LP2L20-D27 and 20LP10L20-GLL40 based bupivacaine microspheres prepared at a scale of 5 g. Bupivacaine release from the microspheres prepared at 5 g was similar or slightly slower as compared to bupivacaine release from the same microsphere formulations prepared at 1 g scale in Examples 7 and 8.

### Example 10. Injectability

Injectability of bupivacaine microsphere suspensions in 0.6% aqueous CMC solution was determined by means of a tensile tester. A 100 mg/mL suspension of bupivacaine microspheres was prepared by reconstituting 414 mg of bupivacaine microspheres (120A-210039) in 3.7 mL 0.6 % aqueous CMC solution. Using a 18G needle, 0.5 mL suspension was collected into a 1 mL syringe. After removal of air bubbles the 18G needle was replaced by a 25 G 5/8" needle, whereafter the syringe with needle was positioned vertically in the tensile tester. The suspension was ejected via the needle at a displacement rate of 100 mm/min and the force displacement curve was recorded. At a concentration of 100 mg/mL, bupivacaine microsphere suspensions (0.4 mL, n=7) were well injectable via the 25 G 5/8" needle with injection forces varying from of 1.6 to 2.9 N, well below the acceptance limit of 20 N for injection force.

Additionally, the injectability of more concentrated suspensions was studied. Suspensions with a concentration of 140 and 234 mg/mL were prepared by reconstituting the required amount of bupivacaine microspheres ((120A-200341) in 0.6 % aqueous CMC solution and evaluated for their injectability using the procedures described above.

Both the 140 mg/mL (0.4 mL) and 234 mg/mL (0.3 mL) bupivacaine microsphere suspensions (were well injectable via 23 G 1" and 25 G 5/8" needles (Table 13). The injection force ranged from 0.8 - 6.1 N for 140 mg/mL and 1.3 - 10.3 N for 234 mg/mL. Ejected microsphere suspensions were collected, washed and weighed to determine the recovery (fraction of microspheres ejected). The recovery was ≥ 90% for all tested samples (Table 13).

**Table 13. Results from injectability testing of 60LP2L20-D27-based bupivacaine microspheres (120A-200341), suspended in 0.6 % CMC solution.**

| **Suspension concentration (mg/mL)** | **Injection volume (mL)** | **Needle size** | **Max Injection force (N)** | **Recovery (%)** |
|---|---|---|---|---|
| 140 | 0.4 | 23G | 6.1 | 92 |
| 140 | 0.4 | 23G | 2.6 | 90 |
| 140 | 0.4 | 23G | 1.4 | 101 |
| 140 | 0.4 | 23G | 0.8 | 97 |
| 140 | 0.4 | 23G | 4.9 | 99 |
| 140 | 0.4 | 25G | 2.7 | 102 |
| 234 | 0.3 | 23G | 2.5 | 109 |
| 234 | 0.3 | 23G | 10.3 | 106 |
| 234 | 0.3 | 23G | 1.3 | 105 |
| 234 | 0.3 | 23G | 2.6 | 101 |
| 234 | 0.3 | 25G | 2.0 | 99 |
| 234 | 0.3 | 25G | 1.4 | 91 |

### Example 11. In vivo pharmacokinetic and animal studies

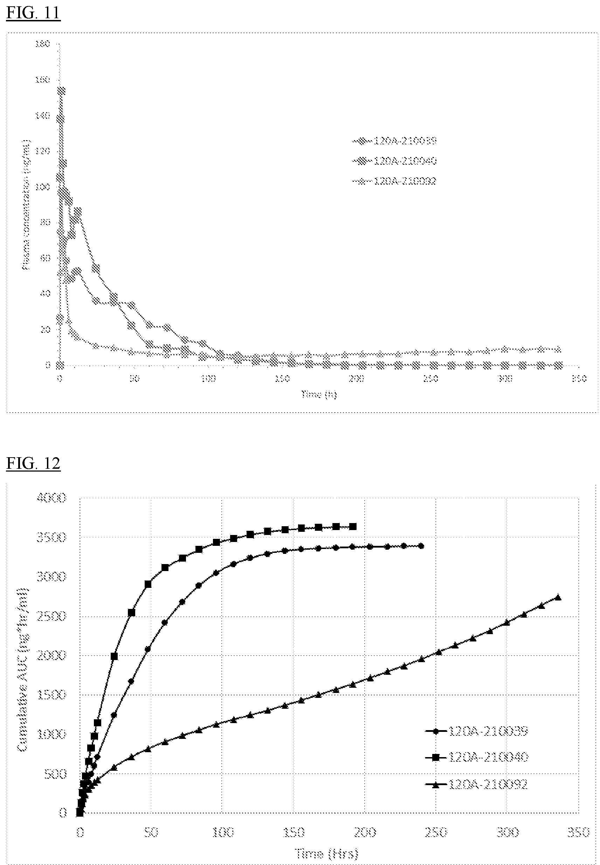

The *in vivo* pharmacokinetics of three exemplary formulations were studied in dogs. The formulations were injected into male Beagle dogs at a dose of 45 mg/dog, as summarized in Table 14. The resulting plasma concentration profiles are shown in FIG. 11, and the extracted pK parameters are provided in Table 15. The cumulative area under the curve (AUC) from the *in vivo* pharmacokinetics of the exemplary bupivacaine formulations are shown in FIG. 12.

**Table 14. Test formulations used in pK study**

| **Test formulation** | **Dose (mg/dog)** | **Dose Volume(mL/dog)** | **Route of Administration** |
|---|---|---|---|
| Test 1 | 45 | 1 | IM |
| (120A-210039) | | | |
| Test 2 (120A-210040) | 45 | 1 | IM |
| Test 3 (120A-210092) | 45 | 1 | IM |

**Table 15. Pharmacokinetic parameters obtained from in vivo dog experiment**

| **PK Parameters** | **Group 1 (Test 1)** | | **Group 2 (Test 2)** | | **Group 3 (Test 3)** | |
|---|---|---|---|---|---|---|
| | **Dose: 45 mg** | | **Dose: 45 mg** | | **Dose: 45 mg** | |
| | **Mean** | **SD** | **Mean** | **SD** | **Mean** | **SD** |
| **Tₘₐₓ (h)^{@}** | 1 | NA | 1 | NA | 1 | NA |
| **Cmax (mg/mL)** | 98.13 | 18.92 | 156.06 | 94.61 | 76.37 | 45.67 |
| **AUC₀₋ₗₐₛₜ (ng•h/mL)** | 3390.31 | 436.53 | 3634.53 | 2685.12 | 2749.09 | 1439.13 |
| **AUC_{0-inf} (ng•h/mL)** | 3425.80 | 451.96 | 3657.92 | 2677.92 | 6602.85 | 5534.33 |
| **T_{1/2} (h)** | 29.93 | 8.5 | 22.46 | 9.95 | 254.07 | 91.70 |

## Claims

1. A stable pharmaceutically acceptable formulation comprising a microsphere, the microsphere comprising:
a biodegradable multiblock copolymer; and
an active drug load of bupivacaine free base or a pharmaceutically acceptable salt of bupivacaine, wherein
the multiblock copolymer comprises at least one hydrolysable prepolymer (A) segment and at least one hydrolysable prepolymer (B) segment, wherein the segments are linked by a multifunctional chain extender, and wherein the segments are randomly and non-alternatingly distributed over the polymer chain,
the prepolymer (A) segment comprises polyethylene glycol,
the prepolymer (A) segment comprises reaction products of glycolide and/or lactide (D and/or L),
the prepolymer (B) segment comprises poly(glycolide-*co*-L-lactide) with a Mₙ of about 4000 g/mol,
the prepolymer (B) segment comprises a molar amount of 5 % to 25 % of glycolide relative to combined molar amount of glycolide and L-lactide,
the multiblock copolymer comprises from 10 % to 99 % of the prepolymer (B) segment relative to the total weight of the multiblock copolymer,
the multifunctional chain extender is 1,4-butane diisocyanate,
wherein the stable pharmaceutically acceptable formulation has a shelf life of 14 days at 25 °C following refrigeration.

2. The stable pharmaceutically acceptable formulation of claim **1,** wherein the multiblock copolymer is amorphous and has a glass transition temperature of 37 °C or less at physiological conditions.

3. The stable pharmaceutically acceptable formulation of any one of claims 1-2, wherein the content of the prepolymer (A) segment in the multiblock copolymer is from 1 % to 90 % based on the total weight of the multiblock copolymer.

4. The stable pharmaceutically acceptable formulation of any one of claims 1-3, wherein the prepolymer (A) segment has a Mₙ of 500 g/mol or more.

5. The stable pharmaceutically acceptable formulation of any one of claims 1-4, wherein the prepolymer (B) segment comprises poly(glycolide-*co*-L-lactide) with Mₙ of about 4000 g/mol.

6. The stable pharmaceutically acceptable formulation of any one of claims 1-5, wherein, the prepolymer (B) segment comprises a molar amount of about 15 % of glycolide relative to the combined molar amount of glycolide and L-lactide.

7. The stable pharmaceutically acceptable formulation of any one of claims 1-6, wherein the active drug load of bupivacaine free base or a pharmaceutically acceptable salt of bupivacaine in the microsphere is 10 % to 80 % w/w.

8. The stable pharmaceutically acceptable formulation of any one of claims 1-7, comprising less than 5 % w/w of a bupivacaine-derived impurity after sealed storage for 24 months at a temperature of 25 °C.

9. The stable pharmaceutically acceptable formulation of any one of claims 1-8, wherein the formulation is provided in an injector.

10. A method of producing a stable pharmaceutically acceptable formulation comprising a microsphere, the method comprising:
providing a first phase comprising:
a biodegradable polymer as defined in any one of claims 1-6; and
an active drug load of bupivacaine free base or a pharmaceutically
acceptable salt of bupivacaine;
adding a second phase comprising an aqueous surfactant continuously into the first phase to form an emulsion;
adding a quench solution to the emulsion to produce a volume comprising a microsphere; and
washing, filtering, and drying the microsphere to reduce solvent content.

11. A method of producing a stable pharmaceutically acceptable formulation comprising a microsphere, the method comprising:
providing a first phase comprising:
a biodegradable polymer as defined in any one of claims 1-6;
an active drug load of bupivacaine free base or a pharmaceutically acceptable salt of bupivacaine; and
a solvent system suitable to dissolve the polymer and bupivacaine;
emulsifying the first phase with a second phase, thereby forming an emulsion;
wherein the second phase comprises an aqueous solution which comprises a surfactant; and
removing a substantial portion of the solvent system from the emulsion, thereby obtaining a microsphere.

12. A stable pharmaceutically acceptable formulation for use in the treatment of pain comprising a microsphere, the microsphere comprising a biodegradable polymer as defined in any one of claims 1-6 and 200 mg to 8000 mg of bupivacaine free base or a pharmaceutically acceptable salt thereof, wherein the pharmaceutical formulation effects a therapeutically effective concentration of bupivacaine free base or a pharmaceutically acceptable salt thereof for 2 days to 14 days following administration to a subject.

13. A pre-filled injector comprising:
a stable pharmaceutically acceptable formulation comprising:
a microsphere comprising:
a biodegradable polymer as defined in any one of claims 1-6; and
an active drug load of bupivacaine free base or a pharmaceutically acceptable salt of bupivacaine.

14. A method of manufacturing a pre-filled injector comprising a stable pharmaceutically acceptable formulation, the method comprising:
preparing a stable pharmaceutically acceptable formulation comprising:
a microsphere comprising:
a biodegradable polymer as defined in any one of claims 1-6; and
an active drug load of bupivacaine free base or a pharmaceutically acceptable salt of bupivacaine.
loading a sterile cartridge with the stable pharmaceutically acceptable formulation; and
attaching the sterile cartridge operably to an injector.

15. A long-acting dosage form comprising:
a microsphere comprising
a biodegradable polymer as defined in any one of claims 1-6; and
500 mg to 4000 mg of bupivacaine free base or a pharmaceutically acceptable salt thereof,
wherein administration of a single dose of the long-acting dosage form to a subject results in at least one of the pharmacokinetic parameters selected from the group consisting of:
a steady state plasma profile of bupivacaine from day 1 to day 7 following administration exhibiting a mean Cmax value no greater than the steady state plasma level of bupivacaine provided by 100 mg of immediate release subcutaneous injection of bupivacaine hydrochloride;
a bupivacaine elimination half-life of 2 hours to 4 hours; and
a zero-order or first order release profile corresponding to 1 % to 50 % release of the total administered dose of bupivacaine or a pharmaceutically acceptable salt thereof per day.

16. A kit comprising:
a first vial comprising a concentrated form of the stable pharmaceutically acceptable formulation of any one of claims 1-9 or of the long-acting dosage form of claim 15;
a second vial comprising a pharmaceutically acceptable diluent;
a first syringe suitable for withdrawing the pharmaceutically acceptable diluent from the second vial;
an adapter which can operably attach to the first syringe and is suitable for dispensing the pharmaceutically acceptable diluent into the first vial;
a second syringe suitable for withdrawing a liquid from the second vial and for injecting the liquid into a subject in need thereof; and
instructions for diluting the concentrated form and for administering the stable pharmaceutically acceptable formulation or the long-acting dosage form to the subject.

## Patentansprüche

1. Stabile pharmazeutisch unbedenkliche, eine Mikrokugel umfassende Formulierung, wobei die Mikrokugel Folgendes umfasst:
ein biologisch abbaubares Multiblock-Copolymer und
eine Wirkstoffbeladung aus der freien Base von Bupivacain oder einem pharmazeutisch unbedenklichen Salz von Bupivacain, wobei
das Multiblock-Copolymer mindestens ein hydrolysierbares Präpolymer(A)-Segment und mindestens ein hydrolysierbares Präpolymer(B)-Segment umfasst, wobei die Segmente über einen multifunktionellen Kettenverlängerer verknüpft sind und wobei die Segmente statistisch und nicht alternierend über die Polymerkette verteilt sind,
das Präpolymer(A)-Segment Polyethylenglykol umfasst,
das Präpolymer(A)-Segment Reaktionsprodukte von Glycolid und/oder Lactid (D und/oder L) umfasst,
das Präpolymer(B)-Segment Poly(glycolid-co-L-lactid) mit einem Mₙ von etwa 4000 g/mol umfasst,
das Präpolymer(B)-Segment eine molare Menge von 5 % bis 25 % Glycolid, bezogen auf die kombinierte molare Menge von Glycolid und L-Lactid, umfasst,
das Multiblock-Copolymer 10 % bis 99 % an Präpolymer(B)-Segment, bezogen auf das Gesamtgewicht des Multiblock-Copolymers, umfasst,
der multifunktionelle Kettenverlängerer 1,4-Butandiisocyanat ist,
wobei die stabile pharmazeutisch unbedenkliche Formulierung nach dem Kühlen eine Haltbarkeit von 14 Tagen bei 25 °C aufweist.

2. Stabile pharmazeutisch unbedenkliche Formulierung nach Anspruch 1, wobei das Multiblock-Copolymer amorph ist und unter physiologischen Bedingungen eine Glasübergangstemperatur von 37 °C oder weniger aufweist.

3. Stabile pharmazeutisch unbedenkliche Formulierung nach einem der Ansprüche 1-2, wobei der Gehalt an Präpolymer(A)-Segment in dem Multiblock-Copolymer 1 % bis 90 %, bezogen auf das Gesamtgewicht des Multiblock-Copolymers, beträgt.

4. Stabile pharmazeutisch unbedenkliche Formulierung nach einem der Ansprüche 1-3, wobei das Präpolymer(A)-Segment eine Mₙ von 500 g/mol oder mehr aufweist.

5. Stabile pharmazeutisch unbedenkliche Formulierung nach einem der Ansprüche 1-4, wobei das Präpolymer(B)-Segment Poly(glykolid-co-L-lactid) mit einer Mₙ von etwa 4000 g/mol umfasst.

6. Stabile pharmazeutisch unbedenkliche Formulierung nach einem der Ansprüche 1-5, wobei das Präpolymer(B)-Segment eine molare Menge von etwa 15 % Glycolid im Verhältnis zu der kombinierten molaren Menge Glycolid und L-Lactid umfasst.

7. Stabile pharmazeutisch unbedenkliche Formulierung nach einem der Ansprüche 1-6, wobei die Wirkstoffbeladung aus der freien Base von Bupivacain oder einem pharmazeutisch unbedenklichen Salz von Bupivacain in der Mikrokugel 10 Gew.-% bis 80 Gew.-% beträgt

8. Stabile pharmazeutisch unbedenkliche Formulierung nach einem der Ansprüche 1-7, umfassend weniger als 5 Gew.-% einer von Bupivacain stammenden Verunreinigung nach 24-monatiger versiegelter Lagerung bei einer Temperatur von 25 °C.

9. Stabile pharmazeutisch unbedenkliche Formulierung nach einem der Ansprüche 1-8, wobei die Formulierung in einem Injektor bereitgestellt wird.

10. Verfahren zur Herstellung einer stabilen pharmazeutisch unbedenklichen, eine Mikrokugel umfassenden Formulierung, wobei das Verfahren Folgendes umfasst:
die Bereitstellung einer ersten Phase, die Folgendes umfasst:
ein biologisch abbaubares Polymer nach einem der Ansprüche 1-6 und
eine Wirkstoffbeladung aus der freien Base von Bupivacain oder einem pharmazeutisch unbedenklichen Salz von Bupivacain,
die kontinuierliche Zugabe einer zweiten, ein wässriges Tensid umfassenden Phase zur ersten Phase unter Bildung einer Emulsion,
die Zugabe einer Quenchlösung zu der Emulsion unter Bildung eines eine Mikrokugel umfassenden Volumens und
das Waschen, Filtrieren und Trocknen der Mikrokugel zum Reduzieren des Lösungsmittelgehalts.

11. Verfahren zur Herstellung einer stabilen pharmazeutisch unbedenklichen, eine Mikrokugel umfassenden Formulierung, wobei das Verfahren Folgendes umfasst:
die Bereitstellung einer ersten Phase, die Folgendes umfasst:
ein biologisch abbaubares Polymer nach einem der Ansprüche 1-6,
eine Wirkstoffbeladung aus der freien Base von Bupivacain oder einem pharmazeutisch unbedenklichen Salz von Bupivacain und
ein Lösungsmittelsystem, das geeignet ist, das Polymer und Bupivacain zu lösen,
das Emulgieren der ersten Phase mit einer zweiten Phase unter Bildung einer Emulsion,
wobei die zweite Phase eine ein Tensid umfassende wässrige Lösung umfasst und
das Entfernen eines wesentlichen Teils des Lösungsmittelsystems aus der Emulsion unter Erhalt einer Mikrokugel.

12. Stabile pharmazeutisch unbedenkliche, eine Mikrokugel umfassende Formulierung zur Verwendung bei der Behandlung von Schmerzen, wobei die Mikrokugel ein biologisch abbaubares Polymer gemäß einem der Ansprüche 1-6 und 200 mg bis 8000 mg der freien Base von Bupivacain oder eines pharmazeutisch unbedenklichen Salzes davon umfasst, wobei die pharmazeutische Formulierung eine therapeutisch wirksame Konzentration der freien Base von Bupivacain oder eines pharmazeutisch unbedenklichen Salzes davon für 2 Tage bis 14 Tage nach Verabreichung an ein Subjekt bewirkt.

13. Vorgefüllter Injektor, der Folgendes umfasst:
eine stabile pharmazeutisch unbedenkliche Formulierung, die Folgendes umfasst:
eine Mikrokugel, die Folgendes umfasst:
ein biologisch abbaubares Polymer nach einem der Ansprüche 1-6 und
eine Wirkstoffbeladung aus der freien Base von Bupivacain oder einem pharmazeutisch unbedenklichen Salz von Bupivacain.

14. Verfahren zur Herstellung eines vorgefüllten, eine stabile pharmazeutisch unbedenkliche Formulierung umfassenden Injektors, wobei das Verfahren Folgendes umfasst:
die Herstellung einer stabilen pharmazeutisch unbedenklichen Formulierung, die Folgendes umfasst:
eine Mikrokugel, die Folgendes umfasst:
ein biologisch abbaubares Polymer nach einem der Ansprüche 1-6 und
eine Wirkstoffbeladung aus der freien Base von Bupivacain oder einem pharmazeutisch unbedenklichen Salz von Bupivacain.
das Beladen einer sterilen Kartusche mit der stabilen pharmazeutisch unbedenklichen Formulierung und
das funktionsfähige Befestigen der sterilen Kartusche an einem Injektor.

15. Langwirksame Verabreichungsform, die Folgendes umfasst:
eine Mikrokugel, die Folgendes umfasst:
ein biologisch abbaubares Polymer nach einem der Ansprüche 1-6 und
500 mg bis 4000 mg der freien Base von Bupivacain oder eines pharmazeutisch unbedenklichen Salzes davon,
wobei die Verabreichung einer Einzeldosis der langwirksamen Dosierungsform an ein Subjekt zu mindestens einem der aus der aus Folgendem bestehenden Gruppe ausgewählten pharmakokinetischen Parameter führt:
ein Steady-State-Plasmaprofil von Bupivacain von Tag 1 bis Tag 7 nach Verabreichung, das einen mittleren Cmax-Wert aufweist, der nicht größer ist als der Steady-State-Plasmaspiegel von Bupivacain, der durch eine subkutane Injektion von 100 mg Bupivacainhydrochlorid mit sofortiger Freisetzung bereitgestellt wird,
eine Bupivacain-Eliminationshalbwertszeit von 2 Stunden bis 4 Stunden und
ein Freisetzungsprofil nullter Ordnung oder erster Ordnung, was einer Freisetzung von 1 % bis 50 % der gesamten verabreichten Dosis von Bupivacain oder eines pharmazeutisch unbedenklichen Salzes davon pro Tag entspricht.

16. Kit, das Folgendes umfasst:
ein erstes Fläschchen, das eine konzentrierte Form der stabilen pharmazeutisch unbedenklichen Formulierung nach einem der Ansprüche 1-9 oder der langwirksamen Verabreichungsform nach Anspruch 15 umfasst,
ein zweites Fläschchen, das ein pharmazeutisch unbedenkliches Verdünnungsmittel umfasst,
eine erste Spritze, die zum Entnehmen des pharmazeutisch unbedenklichen Verdünnungsmittels aus dem zweiten Fläschchen geeignet ist,
einen Adapter, der funktionsfähig an der ersten Spritze angebracht werden kann und zum Abgeben des pharmazeutisch unbedenklichen Verdünnungsmittels in das erste Fläschchen geeignet ist,
eine zweite Spritze, die zum Entnehmen einer Flüssigkeit aus dem zweiten Fläschchen und zum Injizieren der Flüssigkeit in ein dessen bedürftiges Subjekt geeignet ist, und
eine Anleitung zum Verdünnen der konzentrierten Form und zur Verabreichung der stabilen pharmazeutisch unbedenklichen Formulierung oder der langwirksamen Verabreichungsform an das Subjekt.

## Revendications

1. Formulation pharmaceutiquement acceptable stable comprenant des microsphères, les microsphères comprenant :
un copolymère multibloc biodégradable ; et
une charge médicamenteuse active de base libre de bupivacaïne ou d'un sel pharmaceutiquement acceptable de bupivacaïne,
le copolymère multibloc comprenant au moins un segment de prépolymère hydrolysable (A) et au moins un segment de prépolymère hydrolysable (B), les segments étant reliés par un allongeur de chaîne multifonctionnel et les segments étant répartis de manière aléatoire et non alternée sur la chaîne du polymère,
le segment de prépolymère (A) comprenant du polyéthylèneglycol,
le segment de prépolymère (A) comprenant des produits réactionnels de glycolide et/ou de lactide (D et/ou L),
le segment de prépolymère (B) comprenant du poly(glycolide-co-L-lactide) présentant une Mₙ d'environ 4000 g/mol,
le segment de prépolymère (B) comprenant une quantité molaire de 5 % à 25 % de glycolide par rapport à la quantité molaire combinée de glycolide et de L-lactide,
le copolymère multibloc comprenant de 10 % à 99 % du segment de prépolymère (B) par rapport au poids total du copolymère multibloc,
l'allongeur de chaîne multifonctionnel étant le 1,4-diisocyanate de butane,
la formulation pharmaceutiquement acceptable stable ayant une durée de conservation de 14 jours à 25 °C après réfrigération.

2. Formulation pharmaceutiquement acceptable stable selon la revendication 1, le copolymère multibloc étant amorphe et ayant une température de transition vitreuse inférieure ou égale à 37 °C dans des conditions physiologiques.

3. Formulation pharmaceutiquement acceptable stable selon l'une quelconque des revendications 1 à 2, la teneur du segment de prépolymère (A) dans le copolymère multibloc rapportée au poids total du copolymère multibloc allant de 1 % à 90 %.

4. Formulation pharmaceutiquement acceptable stable selon l'une quelconque des revendications 1 à 3, le segment de prépolymère (A) ayant une Mₙ supérieure ou égale à 500 g/mol.

5. Formulation pharmaceutiquement acceptable stable selon l'une quelconque des revendications 1 à 4, le segment de prépolymère (B) comprenant du poly(glycolide-co-L-lactide) présentant une Mₙ d'environ 4000 g/mol.

6. Formulation pharmaceutiquement acceptable stable selon l'une quelconque des revendications 1 à 5, le segment de prépolymère (B) comprenant une quantité molaire d'environ 15 % de glycolide par rapport à la quantité molaire combinée de glycolide et de L-lactide.

7. Formulation pharmaceutiquement acceptable stable selon l'une quelconque des revendications 1 à 6, la charge médicamenteuse active de base libre de bupivacaïne ou d'un sel pharmaceutiquement acceptable de bupivacaïne dans les microsphères étant de 10 % à 80 % p/p.

8. Formulation pharmaceutiquement acceptable stable selon l'une quelconque des revendications 1 à 7, comprenant moins de 5 % p/p d'une impureté dérivée de bupivacaïne après stockage dans un récipient hermétiquement fermé pendant 24 mois à une température de 25 °C.

9. Formulation pharmaceutiquement acceptable stable selon l'une quelconque des revendications 1 à 8, la formulation étant fournie dans un injecteur.

10. Procédé de production d'une formulation pharmaceutiquement acceptable stable comprenant des microsphères, le procédé comprenant :
la fourniture d'une première phase comprenant :
un polymère biodégradable tel que défini dans l'une quelconque des revendications 1 à 6 ; et
une charge médicamenteuse active de base libre de bupivacaïne ou d'un sel pharmaceutiquement acceptable de bupivacaïne ;
l'ajout en continu d'une seconde phase comprenant un tensioactif en solution aqueuse dans la première phase pour former une émulsion ;
l'ajout d'une solution d'arrêt à l'émulsion pour produire un volume comprenant des microsphères ; et
le lavage, la filtration et le séchage des microsphères pour réduire la teneur en solvant.

11. Procédé de production d'une formulation pharmaceutiquement acceptable stable comprenant des microsphères, le procédé comprenant :
la fourniture d'une première phase comprenant :
un polymère biodégradable tel que défini dans l'une quelconque des revendications 1 à 6 ;
une charge médicamenteuse active de base libre de bupivacaïne ou d'un sel pharmaceutiquement acceptable de bupivacaïne ; et
un système solvant convenant pour dissoudre le polymère et la bupivacaïne ;
l'émulsification de la première phase avec une seconde phase, ce qui permet de former une émulsion ;
la seconde phase comprenant une solution aqueuse qui comprend un tensioactif ; et
l'élimination d'une grande partie du système solvant de l'émulsion, ce qui permet d'obtenir des microsphères.

12. Formulation pharmaceutiquement acceptable stable destinée à être utilisée dans le traitement de la douleur comprenant des microsphères, les microsphères comprenant un polymère biodégradable tel que défini dans l'une quelconque des revendications 1 à 6 et 200 mg à 8000 mg de base libre de bupivacaïne ou d'un sel pharmaceutiquement acceptable de celle-ci, la formulation pharmaceutique assurant une concentration thérapeutiquement efficace de base libre de bupivacaïne ou d'un sel pharmaceutiquement acceptable de celle-ci pendant 2 jours à 14 jours après l'administration à un sujet.

13. Injecteur prérempli comprenant :
une formulation pharmaceutiquement acceptable stable comprenant :
des microsphères comprenant :
un polymère biodégradable tel que défini dans l'une quelconque des revendications 1 à 6 ; et
une charge médicamenteuse active de base libre de bupivacaïne ou d'un sel pharmaceutiquement acceptable de bupivacaïne.

14. Procédé de fabrication d'un injecteur prérempli comprenant une formulation pharmaceutiquement acceptable stable, le procédé comprenant :
la préparation d'une formulation pharmaceutiquement acceptable stable comprenant :
des microsphères comprenant :
un polymère biodégradable tel que défini dans l'une quelconque des revendications 1 à 6 ; et
une charge médicamenteuse active de base libre de bupivacaïne ou d'un sel pharmaceutiquement acceptable de bupivacaïne.
le chargement de la formulation pharmaceutiquement acceptable stable dans une cartouche stérile ; et
la fixation de manière fonctionnelle de la cartouche stérile à un injecteur.

15. Forme galénique à action prolongée comprenant :
des microsphères comprenant
un polymère biodégradable tel que défini dans l'une quelconque des revendications 1 à 6 ; et
500 mg à 4000 mg de base libre de bupivacaïne ou d'un sel pharmaceutiquement acceptable de celle-ci,
l'administration d'une seule dose de la forme galénique à action prolongée à un sujet conduisant à au moins l'un des paramètres pharmacocinétiques choisis dans le groupe constitué par :
un profil plasmatique à l'état d'équilibre de la bupivacaïne du jour 1 au jour 7 après l'administration montrant une valeur moyenne de Cmax qui n'est pas plus élevée que le taux plasmatique à l'état d'équilibre de la bupivacaïne fourni par l'injection sous-cutanée de 100 mg de chlorhydrate de bupivacaïne à libération immédiate ;
une demi-vie d'élimination de la bupivacaïne de 2 heures à 4 heures ; et
un profil de libération d'ordre zéro ou du premier ordre correspondant à une libération de 1 % à 50 % de la dose totale administrée de bupivacaïne ou d'un sel pharmaceutiquement acceptable de celle-ci par jour.

16. Trousse comprenant :
un premier flacon comprenant une forme concentrée de la formulation pharmaceutiquement acceptable stable selon l'une quelconque des revendications 1 à 9 ou de la forme galénique à action prolongée selon la revendication 15 ;
un second flacon comprenant un diluant pharmaceutiquement acceptable ;
une première seringue convenant pour le prélèvement du diluant pharmaceutiquement acceptable du second flacon ;
un adaptateur qui peut être fixé de manière fonctionnelle à la première seringue et qui convient pour l'introduction du diluant pharmaceutiquement acceptable dans le premier flacon ;
une seconde seringue convenant pour le prélèvement d'un liquide du second flacon et pour l'injection du liquide chez un sujet qui en a besoin ; et
des instructions pour la dilution de la forme concentrée et pour l'administration de la formulation pharmaceutiquement acceptable stable ou de la forme galénique à action prolongée au sujet.
